# EUROPEAN PATENT APPLICATION

(11) **EP 4 512 826 A2**
(43) Date of publication of application: **26.02.2025**
(21) Application number: 24222974.8
(22) Date of filing: 11.07.2022
(51) Int. Cl.: C07K 16/00

(54) **DIMERIC PROTEIN COMPLEXES AND USES THEREOF**

(30) Priority: 09.07.2021 EP 21184691
(62) Divisional of application: 22747688.4
(71) Applicant: Luxembourg Institute Of Health (LIH), 1445 Strassen (LU)
(72) Inventor: DERVILLEZ, Xavier, 54668 Prümzurlay (DE); SCHOBER, Rafaëla, Maria, 57390 Audun-le-Tiche (FR); BRANDUS, Bianca, 57390 Audun-le-Tiche (FR); DEVAUX, Carole, 57330 Zoufftgen (FR); ZIMMER, Jacques, René, 4418 Soleuvre (LU); COHEN, Jacques, Henri, Max, 51100 Reims (FR)
(74) Representative: De Clercq & Partners

(57) **Abstract**

The present invention is situated in the field of multimers used for targeted therapies. More particularly, the present invention provides a dimeric protein complex comprising a first polypeptide comprising a first functional component and the C-terminal fragment of the C4bp beta-chain, wherein said first functional component is coupled to the C-terminus of the C-terminal fragment of the C4bp beta-chain; and a second polypeptide comprising a second functional component and the C-terminal C4bp beta-chain, wherein said second functional component is coupled to the C-terminus of the C-terminal fragment of the C4bp beta-chain; wherein said first and second polypeptides are identical or different and wherein said first and second functional components are proteins or polypeptides. Further provided are nucleic acids encoding said first or second polypeptide of the dimeric protein complex and pharmaceutical compositions comprising the dimeric protein complex. The invention also relates to the use of the dimeric protein complexes in immunotherapy.

## Description

### TECHNICAL FIELD

The present invention is situated in the field of multimeric protein complexes and the use thereof for targeted therapies. More particularly, the invention relates to dimeric protein complexes comprising a scaffold, which display two or more functional components.

### BACKGROUND OF THE INVENTION

Recombinant DNA technology has provided the possibility of large-scale production of biologically active proteins for use in several therapeutic applications. Many recombinant DNA produced products are already in the clinic or currently under development, such as large proteins, small peptides, or antibody fragments.

Multimerization of protein and peptide molecules has been shown to increase the half-life of these molecules in vivo, allowing them to exert their activity over a longer period of time. Moreover, multimeric protein complexes offer the opportunity to combine several functional components (such as components with a therapeutic and a targeting functionality) into one molecule, allowing targeted therapy which often increases effectiveness and reduces unwanted side-effects. Furthermore, joint delivery of different therapeutic components can lead to a synergistic therapeutic effect. In addition or alternatively, the combination of different targeting components may increase the binding specificity and affinity or may bring two target cells into proximity of each other.

Although highly efficient, such multimeric protein complexes have some drawbacks. First, their chemical formulation is not precisely defined. Second, the number of functional components being added to a polymer molecule is often random and cannot be controlled. This is particularly troublesome when it is of interest to load a combination of functional components on such a polymer. Furthermore, in most cases, the functional components are attached to a scaffold or backbone. Such scaffold and backbones are often large molecules, which may result in poor penetration into tissues, poor binding to regions on the surface of some molecules which are accessible by molecules of smaller size and/or poor production yields.

Accordingly, there remains a pressing need for improved multimeric protein complexes.

### SUMMARY

The C4b binding protein (C4bp) is a normal plasma protein and hence, non-immunogenic. C4bp is a powerful soluble negative regulator of the complement classical pathway (CP), but also participates to the regulation of the coagulation processes through the binding of protein S to the first two short consensus repeats (SCRs) (SCR1-SCR2) of the C4bp beta-chain. The C4bp has a quaternary structure consisting of 7 identical alpha-chains and a single beta-chain (7α1β) for the main molecular species (70%). Additional minor molecular species are also found, displaying 6α1β, 6α or 7α without β. Each alpha-chain is composed of 8 short consensus repeats (SCRs, SCR1 to SCR8) followed by a non-SCR C-terminal end (C4bpα) of about 58 amino acids including 2 cysteines. The first three SCRs of C4bp alpha-chain (SCR1-SCR3) bind soluble or membrane surface-bound C4b, thus preventing further the formation of the C3*-convertase* (*C4b2a*) of the classical pathway at a target membrane surface, or bind the formed *C4b3a* C3*-convertase* which decays the biological activity of the latest. The single beta-chain is composed of 3 SCRs followed by a non-SCR C-terminal end (C4bpβ) of 59 amino acids including 2 cysteines (C202, C216) making disulphide bonds with 2 adjacent alpha-chains. Thus, the 7 alpha-chains plus the beta-chain are covalently associated through the formation of interchain disulfide bridges.

To their surprise, present inventors have found that the C-terminal part of the C4bp beta-chain (C4bpb) can be used as a scaffold for dimerization and that functional components can be introduced C-terminally (i.e. downstream) of said C-terminal part of the C4bp beta-chain. Such dimeric protein complexes may be referred to herein as "Bolos" or "Beta (β)-Bolos". Unexpectedly, the presence of a functional component downstream from the C4bp beta-chain in a non-natural location was found not to prevent its natural spontaneous dimerisation in eukaryotic cells in the endoplasmic reticulum/Golgi molecule export cellular organelles.

There are many advantages to couple a functional component C-terminally of the C-terminal fragment of the C4bp beta-chain. Coupling a functional component that is naturally located in C-terminal position of a polypeptide, as is the case for instance for the Fc fragment of an antibody, upstream of the C-terminal part of the C4bp beta-chain could impair the biological activity of such functional components, as steric hindrance issues may occur with the proximal dimerising scaffold. When introducing these polypeptides C-terminally of the C-terminal fragment of the C4bp beta chain, these C-terminal functional components are able to exert their biological functions, such as for the "pseudo IgGs" as described herein.

Moreover, even for protein or polypeptides having a biological function/activity, such as a binding domain, at their N-terminal end, such as for instance scFv or V_{H}H polypeptides, it was surprisingly found that cloning of said protein or polypeptide C-terminally of the dimerization scaffold of C4bp beta did not result in the biological activity of said protein or polypeptide being reduced or eliminated (e.g. reduced binding capacity), such as for some of the "TetraBolos" as described herein. An advantage thereof is that such protein or polypeptide, like scFv or V_{H}H, can be included both N- and C-terminally of the dimerization scaffold of C4bp beta, resulting in dimeric protein complexes with a high valence (e.g. such as up to 4 valences) of said protein or polypeptide. In addition, the inventors have found that it is possible to obtain multifunctional (such as bifunctional) dimeric protein complexes from a single expression vector including at least one functional component upstream as well as at least one functional component downstream of the C-terminal fragment of the C4bp beta chain. When transfecting a single expression vector comprising at least two functional components into a cell, this cell will release a single multifunctional dimeric molecular species into the cell culture supernatant.

In contrast to the use of the C4bp alpha-chain as a scaffold, the use of the C-terminal fragment of the C4bp beta-chain as a scaffold allows to obtain dimeric protein complexes of a relatively small size (e.g. around 70-240 kDa, such as 100-240 kDa). Small protein complexes are more physiological than larger protein complexes, offer an improved bio-distribution space and may have a faster renal elimination, thereby making them more suitable as therapeutics.

For example, the use of the C-terminal fragment of the C4bp beta chain as a scaffold allows producing molecules whose molecular weight, and consequently whose extravascular diffusion space, is in the same range as a monomeric IgG. As the dimeric protein complexes of the present invention can comprise more than 2 valences, such as 3 or 4 valences, the avidity of the dimeric protein complexes of present invention, such as mono-functional "HomoTetraBolos" as described in the present specification, can be superior to that of an IgG. More particularly, such "HomoTetraBolos" may have a better binding, larger antigen capture, and/or better neutralization ability than IgGs.

The dimeric protein complexes of present invention also allow to selectively modulate a cellular function. For example, dimeric protein complexes can be generated that physically bring tumour cells or pathogens towards immune effector cells, such as NK cells, to force the synapse formation in order to activate the immune effector cells towards this tumour cell or pathogen.

Furthermore, as the C-terminal part of the C4bp beta-chain is derived from a naturally circulating molecule and the C-terminal part of the C4bp beta-chain has no known biological function, the potential immunogenicity of the dimeric protein complexes of present invention is limited.

Moreover, the dimeric protein complexes of present invention comprising a first and a second polypeptide which are identical can be prepared using a single construct generating a single molecular species (as could be determined using a SDS-PAGE followed by a SYPRO ruby staining or a Western blotting, under non-reducing or reducing conditions), which greatly simplifies the design and process of production and purification.

Accordingly, a first aspect provides a dimeric protein complex comprising two polypeptides, each of which comprises a sequence corresponding to a functional component and the C-terminal fragment of the C4bp beta-chain. The term "functional component" is defined more in detail herein but generally refers to an amino acid sequence with a predetermined function. In particular embodiments, the first aspect can be described to relate to a dimeric protein complex comprising
a first polypeptide comprising a first functional component and the C-terminal fragment of the C4bp beta-chain, wherein said first functional component is coupled to the C-terminus of the C-terminal fragment of the C4bp beta-chain; and
a second polypeptide comprising a second functional component and the C-terminal fragment of the C4bp beta-chain, wherein said second functional component is coupled to the C-terminus of the C-terminal fragment of the C4bp beta-chain;
wherein said first and second polypeptides are identical or different, preferably wherein said first and second functional components are peptides, proteins or polypeptides.

In particular embodiments, said first polypeptide further comprises a third functional component that is coupled to the N-terminus of said C-terminal fragment of the C4bp beta-chain; and said second polypeptide further comprises a fourth functional component that is coupled to the N-terminus of said C-terminal fragment of the C4bp beta-chain;
wherein said first, said second, said third and said fourth functional components are identical or different.

In particular embodiments, said first, said second, said third and/or said fourth functional component is a protein or polypeptide, preferably a protein or polypeptide selected from the group consisting of a binding domain, a recombinant viral structural protein, an oncolytic agent, a cytotoxic agent, a cytokine, a receptor-binding peptide, or monomeric Fc, more preferably a protein or polypeptide selected from the group consisting of a binding domain (e.g. an antigen recognition domain), a recombinant viral structural protein, a cytotoxic agent, a cytokine, a receptor-binding peptide, or monomeric Fc.

In particular embodiments, said a binding domain (e.g. antigen recognition domain) is a single chain variable fragment of an antibody (scFv) or a single domain variable fragment of a heavy chain antibody (V_{H}H) specific for the antigen, an antibody-like scaffold, an extracellular domain of a viral envelope protein, a cognate extracellular domain of a receptor or ligand for the antigen or an antigen-binding portion of said receptor or ligand, a soluble receptor or a synthetic receptor.

In particular embodiments, said a binding domain (e.g. antigen recognition domain) specifically binds to a tumor specific antigen (TSA), a tumor associated antigen (TAA), a bacterial antigen, viral antigen or a virus-associated antigen, a fungal antigen, an activating NK cell receptor, a cytokine, a toxin, or a contaminant.

In particular embodiments, two of said first, said second, said third or said forth functional components comprise a binding domain (e.g. an antigen recognition domain) specifically binding to an activating NK cell receptor, such as wherein binding of the binding domain (e.g. the antigen recognition domain) to the activating NK cell receptor is capable of activating the NK cell; and wherein two of said first, said second, said third or said forth functional components comprise a binding domain (e.g. an antigen recognition domain) specifically binding to a TSA, a TAA, a bacterial antigen, a viral antigen, a viral-associated antigen or a fungal antigen.

Present inventors also found that the inclusion of a monomeric Fc, preferably monomeric Fc of IgG comprising the hinge, CH2 domain and CH3 domain of IgG, downstream (C-terminally) of the C-terminal fragment of the C4bp beta-chain of both the first and second polypeptide of the dimeric protein complex leads to unexpectedly enhanced, but controlled, Fc functions, such as Fc receptor (FcR) binding properties, complement activation (as illustrated by complement deposition), cell lysis and/or cell activation, when bound to an antigen (e.g. enhanced complement-dependent cytotoxicity (CDC), antibody-dependent cell-mediated cytotoxicity (ADCC)/antibody dependent cellular phagocytosis (ADCP) and complement-dependent cytotoxicity (CDCC)/complement-dependent cell-mediated phagocytosis (CDCP)) compared to conventional antibodies as a result of the double hinge that is created (i.e. the presence of a Fc hinge and a C4bp beta-chain hinge). For example, such dimeric protein complexes exert strong host complement activation, enhanced NK activation, such as through (i) Fc-CD16 [Fc(gamma)RIIIA] interaction and (ii) C3b breakdown products (i.e. iC3b, C3d,g, C3d) -CD11b interaction, and enhanced macrophage-mediated phagocytosis of target cells. Examples of such dimeric protein complexes comprising the monomeric Fc of IgG are also referred to herein as "pseudo IgGs". Furthermore, without being bound by theory, the aforementioned properties of the pseudo IgGs may result from the inert nature of the C-terminal fragment of the C4bp beta-chain and/or the flexibility of the monomeric Fc located at the C-terminal part of the pseudo IgG.

It is noted that no complement activation occurs when the dimeric protein complex as taught herein comprising downstream (C-terminally) of the C-terminal fragment of the C4bp beta-chain of both the first and second polypeptide of the dimeric protein complex a monomeric Fc, such as the pseudo IgGs as referred to herein, is in the liquid phase (i.e. unbound), as binding of such dimeric protein complex to a cell or an antigen is required to result in complement activation. Accordingly, in particular embodiments,
the first and second functional component are monomeric Fc, preferably monomeric Fc of IgG comprising the hinge, CH2 domain and CH3 domain of IgG; and wherein the hinge region of the monomeric Fc in the first polypeptide is connected to the hinge region of the monomeric Fc in the second polypeptide by at least two disulfide bonds; and
the third and/or fourth functional component comprises a binding domain (e.g. an antigen recognition domain).

According to another aspect, there is provided a dimeric protein complex comprising
a first polypeptide comprising a first functional component and the C-terminal fragment of the C4b binding protein (C4bp) beta-chain, wherein said first functional component is coupled to the C-terminus of the C-terminal fragment of the C4bp beta-chain; and
a second polypeptide comprising a second functional component and the C-terminal fragment of the C4bp beta-chain, wherein said second functional component is coupled to the C-terminus of the C-terminal fragment of the C4bp beta-chain;
wherein said first polypeptide further comprises a third functional component that is coupled to the N-terminus of said C-terminal fragment of the C4bp beta-chain;
wherein said second polypeptide further comprises a fourth functional component that is coupled to the N-terminus of said C-terminal fragment of the C4bp beta-chain;
wherein said first, second, third and fourth functional components are proteins or polypeptides;
wherein two of said first, second, third and/or fourth functional components comprise a binding domain;
wherein said first and second polypeptides are identical or different; and
wherein the C-terminal fragment of the C4bp beta-chain is the 194-252 fragment of the C4bp beta-chain.

A further aspect provides a nucleic acid
encoding the first polypeptide of the dimeric protein complex comprising a first functional component and a C-terminal fragment of the C4bp beta-chain, wherein said first functional component is coupled to the C-terminus of the C-terminal fragment of the C4bp beta-chain as defined herein; and/or
encoding the second polypeptide comprising a second functional component and a C-terminal fragment of the C4bp beta-chain, wherein said second functional component is coupled to the C-terminus of said C-terminal fragment of the C4bp as defined herein. A further aspect provides an expression cassette comprising the nucleic acid as taught herein.

A further aspect provides an expression vector comprising the nucleic acid as taught herein or the expression cassette as taught herein.

A further aspect provides an expression vector comprising a nucleic acid encoding the C-terminal fragment of the C4bp beta-chain, an insertion site for a nucleic acid encoding a functional component located immediately 3' of the nucleic acid encoding the C-terminal fragment C4bp beta-chain; and optionally an insertion site for a nucleic acid encoding a functional component immediately 5' of the nucleic acid encoding the C-terminal fragment of the C4bp beta-chain.

A further aspect provides a pharmaceutical composition comprising the dimeric protein complex as taught herein, the nucleic acid as taught herein, the expression cassette as taught herein, or the expression vector as taught herein, and a pharmaceutically acceptable carrier.

A further aspect provides the dimeric protein complex as taught herein, the nucleic acid as taught herein, the expression cassette as taught herein, the expression vector as taught herein, or the pharmaceutical composition as taught herein, for use as a medicament, preferably for use in immunotherapy.

A further aspect provides the dimeric protein complex as taught herein, the nucleic acid as taught herein, the expression cassette as taught herein, the expression vector as taught herein, or the pharmaceutical composition as taught herein, for use in the treatment of a neoplastic disease or an infectious disease.

A further aspect provides the dimeric protein complex as taught herein, the nucleic acid as taught herein, the expression cassette as taught herein, the expression vector as taught herein, for use in a method of molecular imaging of a living body. These and further aspects and preferred embodiments of the invention are described in the following sections and in the appended claims. The subject-matter of the appended claims is hereby specifically incorporated in this specification.

### BRIEF DESCRIPTION OF DRAWINGS

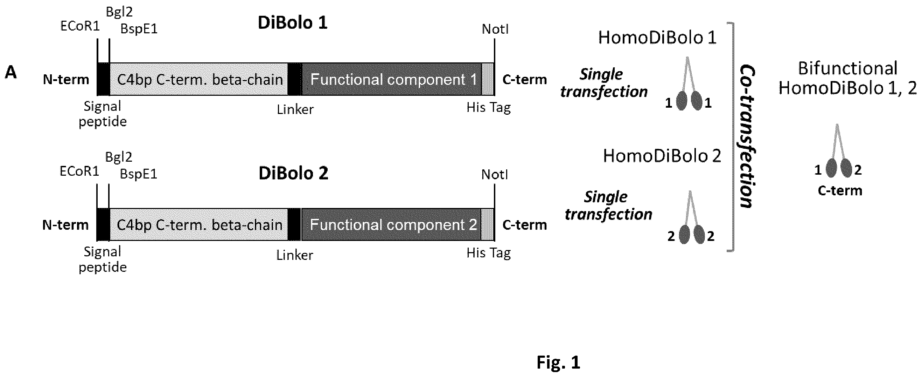

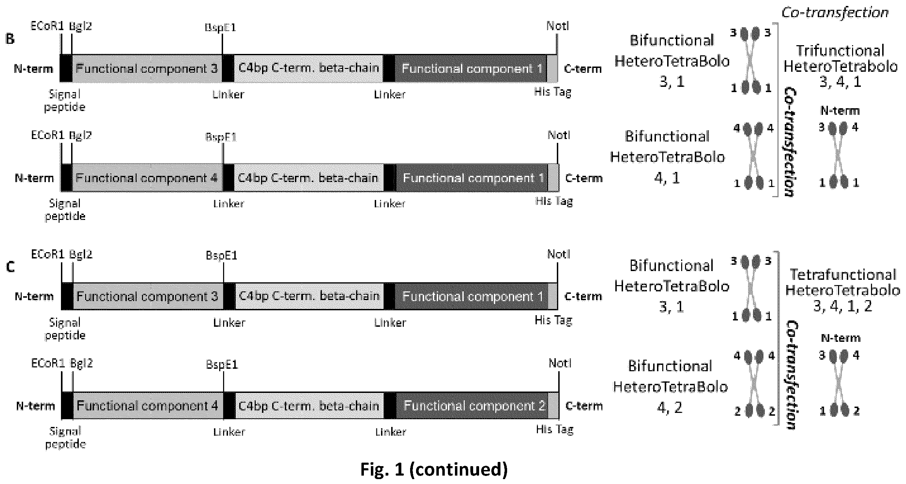

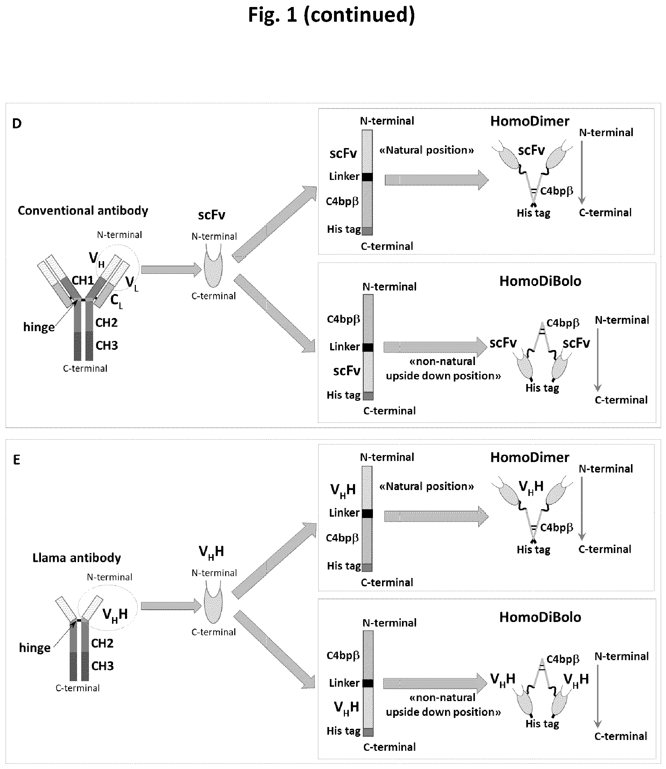

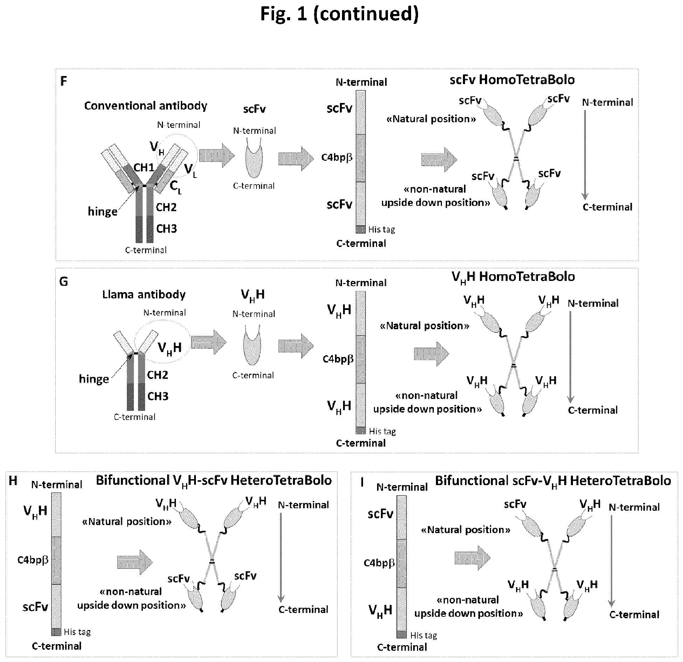

**Fig. 1** illustrates the "BOLO" technology as taught herein based on the inclusion of a functional component C-terminally of the C-terminal part of the C4bp beta-chain, wherein the C4bp beta-chain acts as a scaffold for dimerization of therapeutic biologics. **(A)** Exemplary schematic overview of expression cassettes for the expression of "DiBolos" of which the first and second polypeptide of the dimeric protein complex comprise a functional component C-terminally of the C-terminal part of the C4bp beta-chain and no functional component N-terminally of the C-terminal part of the C4bp beta-chain. Transfection of one of the exemplified expression cassettes allows the formation of HomoDiBolos comprising either the functional component 1 or 2 (i.e. mono-functional). Co-transfection of two different expression cassettes for DiBolos, one with functional component 1 and a second with functional component 2, results in the formation of a bifunctional HeteroDiBolo. **(B)-(C)** Exemplary schematic overview of expression cassettes for the expression of "TetraBolos" of which the first and second polypeptide of the dimeric protein complex comprise a functional component C-terminally of the C-terminal part of the C4bp beta-chain and a functional component N-terminally of the C-terminal part of the C4bp beta-chain. A transfection of one of the exemplified expression cassettes allows the formation of bifunctional TetraBolos. Co-transfection of two different expression cassettes for TetraBolos results in the formation of a tri- **(B)** or tetrafunctional **(C)** TetraBolos. **(D)-(E)** Exemplary schematic overview of expression cassettes for the expression of scFv **(D)** or V_{H}H **(E)** where the scFv or V_{H}H is cloned either upstream (Homodimer) or downstream (HomoDiBolo) of C4bp C-terminal beta-chain. A scFv is the result of an association of the 2 N-terminal variable domains of the light & heavy chains of a human immunoglobulin, whereas a V_{H}H is the N-terminal single variable domain of the heavy chain of a camelidae immunoglobulin (e.g. Bactrian camels, dromedary camels, llamas, alpacas, vicuñas & guanacos). When scFv or V_{H}H are located N-terminally of a construct, and thus linked at their C-terminal ends, such as upstream of C4bp C-terminal beta-chain (D & E top right), they are located in their "natural position" and are referred to herein as homodimers. In contrast, when they are located C-terminally of a construct and linked at their N-terminal ends, such as downstream of C4bp C-terminal beta-chain (D & E bottom right), they are located in a "non-natural" upside down position, also referred to in the present specification as "BOLO" position, such multimers are referred to herein as "HomoDiBolos". They are drawn as having the recognition domain (white half-circle) in the up position, with a bond linker just closed to it. In the first situation, a His tag may be located immediately downstream of C4bp C-terminal beta-chain, whereas in the second situation, it may be located downstream of the Bolo scFv or V_{H}H. **(F)-(G)** Exemplary schematic overview of expression cassettes for the expression of scFv HomoTetraBolos **(F)** & V_{H}H HomoTetraBolos **(G).** The scFv or V_{H}H downstream of C4bp C-terminal beta-chain as drawn upside down with recognition domain up and the bond is nearby the recognition domain. **(H)-(I)** Exemplary schematic overview of expression cassettes for the expression of bifunctional V_{H}H-scFv **(H)** or scFv-V_{H}H **(I)** HeteroTetraBolos.

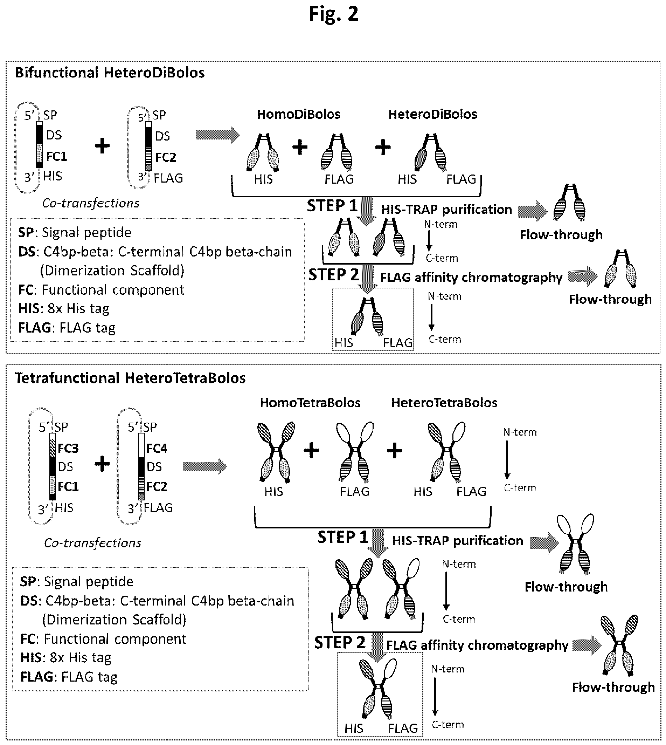

**Fig. 2** illustrates a 2-step purification strategy for selecting the multifunctional DiBolos or TetraBolos. The introduction of a tag, such as a HIS tag or FLAG tag allows to select the desired multifunctional DiBolos or TetraBolos, for example, by use of a HIS-TRAP purification (step 1) followed by a FLAG-affinity chromatography (step 2). FuCo: functional component, SP: signal peptide, DS: C-terminal C4bp beta-chain, HIS: 8x His tag, FLAG: FLAG tag.

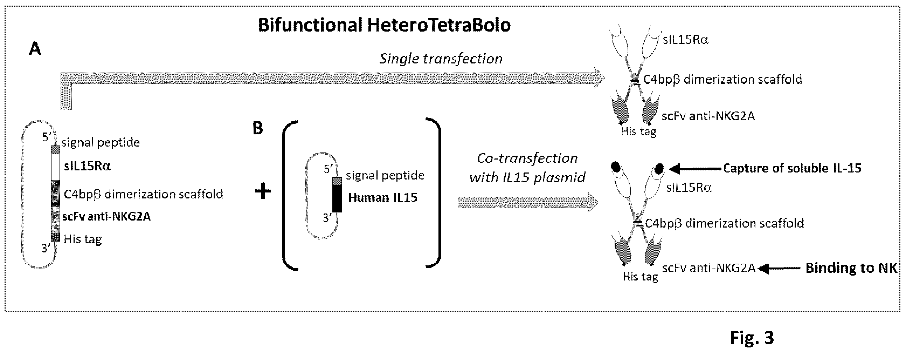

**Fig. 3** illustrates the expression of **(A-B)** bifunctional HeteroTetraBolo sIL-15Rα.C4bpβ.scFv-anti-NKG2A. The construct is either single transfected **(A)** or co-transfected **(B)** with a human recombinant IL-15 plasmid. The scFv anti-NKG2A expressed C-terminally of C4bp C-terminal beta-chain is in an "upside-down" position.

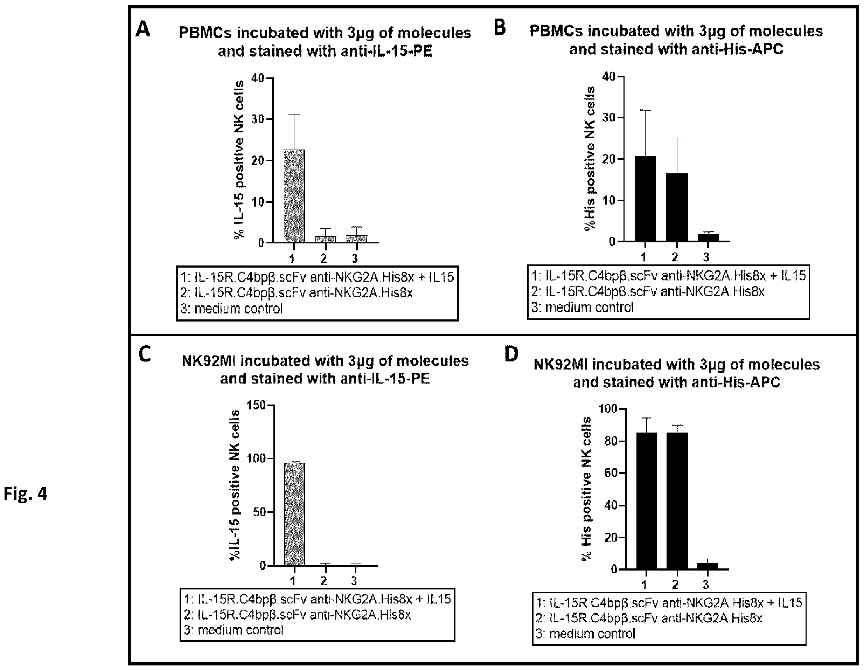

**Fig. 4****.** demonstrates the specific binding of purified HeteroTetraBolos to natural killer (NK) cells. His-Trap^{™} Excel-IMAC purified HeteroTetraBolos sIL-15Rα.C4bpβ.scFv.anti-NKG2A.His8x expressed from HEK293F cells co-transfected or not with human IL-15 were incubated with peripheral blood mononuclear cells (PBMCs) **(A and B)** or natural killer cells NK92MI **(C and D).** Cells were then stained for NK markers, IL-15 and His.

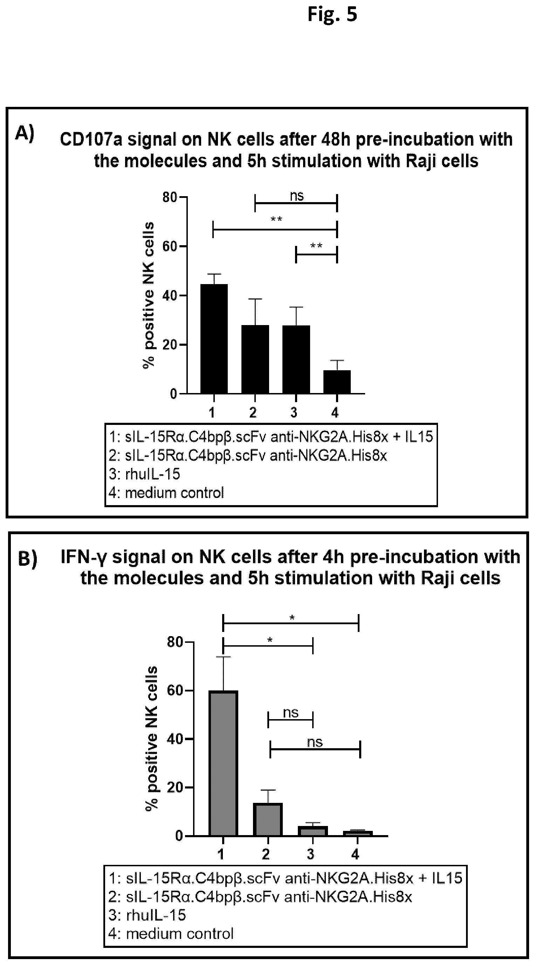

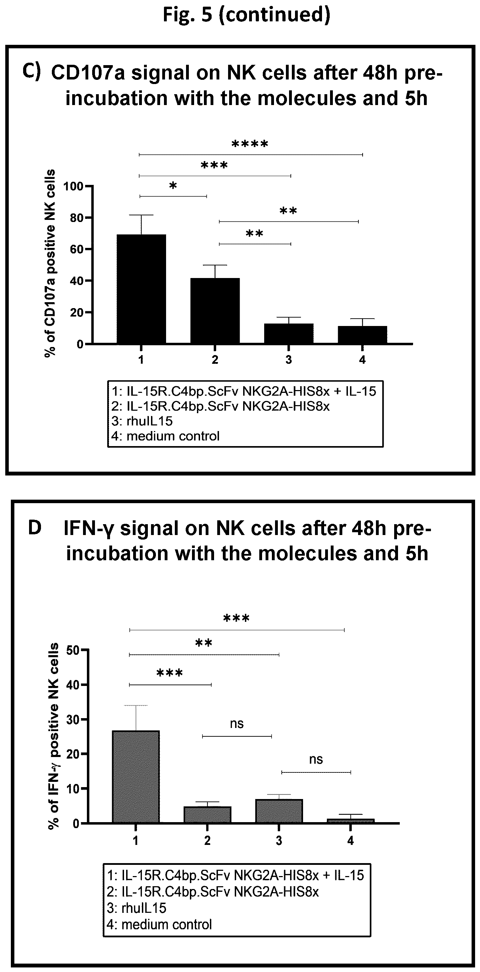

**Fig. 5****.** demonstrates the functional effect of HeteroTetraBolos on the degranulation and cytotoxic capacity of NK cells. His-Trap^{™} Excel-IMAC purified HeteroTetraBolo sIL-15Rα.C4bpβ.scfv.anti-NKG2A.His8x expressed from HEK293F cells co-transfected or not with human IL-15 (huIL-15) were incubated with PBMCs for 48h **(A, C,** D) or 4h **(B).** Cells were then stimulated with Raji target cells and co-incubated with anti-CD107a for 5h **(A),** permeabilized and stained for interferon gamma (IFN-γ) for 5h **(B).** In **(C)** NK cells were stimulated with HIV-1 infected ACH2 cells and co-incubated with anti-CD107a for 5h, and in (D) permeabilized and stained for IFN-γ intracellular expression for 5 h.

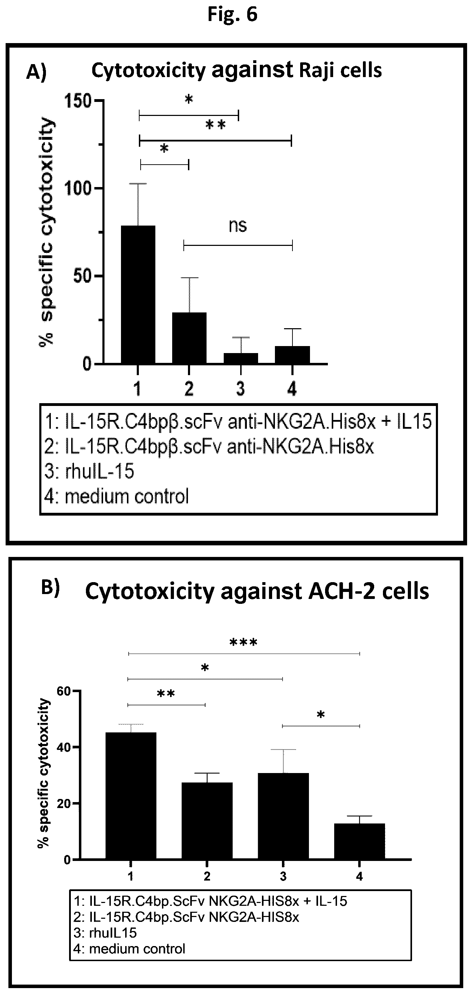

**Fig. 6****.** demonstrates the functional effect of HeteroTetraBolos on the cytotoxicity of NK cells. His-Trap^{™} Excel-IMAC purified HeteroTetraBolo sIL-15Rα.C4bpβ.scFv.anti-NKG2A.His8x expressed from HEK293F cells co-transfected or not with recombinant huIL-15 plasmid were incubated with PBMCs for 48h. Cells were then incubated with Raji target cells (stained with Cell Tracer Violet) (A) or ACH2 cells **(B)** for 24h and stained for L/D.

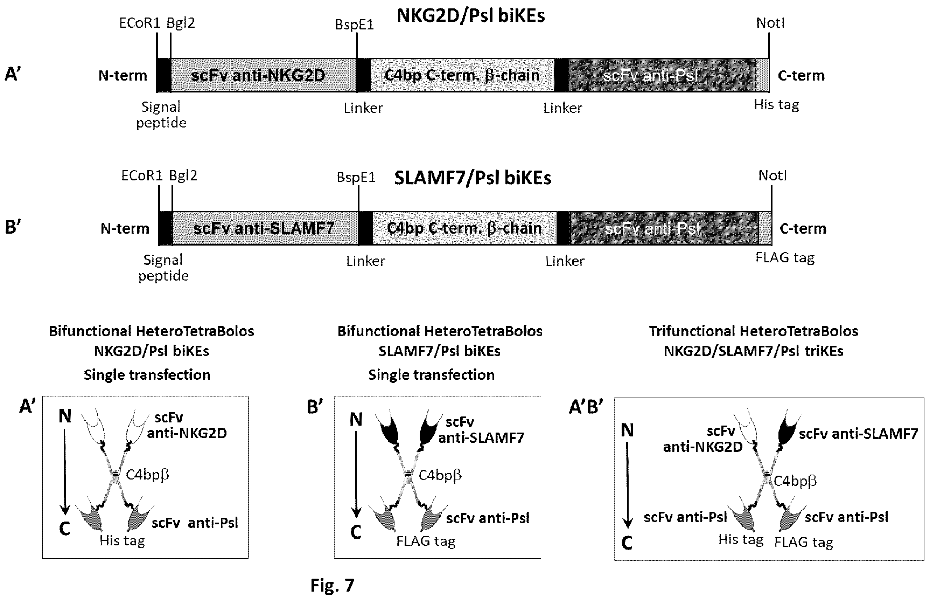

**Fig. 7****.** depicts the details of constructs: Construct A' (NKG2D/Psl): MS scFv [(RTX V_{L} 10-first amino-acids / MS (V_{L}-V_{H})] anti-NKG2D.C4b**pβ**.scFv (V_{L}-V_{H}) anti-Psl.**His8x** and Construct B' (SLAMF7/Psl): ELO scFv [(RTX V_{L} 10-first amino-acids / ELO (V_{L}-V_{H})] anti-SLAMF7.**C4bpβ**.scFv (V_{L}-V_{H}) anti-Psl.**FLAG.** The single tranfection of A' of B' lead to the expression of BiKEs (NKG2D/Psl or SLAMF7/Psl), whereas the co-transfection of A' & B' constructs lead to the expression of TriKEs (NKG2D/SLAMF7/Psl).

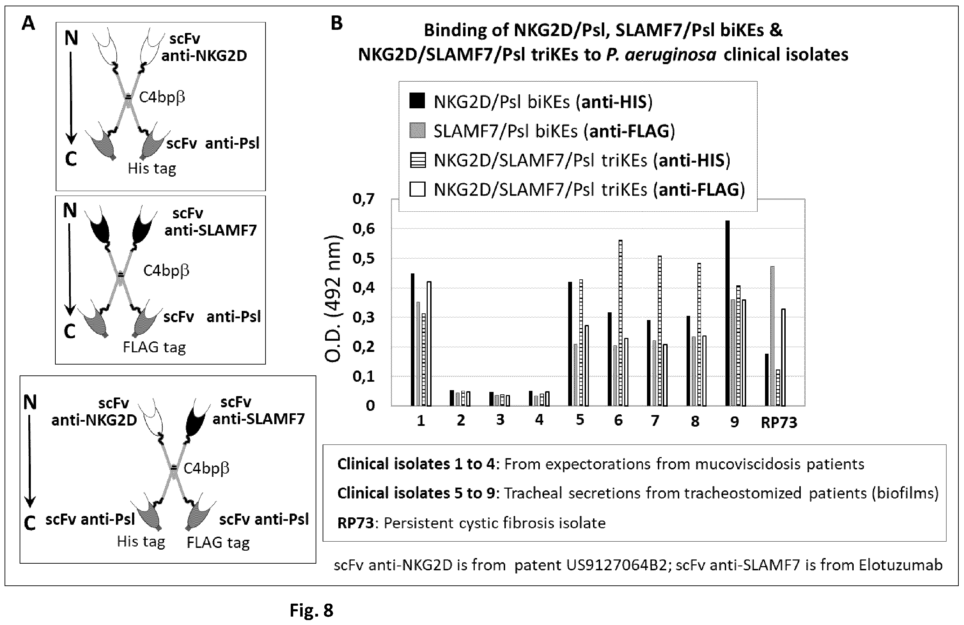

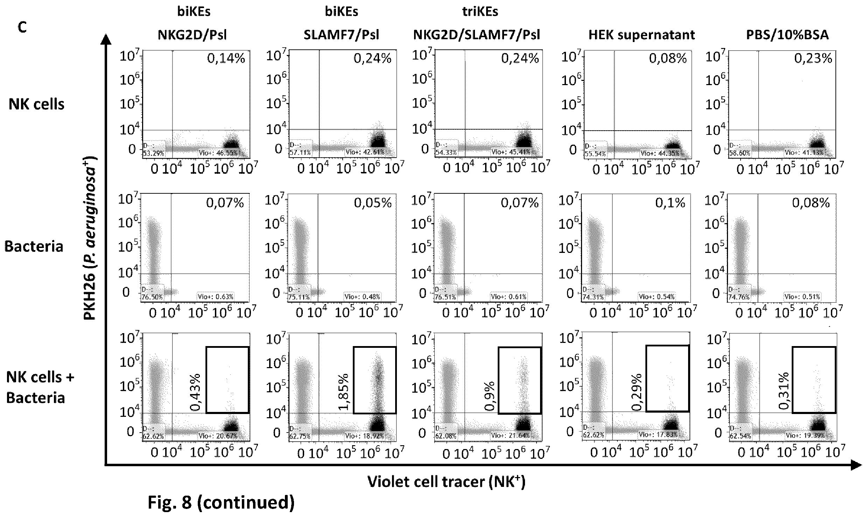

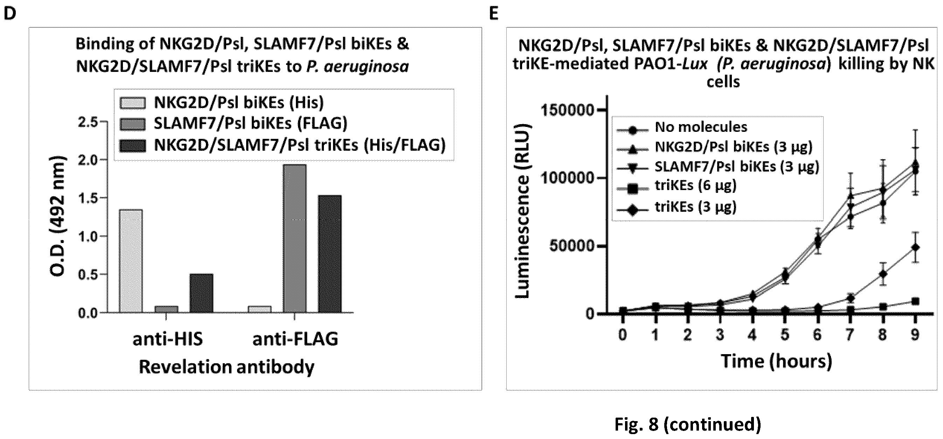

**Fig. 8****.** shows the binding of the BiKEs and TriKEs with scFv-anti-Psl to *Pseudomonas aeruginosa* using ELISA with unfixed coated bacteria. **A)** Sketch of the BiKes & TriKEs biologics used. The two upper panels are BiKEs, while the lower panel is a TriKE biologics. **B)** Results of binding of the molecules to the bacteria. Ten clinical *Pseudomonas* isolates were used. **C)** The double staining violet⁺/PKH26⁺ (cross-linking Pseudomonas/NK92MI) induced by the BiKE anti-SLAMF7/anti-Psl (1.85%) as well as the tTriKE anti-NKG2D/anti-SLAMF7/anti-Psl (0.9%), and in a much lesser extend the BiKE anti-NKG2D/anti-Psl (0.43%). The negative controls display 0.29% and 0.31% double staining. Of note, NKG2D is much less expressed on NK92MI than SLAMF7 (data not shown), which could explain why the NKG2D staining is weak on MK92MI. **(D)** ELISA with unfixed coated *P*. *aeruginosa* and revelation with either anti-HIS or anti-Flag antibodies show that BiKEs NKG2D/Psl, BiKEs SLAMF7/Psl & TriKE NKG2D/SLAMF7/Psl express the Psl scFv as well as their respective NK receptor targeting moieties. BiKE NKG2D/Psl (A') was purified on an HIS-TRAP column, BiKEs SLAMF7/Psl (B') were purified using a FLAG-affinity chromatography while His & FLAG tags in the structure of the TriKEs (A'B') was purified using a 2-step method including first a HIS-affinity chromatography and a subsequent FLAG-affinity chromatography to demonstrate that the selected TriKE clone bear both scFvs anti-NKG2D and anti-SLAMF-7. In (E), the killing of luminescent *Pseudomonas* strain PAO1-*lux* induced by NK cells after binding with 3 and 6 µg of the TriKE NKG2D /SLAMF7 is shown. 2.10⁵ NK92-CD16 cells were plated together with *Pseudomonas* PAO 1-lux strain at a final E/T ratio 1/3 in 200 µl complete RPMI medium without antibiotics and cultured at 37°C. Molecules were added at To and bacterial growth was measured in triplicates over time with a luminescence microplate reader during 9 hours.

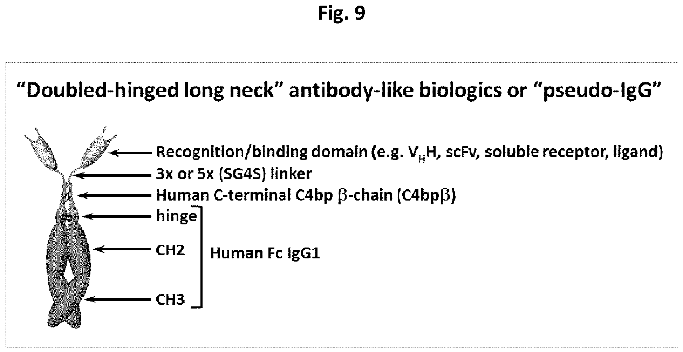

**Fig. 9****.** Depicts a sketch representing the structure of an exemplary double-hinged "long-neck" antibody-like glycoprotein, also referred to herein as "pseudo-IgG". The "double-hinged" states for the concomitant presence of (i) the C4bp C-terminal beta-chain dimerization scaffold displaying 2 cysteines along with following (ii) hinge from IgG1-Fc displaying also 2 cysteines, thus conferring a high stability of the dimers, and conferring a "long neck domain that separates the targeting moiety from the CH2-CH3 Fc IgG1 domain. The linker length between the targeting moiety and the C4bp C-terminal beta-chain can vary.

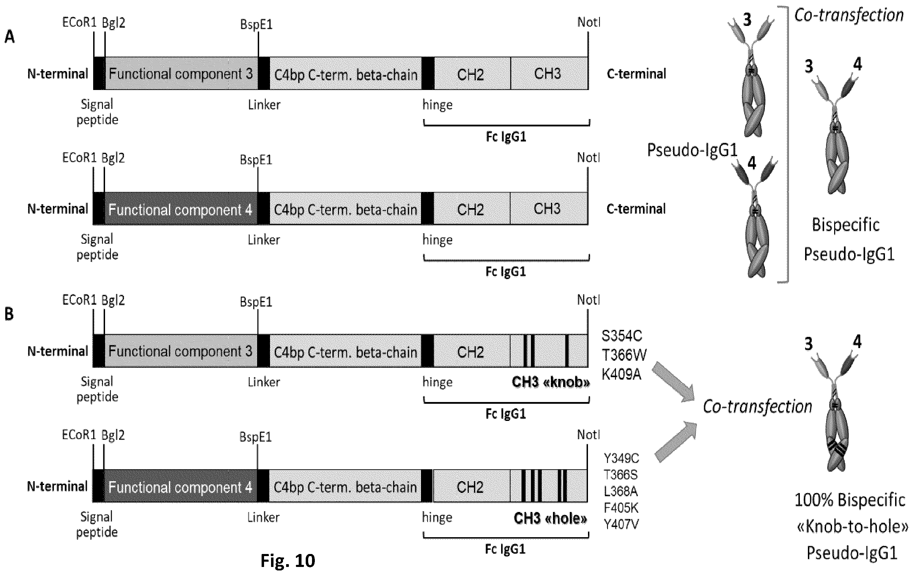

**Fig. 10****.** Illustrates expression cassettes for the pseudo-IgGs: **A)** Two constructs are depicted, both displaying functional component 1 as the IgG1 Fc (hinge+CH2+CH3) downstream of the C4bp(beta) and functional components 3 & 4 upstream of the C4bp(beta), respectively. Single transfections lead to the expression of monospecific pseudo-IgGs with recognition functions 3 or 4. The co-transfection of the 2 constructs lead to the expression of bifunctional 3, 4 pseudo-IgGs along with the monospecific 3 and 4 counterparts. **B)** Combination of the Bolo technology with the charge repulsion-induced heterodimeric Fc platform also known as "knob-into-hole" technology to get a 100% expression of bispecific pseudo-IgGs, thus avoiding having to purify the bispecific pseudo-IgG from the 2 other monospecific counterparts. Alternative knob-into-hole technologies can also be applied (such as described in John BB Ridgway, Leonard G Presta & Paul Carter. Protein Engineering, 1996; 9;7;pp.617-621).

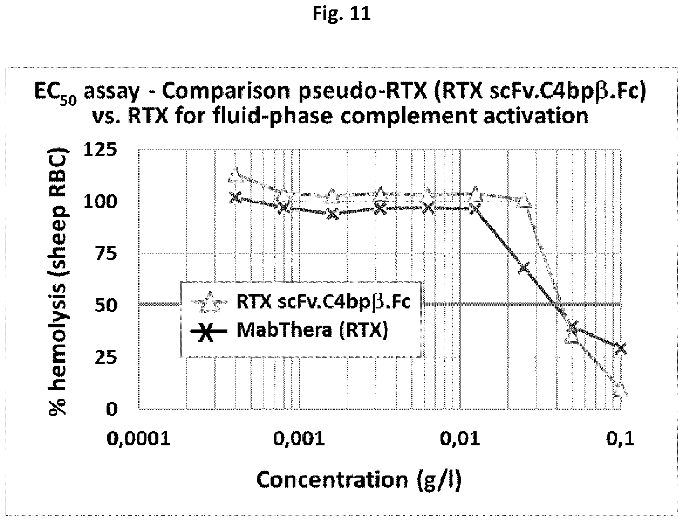

**Fig. 11****.** Shows a comparative test of fluid-phase complement activation using CH₅₀ assay. Importantly, pseudo-IgGs behave the same way as conventional therapeutic antibodies regarding their low-ability to activate soluble-phase plasma.

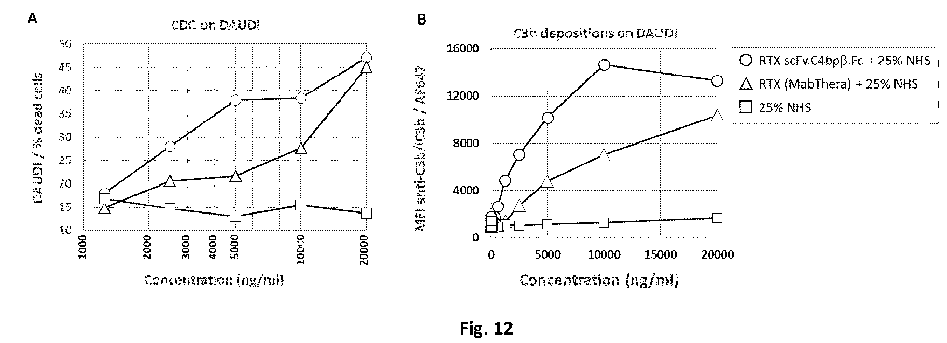

**Fig. 12****.** Analysis using flow cytometry of the CDC **(A)** as well as C3b deposits **(B)** on Daudi cells previously coated with saturating concentrations (20 µg/ml) of (i) RTX scFv.C4bpβ.Fc (pseudo-RTX), (ii) RTX (MabThera) or (iii) control (no molecules), followed by incubation (37°C, 30 min) with 25% normal human serum (NHS) in gelatin veronal buffer (GVB) supplemented with Ca⁺⁺ & Mg⁺⁺ (GVB⁺⁺; comprising 141 mM NaCl, 0.3 mM CaCl2, 1 mM MgCl2, 0.1% gelatin, 1.8 mM Na-barbital & 3.1 mM barbituric acid, pH 7.3-7.4). Cells were then stained with a mouse anti-hum C3b mAb (clone 7C12) and a secondary goat anti-mouse IgG Ab AF647-conjugated and a live/dead UV. The pseudo-IgG elicit superior C3b deposits **(B)** and subsequent increased CDC **(A)** of Daudi cells when compared to reference Rituximab (RTX).

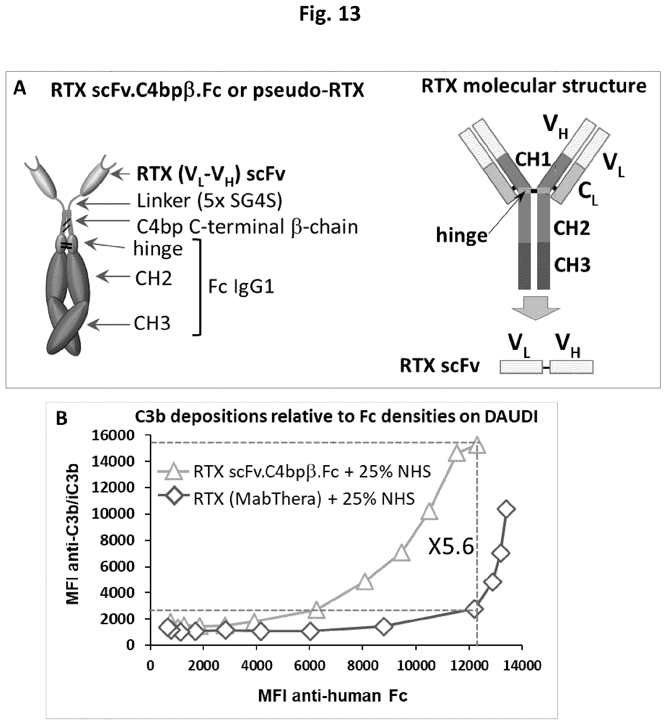

**Fig. 13** shows a comparative analysis using flow cytometry of C3b depositions relative to Fc densities on Daudi cells when using RTX versus pseudo-RTX. **A)** Details of the molecules used. **B)** Daudi cells were incubated with 2-fold serial dilutions of the molecules (starting concentration is 20 µg/ml, 30 min at 4 °C). Molecule-coated Daudi cells were then incubated with 25% normal human serum (NHS) in 50 µl GVB⁺⁺ for 30 min. at 37°C. Cells were stained with a mouse anti-human C3b mab (7C12), and a goat anti-mouse IgG pAb PE-conjugated, a goat anti-human Fc pAb AF647-conjugated and UV live/dead. Mean Fluorescence Intensities (MFI) for Fc densities & for C3b depositions are on the X- and Y-axis, respectively. At similar concentrations, pseudo-RTX leads to 5.6-fold higher C3b depositions than RTX.

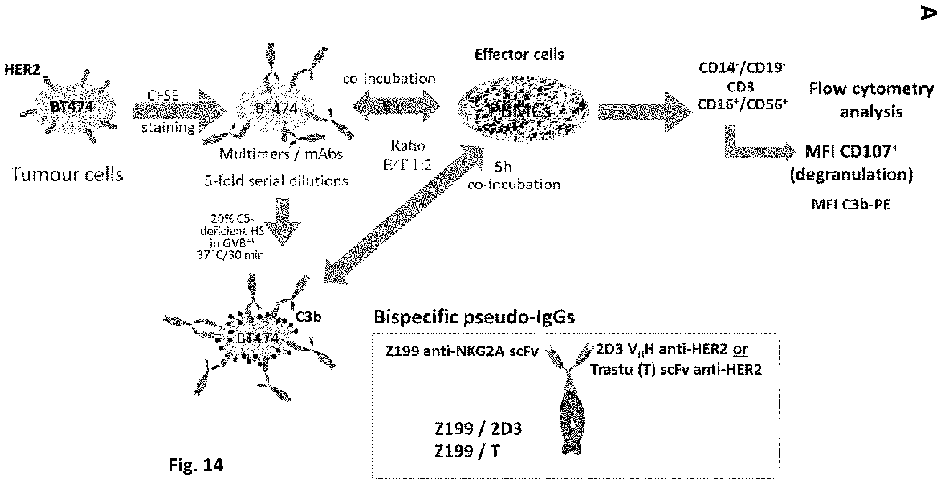

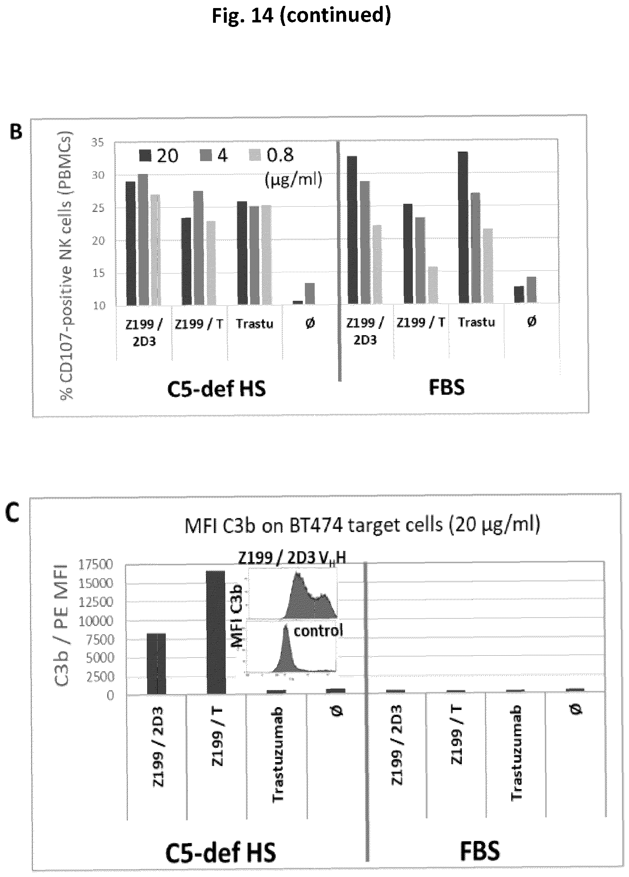

**Fig. 14****.** Shows a flow cytometry analysis of NK activation (% CD107-positive NKs) and complement activation (MFI anti-C3b) on BT474 target cells. **A)** BT474 were stained with CFSE, then incubated with 5-fold serial dilutions (20, 4, 0.8 µg/ml) of bispecific pseudo-IgG (Z199/2D3 or Z199/Trastu), or Trastuzumab, or no molecule. BT474 cells were incubated either with 20% C5-deficient human serum in GVB⁺⁺ (30 min at 37°C/5% CO₂) or with complete RPMI medium prior to co-culture with PBMCs for 5h at 37°C/5% CO₂. Cells were stained with an anti-humCD107/BV421 (after 1h co-incubation), and then with anti-human IgG/AF647 pAb, as well as with anti-C3b/PE, anti-CD3/BUV496, CD14/PE-Cy5, CD16/BUV737, CD19/PE-Cy5, CD56/BV786 mAbs. The gating strategy settings were CD3⁻ /CD14⁻CD19⁻/CD16⁺/CD56⁺ in order to access NK staining for Fc, knowing that BT474 cells are CSFE-positive. **B-C**) NK cells were analysed for CD107 degranulation **(B)** and C3b deposits **(C).** While bispecific pseudo-IgGs display similar efficacy for NK activation compared to Trastuzumab, the pseudo-IgG are strong complement activators, in contrast to Trastuzumab. Using a scFv derived from Trastuzumab cloned in the pseudo-IgG scaffold, a "pseudo-Trastuzumab" with strong complement activation activity was created.

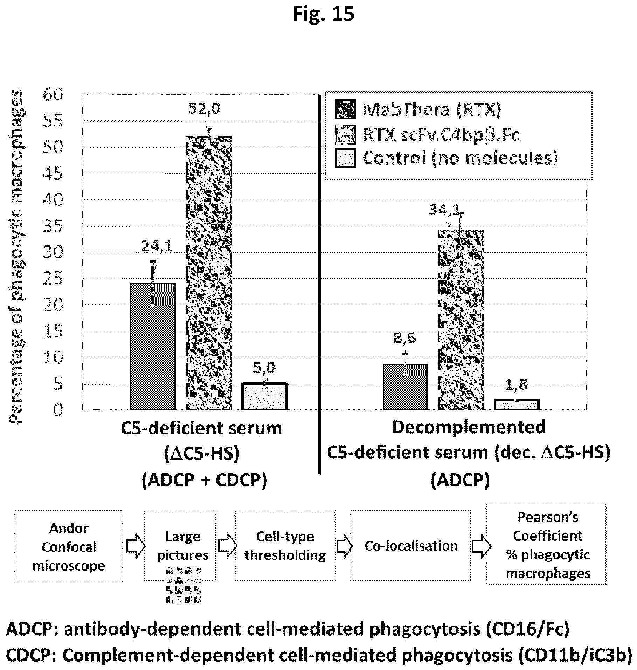

**Fig. 15****.** shows an analysis using Andor spinning-disc confocal microscope of the percentage of phagocytic macrophages, representing the results of antibody-dependent cell-mediated phagocytosis (ADCP when using decomplemented ΔC5-HS, involving IFNyRIIIA/Fc interaction) or ADCP AND complement-dependent cell-mediated phagocytosis (CDCP when using ΔC5-HS, involving both IFNyRIIIA/Fc & CD11b/iC3b interactions) for Daudi cells. Pseudo-RTX is more than 2-time superior to RTX to mediate phagocytosis of Daudi cells by macrophages in the presence of complement, and about 4-time superior to RTX to mediate phagocytosis by macrophages in the absence of complement. Regarding Fc-mediated macrophage-directed phagocytosis of Daudi, pseudo-RTX has a more balanced dual Fc function through both IFNyRIIIA/Fc & CD11b/iC3b interaction compared to RTX.

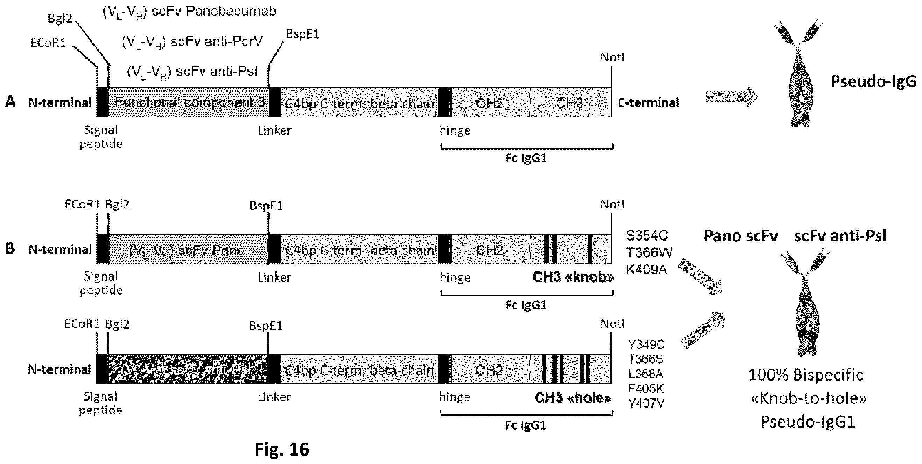

**Fig. 16****.** Design of cassettes and expression vectors, and resulted pseudo-IgG expressed after single (mono-specific) or double (bispecific) transfection in HEK293T cells. The knob-into-hole technology combined to pseudo-IgG allows the expression of 100% bispecific pseudo-IgGs.

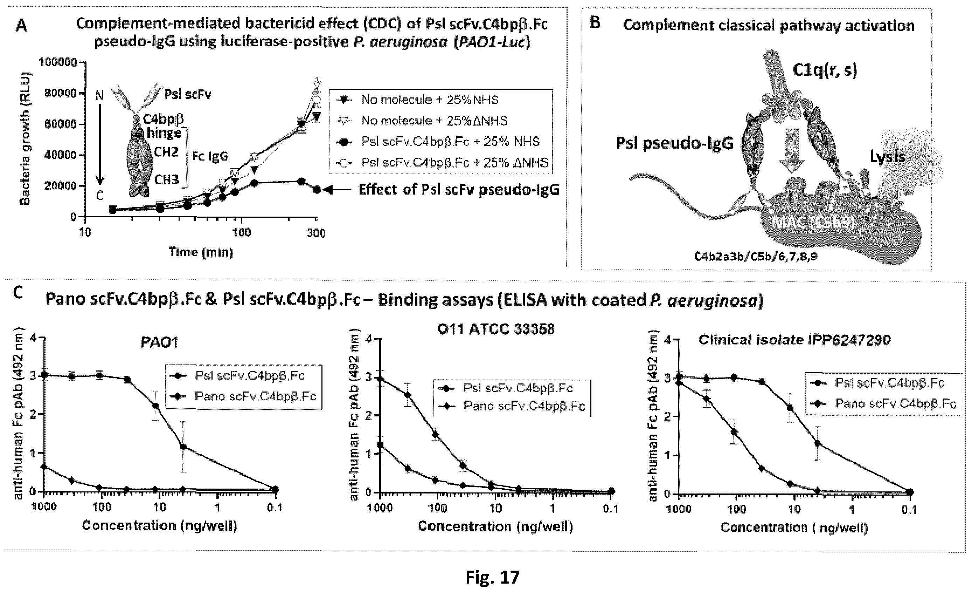

**Fig. 17. A)** Shows the direct bactericidal effect of Psl pseudo-IgG on PAO1-Luciferase *P. aeruginosa* strain. 3 µg of Psl pseudo-IgG is applied to 1.5.10⁵ bacteria/well in 50% NHS or ΔNHS (in GVB⁺⁺ veronal buffer) for 5h. Bacterial growth is measured (luciferase signal in RLU) at different time points. Psl pseudo-IgG, in the presence of 50% NHS, has a strong killing effect preventing bacterial growth. **B)** Depicts the mechanism of action of pseudo-IgG, displaying a strong capacity to recruit C1q which engages the activation of the complement classical pathway. Terminal complement complexes (membrane attack complexes) are ultimately formed, leading to pore formation and membrane disruption and lysis (CDC). **C)** Represents the dose-dependent binding results - using ELISA with non-fixed coated bacteria - of the 2 purified Psl & pano pseudo-IgGs to 3 different *P. aeruginosa* strains: PAO1 reference strain, serotype O11 (ATCC 33358) and the clinical isolate IPP6247290 (tracheal secretions from a tracheostomized patient). Psl pseudo-IgG has a strong binding affinity for PAO1 & IPP6247290, and a low-affinity for O11. Reversely, pano Pseudo-IgG displays only a good affinity for the O11 serotype, which is the main serotype recognized by the original panobacumab.

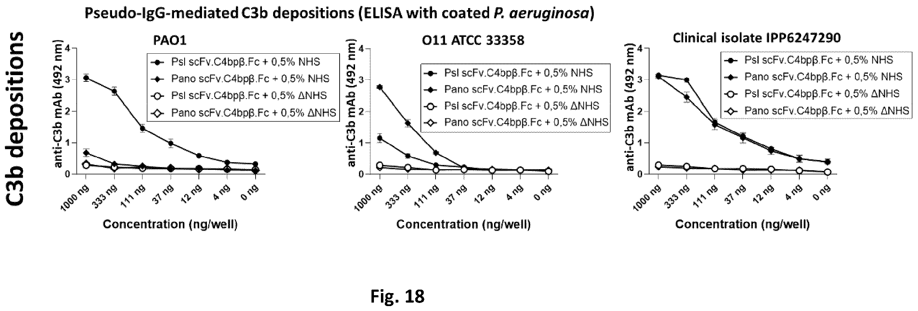

**Fig. 18****.** Shows dose-dependent pano scFv (V_{L}-V_{H}).C4bpβ.Fc pseudo IgG & anti-Psl scFv (V_{L}-V_{H}) C4bpβ.Fc pseudo IgG-mediated C3b deposition on reference strain PAO1 (left) ATCC serotype O11 (middle) & clinical isolate IPP6247290 (right) of *P. aeruginosa.* The results are consistent with the results of dose-response binding and affinity of the 2 pseudo-IgGs in fig. 17C.

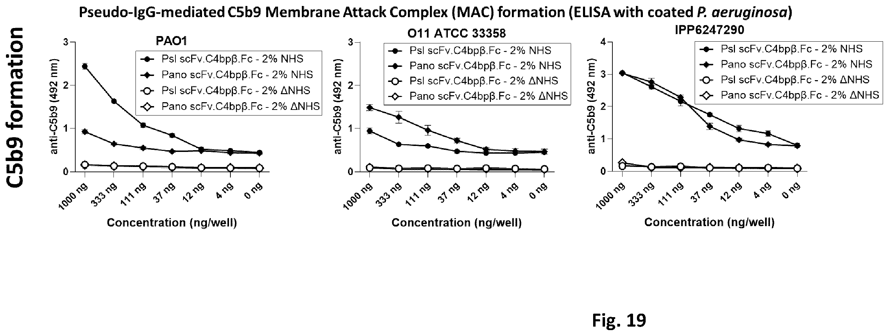

**Fig. 19****.** Shows dose-dependent pano scFv (V_{L}-V_{H}).C4bpβ.Fc pseudo IgG & anti-Psl scFv (V_{L}-V_{H}) C4bpβ.Fc pseudo IgG-mediated C5b9 (MAC) deposition on reference strain PAO1 (left) ATCC serotype O11 (middle) & clinical isolate IPP6247290 (righ) of *P. aeruginosa.* The results are also consistent with the results of dose-response binding and affinity of the 2 pseudo-IgGs and C3b deposition in fig. 17C and Fig. 18, respectively.

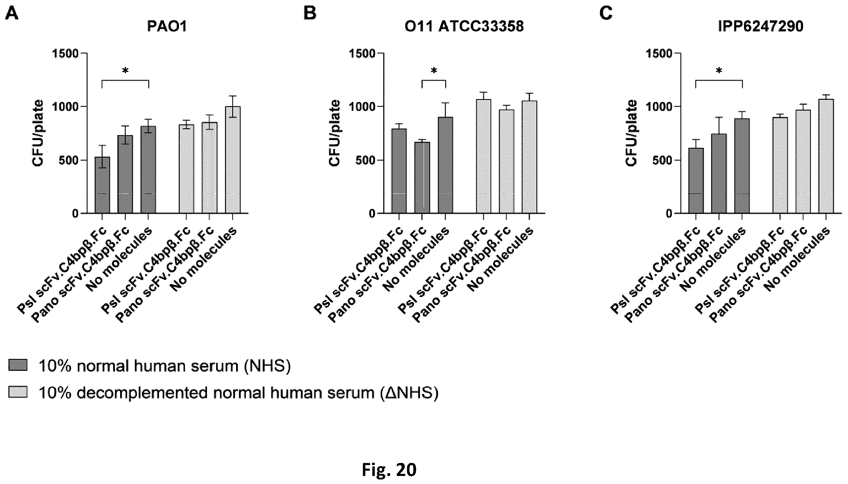

**Fig. 20****.** shows *Panobacumab* scFv (V_{L}-V_{H}).C4bpβ.Fc Pseudo IgG and anti-Psl scFv (V_{L}-V_{H}) C4bpβ.Fc pseudo IgG molecules enhance the complement killing capacity of human serum by 35% (Strain PAO1), 26% (Strain O11) and 31% (clinical isolate IPP6247290).

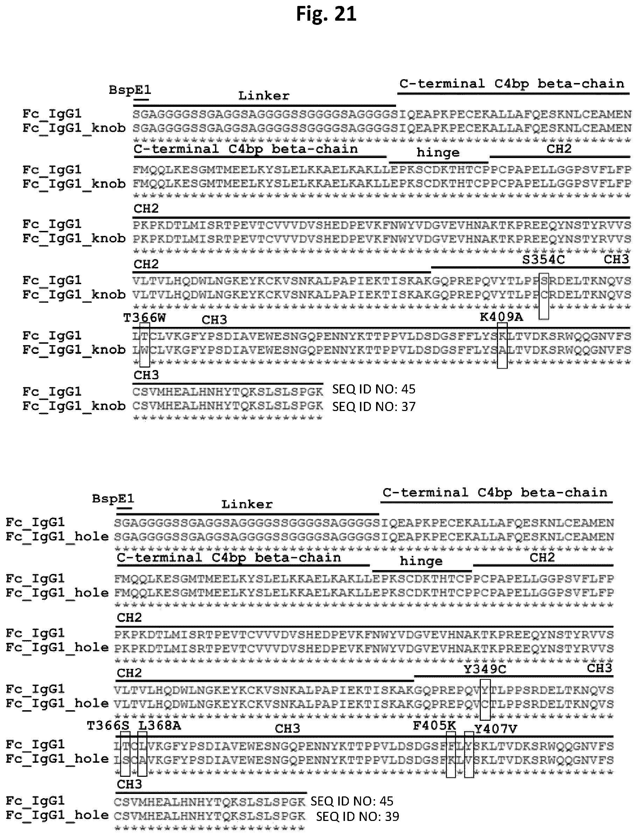

**Fig. 21****.** Shows exemplary amino acid sequences and nucleic acid sequences for the knob-into-hole technology. (A) Sequence codon-optimized for expression in human cells (e.g. HEK293T cells) encoding the linker.C4bpβ.Fc between BspEl (T/CCGGA) and the stop codon **TGA,** followed by a multiple cloning site ending with NotI (GC/GGCCGC), whose CH3 Fc IgG1 displays 3 mutations (S354C, T366W and K409A), also known as Fc[knob]. **(B)** Codon-optimized sequence encoding the linker.C4bpβ.Fc between BspEl and the stop codon **TGA,** followed by a multiple cloning site ending with NotI, whose CH3 Fc IgG1 displays 5 mutations (Y349C, T366S, L368A, F405K and Y407V), also known as Fc[hole]. SEQ ID NO: 45 provides an amino acid sequence of codon-optimized sequence encoding the linker.C4bpbeta.Fc between BspEl and the stop codon TGA, followed by a multiple cloning site ending with NotI.

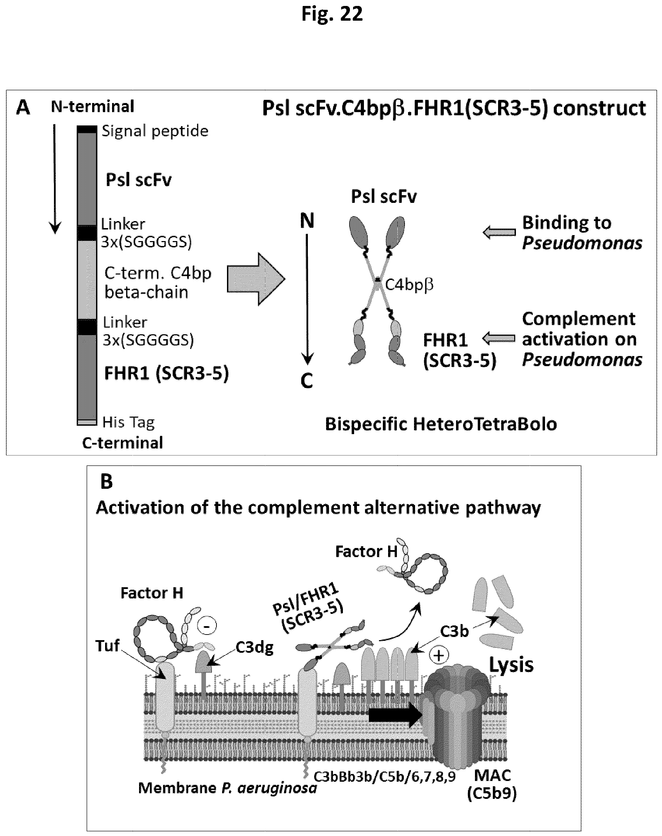

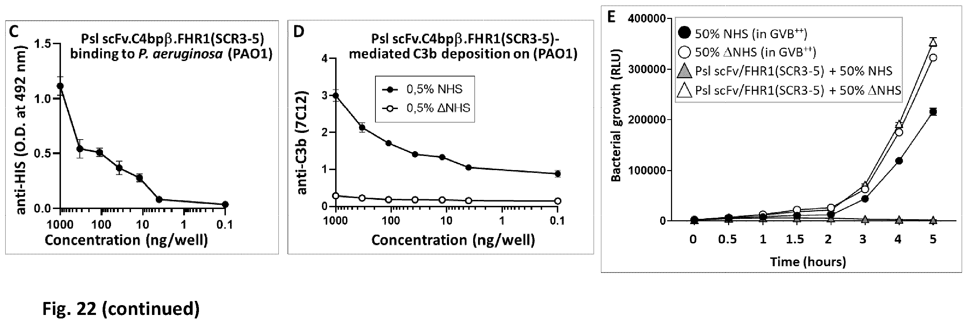

**Fig. 22****.** Depicts a complement-mediated bacteria-targeted destructive strategy using a bifunctional HeteroTetraBolo. Present inventors used the fusion protein consisting of (i) the Psl scFv (V_{L}-V_{H}) and (ii) the last 3 Short Consensus Repeats (SCR3-5) from factor H-related protein 1 (FHR1), cloned upstream and downstream of C4bp C-terminal beta-chain, respectively. **(A)** Exemplary schematic overview of expression cassettes for the expression of Psl/FHR1(SCR3-5) HeteroTetraBolos of which the first and second polypeptide of the dimeric protein complex comprises FHR1 SCR3-5 C-terminally of the C-terminal part of the C4bp beta-chain and the Psl scFv (V_{L}-V_{H}) N-terminally of the C-terminal part of the C4bp beta-chain. Transfection of one of the exemplified expression cassettes allows the formation of Psl/FHR1(SCR3-5) HeteroTetraBolos. **B)** Shows a sketch describing the mechanism of action of Psl/FHR1(SCR3-5) construct. Psl/FHR1(SCR3-5) construct binds to *P. aeruginosa* through its Psl binding moiety. At the targeted bacteria surface, FHR1(SCR3-5) C-terminal effector moiety of the HeteroTetraBolos competes with hijacked bound factor H (FH) for C3b, which deregulated FH-mediated complement decay. As result, FH deregulation leads locally to activation of complement alternative pathway (AP) without engaging the complement classical pathway (CP)/lectin pathway (LP). The ultimate MAC formation disrupts the bacterial membrane, which leads to bacterial lysis. C) Shows the dose-dependent binding of Psl/FHR1(SCR3-5) molecules to PAO1 *P. aeruginosa* cells. 1.5.10⁵ bacteria/well were immobilized onto MaxiSorp^{™} 96-well flat-bottom polystyrene 96-well *ELISA* plates. After blocking a serial dilution of Psl scFv/FHR1(SCR3-5) molecules were added. The bound molecules were revealed with 100ng/well of mouse anti-HIS HRP-conjugated detection monoclonal antibody (SIGMA). **D)** Illustrates the effect of Psl scFv/FHR1(SCR3-5) on C3b deposition and complement activation. 1.5.10⁵ bacteria/well were immobilized and incubated with a serial dilution of Psl scFv/FHR1(SCR3-5) molecules. 0.5% of normal human serum (NHS) or decomplemented human serum (ΔNHS) was added for 30 minutes. C3b-deposition was measured using a mouse anti-human C3/C3b/iC3b mAb (clone 7C12) followed by a goat anti-mouse IgG HRP-conjugated revelation antibody. The addition of Psl scFv/FHR1(SCR3-5) molecules increased C3b deposition, therefore complement activation on PAO1 *P. aeruginosa* cells. **E)** Shows the direct bactericidal effect of Psl/FHR1(SCR3-5) purified construct on PAO1-Luciferase *P. aeruginosa* strain. Three µg of Psl/FHR1(SCR3-5) molecule is applied to 1.5.10⁵ bacteria/well in 50% NHS or decomplemented NHS (ΔNHS) (in GVB⁺⁺ veronal buffer) for 5h. Bacterial growth is measured (luciferase signal in RLU) at different time points. Psl scFv/FHR1(SCR3-5) molecule, in the presence of 50% NHS, has a strong killing effect, preventing bacterial growth. The last three SCRs (SCR3-5) from FHR1 containing the binding domain for C3b that activates the complement alternative pathway are sufficient to elicit locally an efficient FH deregulation and subsequent killing of bacteria.

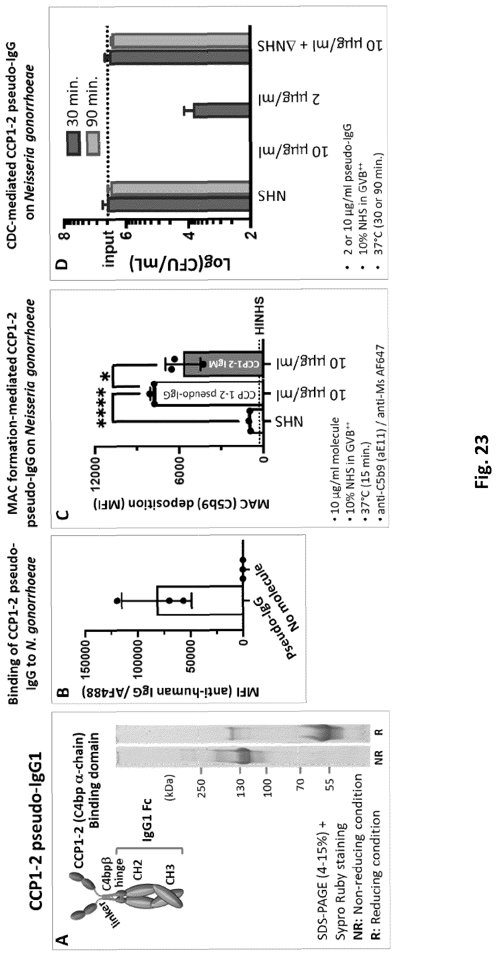

**Fig. 23****.** Depicts a pseudo-IgG that displays the first two N-terminal "complement control proteins" (CCP1-2) or "short consensus repeats (SCR1-2) from the C4bp alpha-chain. **A)** Present inventors used CCP1-2 as anchoring moiety - instead a classical antibody fragment (e.g. scFv, V_{H}H) - that was cloned in the pseudo-IgG scaffold (CCP1-2.C4bpβ.Fc or CCP1-2 pseudo-IgG). Purified CCP1-2 pseudo IgG has been analyzed using SDS-PAGE (4-15% acrylamide gradient gel) under non-reducing (NR) and reducing (R) conditions, followed by a SYPRO RUBY Staining. The apparent MW of CCP1-2 pseudo-IgG is 120 kDa and 60 kDa under NR & R conditions, respectively. **B)** The binding capacity of the CCP1-2 pseudo-IgG to *N. gonorrhoeae* was tested using FACS analysis. Bacteria coated with CCP1-2 pseudo-IgG at 10 µl (or control bacteria) were incubated with a secondary goat anti-human IgG/AF648 pAb. The graph shows the specific binding to *Neisseria gonorrhoeae.* C) FACS analysis of CCP1-2 pseudo-IgG-mediated MAC formation in the presence of 10% NHS or ΔNHS. The results show the CCP1-2 pseudo-IgG-mediated strong MAC formation. Using ΔNHS in the presence of the same concentration of CCP1-2 pseudo-IgG, MAC formation was completely abolished. **D)** FACS analysis of CCP1-2 pseudo-IgG-mediated bacteria killing in the presence of 10% NHS for 30 or 90 min. At 2 µl/ml and 30 min. incubation, 50% reduction growth was observed. Increasing the incubation time of factor 3 led to a complete abrogation of the bacterial growth. At 10 µl/ml, the bacteria growth was completely abrogated after 30 min. When the serum is decomplemented, no activity was observed. The use of NHS alone has no effect on the bacterial growth. This experiment shows that this new pseudo-IgG displaying CCP1-2 from the C4bp N-terminal alpha-chain has a strong bactericidal effect that is linked to selective, local directed complement-mediated killing. In contrast to antibodies that have mostly only a single target, this CCP1-2 pseudo-IgG can potentially target all pathogens that evade complement-mediated killing through the recruitment of C4bp soluble complement regulatory proteins. Present inventors have thus created here a multi-target pseudo-IgG.

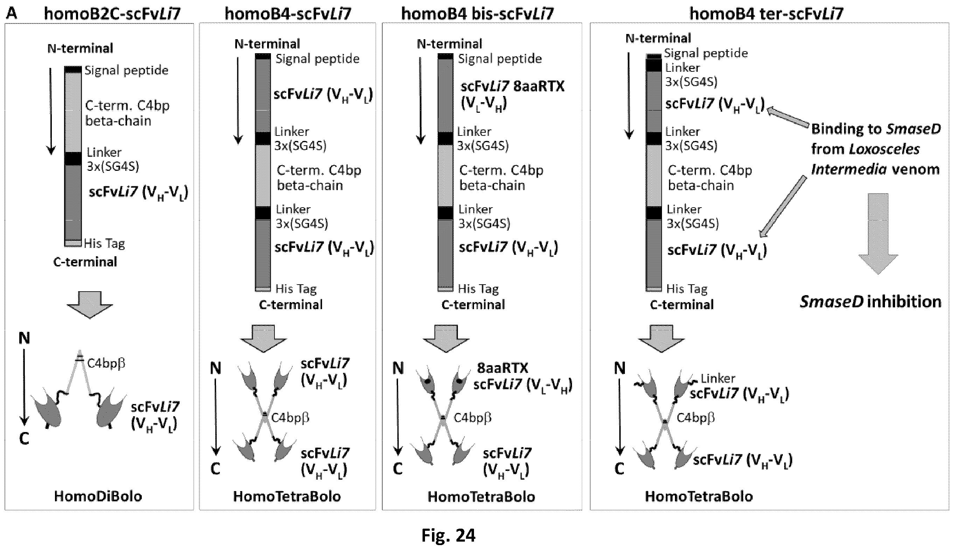

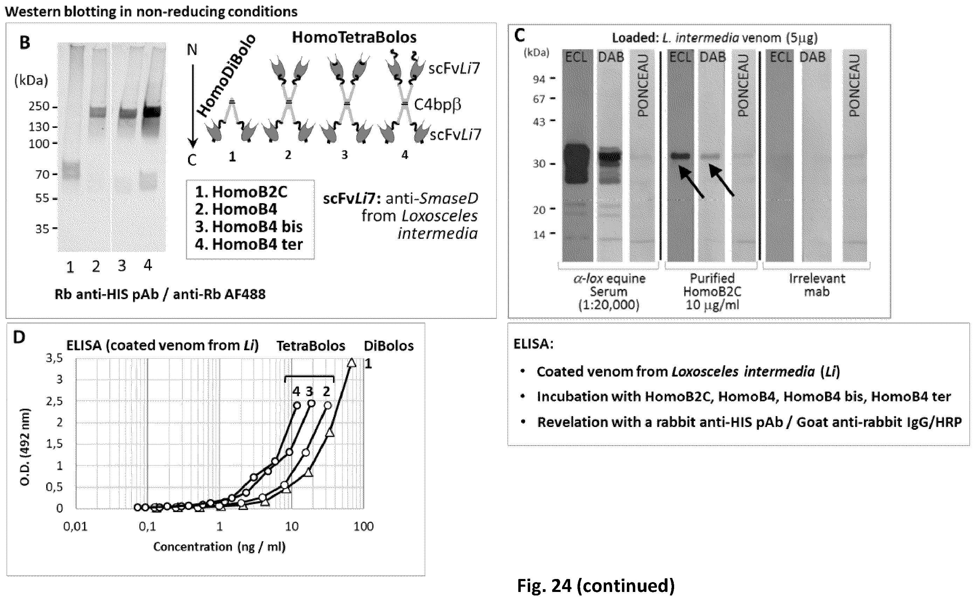

**Fig. 24** illustrates **A)** HomoDiBolo (homoB2C-**scFv*Li*7**) and HomoTetraBolos (homoB4-**scFv*Li*7**) of the scFv anti-sphingomyelinase D (*SMaseD*) from *Loxosceles intermedia* (*Li*) **scFv*Li*7** (V_{H}-V_{L}) generated in HEK293 cells. **HomoDiBolo "HomoB2C":** The gene encoding *scFvLi7* was cloned downstream of C-terminal C4bp beta-chain in a non-natural "upside down" position. No gene was present upstream of the C-terminal C4bp beta-chain in this construct. The HomoDiBolos displayed 2 *scFvLi7* valences. **HomoTetraBolo "HomoB4":** The gene encoding *scFvLi7* was cloned upstream and downstream of C-terminal C4bp beta-chain in natural and upside down positions, respectively. The HomoTetraBolos display 4 *scFvLi7* valences. To increase the protein expression, two variants of homoB4 (referred to herein as homoB4 bis & ter) were generated. For **homoB4 bis,** the original scFvLi7 (V_{H}-V_{L}) upstream of the C-terminal C4bp beta-chain dimerization scaffold was reverted to V_{L}-V_{H}, and the first eight amino acids of V_{L} scFvLi7 (DIVMTQSP (SEQ ID NO: 46)) were replaced by those of rituximab V_{L} (QIVLSQSP (SEQ ID NO: 5)). For **homoB4 ter,** the (AASGGGGSSGGGGSSGGGGS: SEQ ID NO: 47) linker was introduced between the signal peptide and the beginning of original scFvLi7 (V_{H}-V_{L}). **B)** HomoB2C, HomoB4, HomoB4 bis & HomoB4 ter were analyzed using SDS-PAGE under non-reducing conditions, followed by a Western blotting with revelation with a rabbit anti-HIS pAb, and a secondary goat anti-rabbit IgG AF488-conjugated. The Western blot was revealed using a Typhoon Imager. The size of HomoDiBolo (HomoB2C) is 75 kDa, while the size of HomoTetraBolo (HomoB4) is 150 kDa. The introduction of a short linker after the peptide signal increases the yields of expression. **C)** Specific binding of purified HomoB2C to *SmaseD* analyzed using SDS-PAGE and WB (non-reducing conditions) of *Loxosceles intermedia* venom (5 µg/well). Revelation with (i) a horse anti-*SmaseD* Li serum (*α-lox*) (positive control), (ii) purified scFvLi7-HomoB2C (10 µg/ml), or with (iii) an irrelevant mAb (negative control). Revelation with ECL (20-second exposure), DAB and the nitrocellulose membrane was also stained with rouge Ponceau. A band at apparent MW 32-35 kDa was revealed by both HomoB2C and the immunized horse serum, which corresponds to the MW of *SmaseD* from different *Loxosceles* genders (31-34 kDa) (G.J Binford et al. Mol Biol Evol. 2009, 26(3); 547-566). Conclusion: A scFv that is cloned downstream of C-terminal C4bp beta-chain in a non-natural "upside down" position remains functional able to bind its target. **D)** Dose-response binding of HomoB4, HomoB2C using venom from *Li* coated to an ELISA plate. The ELISA plate was blocked and then incubated with serial dilutions of HomoB2C, HomoB4, HomoB4 bis and HomoB4 ter, concentrations expressed as ng/ml. The ELISA plate was revealed with a rabbit anti-His pAb and a goat anti-rabbit IgG HRP-conjugated pAb. The OPD/H₂O₂ chromogenic substrate for HRP revealed the ELISA plate. The concentrations of the molecules that are in Dulbecco's Modified Eagle's medium (DMEM) complete medium were determined using a HIS-HIS enzyme-linked immunosorbent assay (ELISA).

### DESCRIPTION OF EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of' and "consisting essentially of", which enjoy well-established meanings in patent terminology.

The recitation of numerical ranges by endpoints includes all numbers and fractions subsumed within the respective ranges, as well as the recited endpoints. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

Recombinant biomolecules are often used in biotechnology to produce, for example, vaccines, diagnostic kits, or therapeutic molecules. Such recombinant biomolecules may comprise antigens, antibodies or antibody fragments (scFv, V_{H}H), enzymes, hormones, cytokines, growth factors, agonist or antagonist ligands to modulate an immune response or checkpoint inhibitors. Many of these biological molecules require an assembly as to be functional or to display an optimal biological activity. To produce therapeutic dimeric molecules, there is a necessity of using methods allowing the production of dimeric recombinant therapeutics whose association takes place spontaneously without requiring the use of additional step of dimerization that may require the use of immunogenic molecules.

The present inventors found that the C-terminal part of the C4bp beta-chain can be used as a scaffold for dimerization and that functional components can be introduced C-terminally (i.e. downstream) and optionally N-terminally of said C-terminal part of the C4bp beta-chain.

The present invention allows the spontaneous expression of dimeric protein complexes whose (i) dimeric association takes place in the export machinery of eukaryotic cells, (ii) whose assembled dimers are secreted into the cell culture supernatants, and (iii) whose dimerization scaffold (i.e. the C-terminal fragment of the C4bp beta-chain) is derived from a natural circulating human molecule. In the present invention, a gene encoding a protein or polypeptide of interest can be cloned downstream of the C-terminal dimerization scaffold of the C4bp beta- chain, which is a non-natural position, as the natural C4bp beta-chain displays no protein or polypeptide on its C-terminal end. The introduction of a protein or polypeptide of interest in a non-natural location of the C-terminal part of the C4bp β-chain, unexpectedly resulted in the spontaneous dimerization of the fusion protein - in a similar manner as a protein or polypeptide of interest located in the natural N-terminal end of the C-terminal part of the C4bp β-chain. The cloning of a gene encoding a protein or polypeptide of interest in this location is particularly convenient when the biological activity of the protein or polypeptide encoded by the gene is located in its C-terminal end, such as monomeric Fc. The cloning of such gene upstream of the C4bp beta-chain would likely prevent or decrease the activity of the resulting dimeric protein complex because of the proximity of the biological function of the protein or polypeptide of interest and the dimerization scaffold. In contrast, when such protein encoded by the gene of interest is cloned downstream of the C-terminal fragment of the C4bpβ chain, as in present invention, its biological function is in a distal location from the dimerization scaffold and is thus preserved.

Moreover, to the inventor's surprise, cloning of a gene encoding a protein or polypeptide of interest having in its natural (wild-type) form a domain exerting a biological function/activity, such as a binding domain, located at its N-terminal end, such as for instance in scFv or V_{H}H polypeptides, downstream (i.e. C-terminally) of the C-terminal fragment of the C4bpβ chain, as in present invention, does not result in said biological function/ activity of the protein or polypeptide being reduced or eliminated. Moreover, the dimerization of the dimerization scaffold is preserved. An advantage thereof is that such protein or polypeptide, like scFv or V_{H}H, can be included both N- and C-terminally of the dimerization scaffold of C4bp beta, resulting in dimeric protein complexes with a high valence (e.g. such as up to 4 valences) of said protein or polypeptide.Present invention encompasses multifunctional dimeric protein complexes, which can be produced by co-transfecting cells with two expression vectors each encoding a distinct functional component fused C-terminally to the C-terminal part of the C4bpβ chain, and not comprising a functional component fused N-terminally to the N-terminal part of the C4bpβ chain (e.g. first plasmid: N-term-C4bpβ.functional component A-C-terminal; second plasmid: N-term-C4bpβ.functional component B -C-terminal). In such case, three molecular species are released by the cells in the cell culture supernatants: the AA "HomoDiBolos", BB "HomoDiBolos" and the AB bifunctional "heteroDiBolos". The dimeric protein complexes can be purified afterwards (Figures 1 and 2). Present invention also encompasses dimeric protein complexes comprising both C-terminally and N-terminally of the C-terminal part of the C4bp beta-chain a functional component, also referred to in this specification as HomoTetraBolos or HeteroTetraBolos, the former can be mono-functional or bifunctional (which can be produced using single transfection), and the latter can be Tri- or Tetra-functional (which can be produced by a co-transfection of 2 plasmids) (Figures 1 and 2). Moreover, the present invention encompasses dimeric protein complexes comprising a monomeric Fc as a functional component C-terminally of the C-terminal part of the C4bp beta-chain. The molecular weight of such dimeric protein complexes- and thus extravascular distribution space - is of the same range as an IgG, but may be displaying 4 valences for a Homo-TetraBolo, whose consequently functional avidity is far superior to that of an antibody.

In conclusion, the dimeric protein complexes of present invention are of particular interest for use as therapeutics, as they have approximately the size of an antibody, are robust as a result of covalent interchain association between the two C-terminal parts of the C4bp beta-chain, and can be easily cloned with high expression yields.

Accordingly, a first aspect provides a dimeric protein complex comprising
a first polypeptide comprising a first functional component and the C-terminal fragment of the C4bp beta-chain, wherein said first functional component is coupled to the C-terminus of the C-terminal fragment of the C4bp beta-chain; and
a second polypeptide comprising a second functional component and the C-terminal fragment of the C4bp beta-chain, wherein said second functional component is coupled to the C-terminus of the C-terminal fragment of the C4bp beta-chain;
wherein said first and second polypeptides are identical or different.

In particular embodiments, the dimeric protein complex is non-naturally occurring, meaning that the dimeric protein complex is recombinantly produced.

The term "protein" as used throughout this specification generally encompasses macromolecules comprising one or more polypeptide chains, i.e., polymeric chains of amino acid residues linked by peptide bonds. The term may encompass naturally, recombinantly, semi-synthetically or synthetically produced proteins. The term also encompasses proteins that carry one or more co- or post-expression-type modifications of the polypeptide chain(s), such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes protein variants or mutants which carry amino acid sequence variations vis-à-vis a corresponding native proteins, such as, e.g., amino acid deletions, additions and/or substitutions. The term contemplates both full-length proteins and protein parts or fragments, e.g., naturally-occurring protein parts that ensue from processing of such full-length proteins.

The term "polypeptide" as used throughout this specification generally encompasses polymeric chains of amino acid residues linked by peptide bonds. Hence, especially when a protein is only composed of a single polypeptide chain, the terms "protein" and "polypeptide" may be used interchangeably herein to denote such a protein. The term is not limited to any minimum length of the polypeptide chain. The term may encompass naturally, recombinantly, semi-synthetically or synthetically produced polypeptides. The term also encompasses polypeptides that carry one or more co- or post-expression-type modifications of the polypeptide chain, such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes polypeptide variants or mutants which carry amino acid sequence variations vis-à-vis a corresponding native polypeptide, such as, e.g., amino acid deletions, additions and/or substitutions. The term contemplates both full-length polypeptides and polypeptide parts or fragments, e.g., naturally-occurring polypeptide parts that ensue from processing of such full-length polypeptides.

The term "peptide" as used throughout this specification preferably refers to a polypeptide as used herein consisting essentially of 50 amino acids or less, e.g., 45 amino acids or less, preferably 40 amino acids or less, e.g., 35 amino acids or less, more preferably 30 amino acids or less, e.g., 25 or less, 20 or less, 15 or less, 10 or less or 5 or less amino acids.

The reference to any peptides, polypeptides, proteins or nucleic acids may particularly encompass such peptides, polypeptides, proteins or nucleic acids with a native sequence, i.e., ones of which the primary sequence is the same as that of the peptides, polypeptides, proteins or nucleic acids found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to somatic mutations, or post-transcriptional or post-translational modifications. Any such variants or isoforms of peptides, polypeptides, proteins or nucleic acids are intended herein. Accordingly, all sequences of peptides, polypeptides, proteins or nucleic acids found in or derived from nature are considered "native".

In certain embodiments, the peptides, polypeptides, proteins or nucleic acids may be human, i.e., their primary sequence may be the same as a corresponding primary sequence of or present in naturally occurring human peptides, polypeptides, proteins or nucleic acids. In certain embodiments the qualifier "human" relates to the primary sequence of the respective peptides, polypeptides, proteins or nucleic acids, rather than to their origin or source. For example, such peptides, polypeptides, proteins or nucleic acids may be present in or isolated from samples of human subjects or may be obtained by other means (e.g., by recombinant expression, cell-free transcription or translation, or non-biological nucleic acid or peptide synthesis).

In certain embodiments, the peptides, polypeptides, proteins or nucleic acids may be wild-type. While most native peptides, polypeptides, proteins or nucleic acids may be considered wild-type, those carrying naturally-occurring mutations leading to partial or complete loss of function, which may contribute to or be causative of a disease phenotype, are generally excluded from the scope of the term "wild-type".

The reference to any peptides, polypeptides, proteins or nucleic acids may also encompass variants or fragments of such peptides, polypeptides, proteins or nucleic acids, particularly of naturally-occurring, native or wild-type forms thereof.

The term "variant" of a protein, polypeptide, peptide or nucleic acid generally refers to proteins, polypeptides or peptides the amino acid sequence of which, or nucleic acids the nucleotide sequence of which, is substantially identical (i.e., largely but not wholly identical) to the sequence of the protein, polypeptide, peptide, or nucleic acid, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical to the sequence of the recited protein, polypeptide, peptide, or nucleic acid. Preferably, a variant may display such degrees of identity to a recited protein, polypeptide, peptide or nucleic acid when the whole sequence of the recited protein, polypeptide, peptide or nucleic acid is queried in the sequence alignment (i.e., overall sequence identity). Sequence identity may be determined using suitable algorithms for performing sequence alignments and determination of sequence identity as know per se. Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62 (Henikoff et al., 1992, Proc. Natl. Acad. Sci., 89:10915-10919), cost to open a gap = 11, cost to extend a gap = 1, expectation value = 10.0, word size = 3).

An example procedure to determine the percent identity between a particular amino acid sequence and the amino acid sequence of a query polypeptide will entail aligning the two amino acid sequences using the Blast 2 sequences (Bl2seq) algorithm, available as a web application or as a standalone executable programme (BLAST version 2.2.31+) at the NCBI web site (www.ncbi.nlm.nih.gov), using suitable algorithm parameters. An example of suitable algorithm parameters include: matrix = Blosum62, cost to open a gap = 11, cost to extend a gap = 1, expectation value = 10.0, word size = 3). If the two compared sequences share homology, then the output will present those regions of homology as aligned sequences. If the two compared sequences do not share homology, then the output will not present aligned sequences. Once aligned, the number of matches will be determined by counting the number of positions where an identical amino acid residue is presented in both sequences. The percent identity is determined by dividing the number of matches by the length of the query polypeptide, followed by multiplying the resulting value by 100. The percent identity value may, but need not, be rounded to the nearest tenth. For example, 78.11, 78.12, 78.13, and 78.14 may be rounded down to 78.1, while 78.15, 78.16, 78.17, 78.18, and 78.19 may be rounded up to 78.2. It is further noted that the detailed view for each segment of alignment as outputted by Bl2seq already conveniently includes the percentage of identities.

A variant of a protein, polypeptide, peptide or nucleic acid may be a homologue (e.g., orthologue or paralogue) of said protein, polypeptide, peptide or nucleic acid. As used herein, the term "homology" generally denotes structural similarity between two macromolecules from same or different taxons, wherein said similarity is due to shared ancestry.

A variant of a protein, polypeptide, or peptide may comprise one or more amino acid additions, deletions, or substitutions relative to (i.e., compared with) the corresponding protein or polypeptide, such as conservative amino acid substitutions or non-conservative amino acid substitutions relative to (i.e., compared with) the corresponding protein or polypeptide. A variant of a nucleic acid may comprise one or more nucleotide additions, deletions, or substitutions relative to (i.e., compared with) the corresponding nucleic acid.

As described elsewhere in the present specification, C4bp is a normal plasma protein having an octopus-like structure made of seven branching α-chains and one central β-chain. Each chain contains short consensus repeat (SCR). Each SCR of about 60 amino acids includes two intra-chain disulfide bridges. The C-terminal parts of the C4bp alpha- and beta-chains both lack biological functions, the former being responsible for the polymerization of the molecule in the cytoplasm of C4bp producing cells, the latter being responsible for the covalent association of a single beta-chain to the heptameric core of the C4bp glycoprotein through the formation of 2 disulfide bonds with 2 cysteines of C-terminal C4bp alpha-chains. The β-chain subunit is not required for the oligomerization of the α-chains.

The C-terminal fragment of the C4bp beta-chain is able to spontaneously dimerize into a dimeric protein complex in the absence of C4bp alpha-chains. Accordingly, the person skilled in the art will understand that the first and second polypeptide as referred to herein refer to the two components of the dimeric protein complex that are expressed as separate polypeptides and, after expression, spontaneously join together by covalent or intermolecular bonds. The term homodimer is used when the first and second polypeptide are identical. The term heterodimer is used when the first and second polypeptide are not identical.

In particular embodiments, the C-terminal fragment of the C4bp beta-chain comprised by the first polypeptide has the capacity to dimerize with the C-terminal fragment of the C4bp beta-chain comprised by the second polypeptide. The C-terminal fragment of the C4bp beta-chain comprised by the first polypeptide and the C-terminal fragment of the C4bp beta-chain comprised by the second polypeptide are preferably dimerized by a covalent association.

In particular embodiments, the C-terminal fragment of the C4bp beta-chain comprises at least one, preferably at least two, cysteine residues. For example, one of the cysteines of the C-terminal fragment of the C4bp beta-chain may be mutated.

In particular embodiments, the C-terminal fragment of the C4bp beta-chain is the 194-252 fragment of the C4bp beta-chain, or a functional variant conserving the capacity to form an at least dimeric protein.

In particular embodiments, the functional variant of the C-terminal fragment of the C4bp beta-chain, preferably the 194-252 fragment of the C4bp beta-chain, is:
a modified sequence of the 194-252 fragment of the C4bp beta-chain, wherein less than 25% of the amino acids of the 194-252 fragment, preferably less than 10%, have been cut out or replaced, in which the cysteines located in positions 202 and 216 as well as at least 3 amino acids upstream and downstream of each cysteine have been conserved;
a modified sequence of the 194-252 fragment of the C4bp beta-chain, in which a cysteine responsible for dimerization is substituted with an amino acid selected from alanine, valine, phenylalanine, proline, methionine, isoleucine, leucine and tryptophan, and another amino acid of the fragment is substituted with a cysteine;
a sequence of the 194-252 fragment of the C4bp beta-chain modified by insertion of a sequence which is heterologous to the C4bp beta-chain, between the cysteines responsible for dimerization; or a sequence of the 194-252 fragment of the C4bp beta-chain modified by cutting out amino acids between the cysteines responsible for dimerization.

In particular embodiments, the C-terminal fragment of the C4bp beta-chain is a C-terminal fragment of the human C4bp beta-chain. By means of example, the human C4bp beta-chain may comprise an amino acid sequence as annotated under NCBI Genbank accession number NP_001017367.1 (isoform 1 precursor), and Uniprot (www.uniprot.org) accession number P20851.1.

In particular embodiments, the C-terminal fragment of the C4bp beta-chain comprises, consists essentially of or consists of the amino acid sequences as set forth in SEQ ID NO: 1 (IQEAPKPECEKALLAFQESKNLCEAMENFMQQLKESGMTMEELKYSLELKKAELKAKLL); or an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 1, or a functional fragment thereof (e.g. a fragment allowing dimerization of the C-terminal fragment of the C4bp beta-chain). The amino acid sequence as set forth in SEQ ID NO: 1 corresponds to amino acids 194 to 252 of the amino acid sequence of the human C4bp beta-chain as annotated under NCBI Genbank accession number NP_001017367.1 (isoform 1 precursor).

In particular embodiments, the C-terminal fragment of the C4bp beta-chain comprising, consisting essentially of or consisting of consists essentially of an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 1 is a functional variant of the C-terminal fragment of the C4bp beta-chain

In particular embodiments, the dimeric protein complex, the first polypeptide and/or the second polypeptide do not comprise the C4bp alpha-chain or a fragment thereof. For example, the first and second polypeptide do not comprise the C-terminal fragment of the C4bp alpha-chain. By means of example, the precursor of human C4bp alpha-chain comprises an amino acid sequence as annotated under NCBI Genbank accession number NP_000706.1, and Uniprot (www.uniprot.org) accession number P04003.2.

The C-terminal fragment of the C4bp beta-chain is sufficient to achieve spontaneous dimerization, preferably covalent dimeric association. For example, the C-terminal fragment of the C4bp beta-chain may comprise at least two cysteines allowing spontaneous interchain covalent dimeric association of two C-terminal fragments of the C4bp beta-chain in the cellular export machinery.

To allow to obtain dimeric protein complexes of the invention of which the size is as small as possible, the fragments of the C4bp beta-chain that are not required to achieve spontaneous dimerization may be deleted. Accordingly, in particular embodiments, the first and second polypeptide do not comprise the N-terminal fragment of the C4bp beta-chain. In more particular embodiments, the first and second polypeptide do not comprise the amino acid sequences as set forth in SEQ ID NO: 2 (MFFWCACCLMVAWRVSASDAEHCPELPPVDNSIFVAKEVEGQILGTYVCIKGYHLVGKKTL FCNASKEWDNTTTECRLGHCPDPVLVNGEFSSSGPVNVSDKITFMCNDHYILKGSNRSQCLED HTWAPPFPICKSRDCDPPGNPVHGYFEGNNFTLGSTISYYCEDRYYLVGVQEQQCVDGEWSS ALPVCKL); or a fragment thereof.

In particular embodiments, the dimeric protein complex is a homodimer. Or in other words, in particular embodiments, the first polypeptide and the second polypeptide of the dimeric protein complex are identical.

In particular embodiments, the dimeric protein complex is a heterodimer. Or in other words, in particular embodiments, the first polypeptide and the second polypeptide of the dimeric protein complex are not identical (i.e. different from each other).

In particular embodiments, the first polypeptide and the second polypeptide of the dimeric protein complex are identical.

In particular embodiments, the first polypeptide and the second polypeptide of the dimeric protein complex are not identical (i.e. different from each other).

In the context of present invention, the term "coupled" as used herein is synonymous with "connected", "bound", "fused", "joined" and refers to a physical link between at least two elements or components.

In particular embodiments, the first or second functional component is coupled to the C-terminus of the C-terminal fragment of the C4bp beta-chain by a peptide-bond.

Coupling of two proteins may be achieved by any methods known in the art, such as by expressing a nucleic acid comprising a nucleic acid encoding the C-terminal fragment of the C4bp beta-chain and a nucleic acid encoding the first or second functional component, wherein said nucleic acid encoding the first or second functional component is located 3' of the nucleic acid encoding the C-terminal fragment of the C4bp beta-chain, as explained elsewhere in the present specification.

In particular embodiments, the first functional component is located immediately C-terminally of the C-terminal fragment of the C4bp beta-chain of the first polypeptide of the dimeric protein complex and/or the second functional component is located immediately C-terminally of the C-terminal fragment of the C4bp beta-chain of the second polypeptide of the dimeric protein complex. Alternatively, a linker, such as a peptide linker, may be included between the C-terminus of the C-terminal fragment of the C4bp beta-chain and the N-terminus of the first or second functional component.

As used herein, the term "linker" refers to a connecting element that serves to link other elements. The linker may be a rigid linker (also referred to in the present specification as a spacer) or a flexible linker. In particular embodiments, the linker is a covalent linker, achieving a covalent bond. The terms "covalent" or "covalent bond" refer to a chemical bond that involves the sharing of one or more electron pairs between two atoms. For many molecules, the sharing of electrons allows each atom to attain the equivalent of a full outer electron shell, corresponding to a stable electronic configuration. Covalent bonds include different types of interactions, including σ-bonds, π-bonds, metal-to-metal bonds, agostic interactions, bent bonds and three-center two-electron bonds.

In particular embodiments, the linker is a (poly) peptide linker or a non-peptide linker, such as a non-peptide polymer, such as a non-biological polymer. Preferably, the linkage(s) between the C-terminal fragment of the C4bp beta-chain and a functional component may be hydrolytically stable linkage(s), i.e., substantially stable in water at useful pH values, including in particular under physiological conditions, for an extended period of time, e.g., for days. In particular embodiments, the linker is a peptide linker of one or more amino acids.

More particularly, the peptide linker may be 1 to 50 amino acids long or 2 to 50 amino acids long or 1 to 45 amino acids long or 2 to 45 amino acids long, preferably 1 to 40 amino acids long or 2 to 40 amino acids long or 1 to 35 amino acids long or 2 to 35 amino acids long, more preferably 1 to 30 amino acids long or 2 to 30 amino acids long. Further preferably, the linker may be 5 to 25 amino acids long or 5 to 20 amino acids long. Particularly preferably, the linker may be 5 to 15 amino acids long or 7 to 15 amino acids long. Hence, in certain embodiments, the linker may be 1, 2, 3 or 4 amino acids long. In other embodiments, the linker may be 5, 6, 7, 8 or 9 amino acids long. In further embodiments, the linker may be 10, 11, 12, 13 or 14 amino acids long. In still other embodiments, the linker may be 15, 16, 17, 18 or 19 amino acids long. In further embodiments, the linker may be 20, 21, 22, 23, 24 or 25 amino acids long. In certain embodiments, the linker is 4-10 or 5-9 or 6-8 or 7 amino acids long. In other embodiments, the linker is 12-18 or 13-17 or 14-16 or 15 amino acids long.

The nature of amino acids constituting the linker is not of particular relevance so long as the biological activity of the polypeptide segments linked thereby is not substantially impaired and the linker provides for the intended spatial separation of the C-terminal fragment of the C4bp beta-chain and a functional component. Preferred linkers are essentially non-immunogenic and/or not prone to proteolytic cleavage.

In certain preferred embodiments, the peptide linker may comprise, consist essentially of or consist of amino acids selected from the group consisting of Glycine, Serine, Alanine, Threonine, and combinations thereof. In even more preferred embodiments, the linker may comprise, consist essentially of or consist of amino acids selected from the group consisting of Glycine, Serine, and combinations thereof. Such linkers provide for particularly good flexibility. In certain embodiments, the linker may consist of only Glycine residues. In certain embodiments, the linker may consist of only Serine residues.

In particular embodiments, the linker is a flexible linker comprising, consisting essentially of or consisting of an amino acid sequence (SGGGGS)ₙ (SEQ ID NO: 7), wherein n is an integer from 1 to 11, preferably 2 to 7, even more preferably 3 to 7, such as 3, 5 or 7, even more preferably 5 or 7. In particular embodiments, the linker is a flexible linker comprising, consisting essentially of or consisting of an amino acid sequence 3x (SGGGGS) (SEQ ID NO: 3), 5x (SGGGGS) (SEQ ID NO: 48), or 7x (SGGGGS) (SEQ ID NO: 49). The person skilled in the art will understand that the flexible linker and the size thereof may be optimized depending on the one or more functional component being linked to the C-terminal part of the C4bp beta-chain.

In particular embodiments, the linker is a spacer comprising, consisting essentially of or consisting of an amino acid sequence RDCDPPGNPVHGYFEGNNFTLGSTISYYCEDRYYLVGVQEQQCVDGEWSSALPVCKL (SEQ ID NO: 4). Such spacer corresponds to the SCR3 from the C4bp beta-chain which displays 4 cysteine residues folded by the presence of two internal disulphide bridges. The SCR3 has no biological function and is the natural spacer of the C4bp beta-chain to move the first two SCRs away from each other to the dimerization scaffold.

Present inventors have found that by including the peptide QIVLSQSP (SEQ ID NO: 5) C-terminally of the signal peptide (preferably immediately 3' of the signal peptide) and N-terminally of the C-terminal fragment of the C4bp beta-chain, and optionally N-terminally of the third or fourth functional component, an increased expression could be obtained of the first or second polypeptide as described herein. The peptide as defined by SEQ ID NO: 5 is derived from Rituximab (RTX). The peptide as defined by SEQ ID NO: 5 is located N-terminally of the Rituximab hypervariable domain of light chain (RTX VL). This sequence is located N-terminally of the framework 1 (FR1) of VL of RTX, away from (or outside of) the downstream "complementary Determining region 1 (CDR1) of the VL (involved in the recognition part of the paratope), FR1 region being located upstream of CDR1.

Accordingly, in particular embodiments, the first polypeptide comprises C-terminally of a signal peptide and N-terminally of the C-terminal fragment of the C4bp beta-chain, and if a third functional component is present, N-terminally of said third functional component, a peptide QIVLSQSP (SEQ ID NO: 5). In particular embodiments, the second polypeptide comprises C-terminally of a signal peptide and N-terminally of the C-terminal fragment of the C4bp beta-chain, and if a fourth functional component is present, N-terminally of said fourth functional component, a peptide QIVLSQSP (SEQ ID NO: 5).

Preferably, the peptide as defined by SEQ ID NO: 5 is used in combination with a functional component being a scFv, such as Panobacumab. The scFv may have the structure [VH-linker-VL] or the reversed structure [VL-linker-VH]. The N-terminal site of the scFv VL (e.g. the first 8 amino acids) is preferably replaced by the peptide defined by SEQ ID NO: 5.

In particular embodiments, the peptide defined by SEQ ID NO: 5 is located immediately C-terminally of the signal peptide. Preferably, the signal peptide ends with a Bgl2 restriction site, more preferably with the amino acid sequence RS. Accordingly, the first and second polypeptide may comprise N-terminally the sequence "signal peptide-RS-QIVLSQSP (SEQ ID NO: 6)". Such a sequence was found by present inventors to confer high expression yields of the dimeric protein complex.

In particular embodiments, said first polypeptide does not comprise a functional component that is not a tag N-terminally of said C-terminal fragment of the C4bp beta-chain and/or said second polypeptide does not comprise a functional component that is not a tag N-terminally of said C-terminal fragment of the C4bp beta-chain. Such dimeric protein complexes may be referred to herein as "DiBolos". Such DiBolos may be used to test whether a functional component fused C-terminally of said C-terminal fragment of the C4bp beta-chain is effectively functional prior to establishing a dimeric protein complex also comprising functional components N-terminally of said C-terminal fragment of the C4bp beta-chain as described elsewhere in the specification. DiBolos in which the first and second functional components are identical may be referred to in the present specification as homoDiBolos, and DiBolos in which the first and second functional components are different may be referred to in the present specification as heteroDiBolos.

In particular embodiments, said first polypeptide and/or said second polypeptide comprises more than one, such as two or three, functional components, N-terminally and/or C-terminally of said C-terminal fragment of the C4bp beta-chain. Accordingly, in particular embodiments, said first polypeptide and/or said second polypeptide comprises at least one, such as at least two or at least three, functional components N-terminally and/or C-terminally of said C-terminal fragment of the C4bp beta-chain.

In particular embodiments, said first polypeptide further comprises a third functional component that is coupled to the N-terminus of said C-terminal fragment of the C4bp beta-chain; and/or said second polypeptide further comprises a fourth functional component that is coupled to the N-terminus of said C-terminal fragment of the C4bp beta-chain, wherein said first, said second, said third and/or said fourth functional components are identical or different. A dimeric protein complex comprising the first, second, third and fourth functional component may also be referred to in the present specification as "TetraBolos".

In particular embodiments, the third functional component is located immediately N-terminally of the C-terminal fragment of the C4bp beta-chain of the first polypeptide of the dimeric protein complex and/or the fourth functional component is located immediately N-terminally of the C-terminal fragment of the C4bp beta-chain of the second polypeptide of the dimeric protein complex. Alternatively, a linker, such as a peptide linker, may be included between the N-terminus of the C-terminal fragment of the C4bp beta-chain and the C-terminus of the third or fourth functional component, such as described elsewhere in the present specification.

In particular embodiments, said first polypeptide of the dimeric protein complex comprises
a linker N-terminally of the C-terminal fragment of the C4bp beta-chain and C-terminally of the first functional component; and
a linker C-terminally of the C-terminal fragment of the C4bp beta-chain and N-terminally of the third functional component, preferably wherein the linker is a flexible linker, such as flexible linker comprising, consisting essentially of or consisting of an amino acid sequence SGGGGS as defined by SEQ ID NO: 7, or a spacer, such as a spacer comprising, consisting essentially of or consisting of an amino acid sequence RDCDPPGNPVHGYFEGNNFTLGSTISYYCEDRYYLVGVQEQQCVDGEWSSALPVCKL as defined by SEQ ID NO: 4.

In particular embodiments, said second polypeptide of the dimeric protein complex comprises
a linker N-terminally of the C-terminal fragment of the C4bp beta-chain and C-terminally of the second functional component; and
a linker C-terminally of the C-terminal fragment of the C4bp beta-chain and N-terminally of the fourth functional component, preferably wherein the linker is a flexible linker, such as flexible linker comprising, consisting essentially of or consisting of an amino acid sequence as defined by SEQ ID NO: 7, or a spacer, such as a spacer comprising, consisting essentially of or consisting of an amino acid sequence as defined by SEQ ID NO: 4.

The dimeric protein complexes as taught herein may be mono-, bi-, tri- or tetra-functional, depending on the amount of different functional components present in the dimeric protein complexes.

In particular embodiments, the first, second, third and fourth functional components are identical. For example, the first, second, third and fourth functional components may be a functional component A. Accordingly, in such embodiment, the first and second polypeptide of the dimeric protein complex as taught herein each comprise functional components A-A. Such dimeric protein complexes may be referred to in the present specification as monofunctional HomoTetraBolos.

In particular embodiments, the first and second functional components are identical, the third and fourth functional components are identical, and the first and third components are not identical (or different). For example, the first and second functional component may be a functional component A and the third and fourth functional components may be a functional component B, wherein A and B represent different functional components. Accordingly, in such embodiment, the first and second polypeptide of the dimeric protein complex as taught herein each comprise functional components A-B. Such dimeric protein complexes may be referred to in the present specification as bifunctional HomoTetraBolos. An example of such bifunctional HomoTetraBolo may be a dimeric protein complex comprising complementary scFvs for given strains of *Pseudomonas.*

The term "different" when referring to the functional components include differences in their primary amino acid sequence as well as being differences in their post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

In particular embodiments, at least three of the first, second, third and fourth functional components are different (meaning that two of the first, second, third and fourth functional components may be the same). For example, the first and second functional components may each be a functional component A, the third functional component may be a functional component B and the fourth functional component may be a functional component C, wherein A, B and C represent different functional components. For example, the third and fourth functional components may each be a functional component A, the first functional component may be a functional component B and the second functional component may be a functional component C, wherein A, B and C are different functional components. Such dimeric protein complexes may be referred to in the present specification as trifunctional HeteroTetraBolos (Figure 1).

In particular embodiments, all of the first, second, third and fourth functional components are different. For example, the first functional component may be a functional component A, the first functional component may be a functional component B, the third functional component may be a functional component C and the fourth functional component may be a functional component D, wherein A, B, C and D represent different functional components. Such dimeric protein complexes may be referred to in the present specification as tetrafunctional HeteroTetraBolos .

In particular embodiments the first, second, third and/or fourth functional components may be selected independently of each other.

The term "functional component" as used herein refers to an element, more particularly an amino acid sequence which can exert a given function. In present context, three main functions are distinguished: effector function, targeting function, and tracking function, but the functional components to be used in present invention are not to be considered to be limited thereto. The person skilled in the art will select functional component(s) in function of the desired application of the dimeric protein complexes as taught herein. For example, the functional component may be an enzyme (e.g. *SmaseD* from the venom from *Loxosceles intermedia* (Li)), an enzymatic activity regulating factor, a receptor ligand, a hapten, an antigen, an antibody, an antibody fragment (e.g. scFv or V_{H}H), a prophylactic or therapeutic agent (such as immune response regulators (e.g. FHR1 (i.e. FHR-1) or fragments thereof, such as SCR3, 4 and 5 from FHR1), immune checkpoint inhibitors, immunotoxins, antioxidants, antibiotics, growth factors, hormones, cytokines (e.g. IL-2, IL-15, INF gamma or TNF alpha), oncolytic or cytotoxic agents such as toxins and pro-toxins, neuromediators, antimicrobioal agents such as antiviral agents, antibacterial agents and antiparasitics, antineoplastic agents or any other therapeutic agent or prophylactic agent of interest), metallic ion from heavy metal, lanthanide, radionucleotide, chemiluminescent molecule, fluorescent molecule, or a receptor such as monochain receptor or a proteic drug acceptor. In particular embodiments, the first, second, third and fourth functional components are not a tag, as described elsewhere in the present specification.

In particular embodiments, at least one of the first, second, third and/or fourth functional components is a protein or polypeptide. For example, the first, second, third and/or fourth functional components may be an enzyme, an enzymatic activity regulating factor, a receptor ligand, a hapten, an antigen, an antibody, an antibody fragment (e.g. scFv or V_{H}H), a prophylactic or therapeutic agent (such as immune response regulators, immune checkpoint inhibitors, immunotoxins, antioxidants, antibiotics, growth factors, hormones, cytokines, oncolytic and cytotoxic agents such as toxins and pro-toxins, neuromediators, antimicrobioal agents such as antiviral agents, antibacterial agents and antiparasitics, antineoplastic agents or any other therapeutic agent or prophylactic agent of interest), or a receptor such as monochain receptor or a proteic drug acceptor. The receptor may be a soluble receptor or a fragment thereof. In particular embodiments, the first, second, third and/or fourth functional components, preferably the first and second functional components, more preferably the first, second, third and fourth functional components, are proteins or polypeptides, such as functional proteins or polypeptides.

In particular embodiments, the first, said second, said third and said fourth functional component is a protein or polypeptide, preferably a protein or polypeptide selected from the group consisting of a binding domain (e.g. an antigen recognition domain), a receptor (e.g. a soluble receptor), a recombinant viral structural protein (such as a recombinant viral structural protein for a virus-like particle (VLP)), an oncolytic agent, a cytotoxic agent, a cytokine, a receptor-binding peptide (e.g. a ligand), or monomeric Fc, more preferably a protein or polypeptide selected from the group consisting of a binding domain (e.g. an antigen recognition domain)domain, a receptor (e.g. a soluble receptor), a recombinant viral structural protein (such as a recombinant viral structural protein for a virus-like particle (VLP)), a cytotoxic agent, a cytokine, a receptor-binding peptide (e.g. a ligand), or monomeric Fc, even more preferably selected from the group consisting of a binding domain (e.g. an antigen recognition domain), a recombinant viral structural protein (such as a recombinant viral structural protein for a virus-like particle (VLP)), a cytotoxic agent, a cytokine, a receptor-binding peptide (e.g. a ligand), or monomeric Fc.

Present inventors found that SCR3-5 of factor H-related protein 1 (FHR1), is sufficient to compete with factor H (FH), as described elsewhere in the present specification. Accordingly, in particular embodiments, the first, said second, said third or said fourth functional component, preferably said third and said fourth functional components, comprise, consist essentially of or consist of Short Consensus Repeats 3, 4 and 5 (SCR3, 4 and 5) from factor H-related protein 1 (FHR1), preferably SCR3, 4 and 5 from human FHR1.

In particular embodiments, the first, said second, said third or said fourth functional component, preferably said third and said fourth functional components, comprise, consist essentially of or consist of an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 50 (human SCR3), an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 51 (human SCR4), and an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 52 (human SCR5). In particular embodiments, the first, said second, said third or said fourth functional component, preferably said third and said fourth functional components, comprise, consist essentially of or consist of an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 53 (human SCR3-5).

FHR1(SCR3-5) competes with hijacked bound factor H (FH) for C3b, which deregulated FH-mediated complement decay. As result, FH deregulation leads locally to complement alternative pathway (AP) without activating the complement classical pathway. The ultimate MAC formation disrupts a target cell to which the dimeric protein complex is directed, such as a pathogenic cell like bacteria, which leads to cell lysis. Accordingly, in particular embodiments, if the third and fourth functional components of a dimeric protein complex as taught herein comprise, consist essentially of or consist of SCR3, 4 and 5 of FHR1, the first and second functional components of said dimeric protein complex preferably comprise a functional component with a targeting function, more preferably a binding domain (e.g. an antigen recognition domain) binding a pathogen or tumour cell, preferably a binding domain (e.g. an antigen recognition domain) binding a pathogen evading the human complement attack, such as a scFv PsI binding *P. Aeruginosa* as described elsewhere in the present specification.

In particular embodiments, two of said first, said second, said third or said forth functional components comprise SCR3, 4 and 5 from FHR1; and wherein two of said first, said second, said third or said forth functional components comprise a binding domain (e.g. an antigen recognition domain) specifically binding to a TSA, a TAA, a bacterial antigen, a viral antigen, a virus-associated antigen, or a fungal antigen.

In particular embodiments, the first, said second, said third or said fourth functional component, preferably said third and said fourth functional components do not comprise of Short Consensus Repeats 1 and 2 (SCR1 and 2) from factor H-related protein 1 (FHR1), preferably from human FHR1.

In particular embodiments, the third and/or fourth functional component (i.e. the functional component(s) located N-terminally of the C-terminal fragment of the C4bp beta-chain) are proteins or peptides having a biological function/activity located at their N-terminal end, such as an active receptor able to catch a ligand (e.g. enzyme) or an antibody fragment, preferably a scFv (e.g. the VH-VL or VL-VH orientation) or V_{H}H, more preferably a scFv.

In particular embodiments, the third and/or fourth functional component (i.e. the functional component(s) located C-terminally of the C-terminal fragment of the C4bp beta-chain) are proteins or peptides having a biological function/activity located at their N-terminal end, such as an active receptor able to catch a ligand (e.g. enzyme) or an antibody fragment, preferably a scFv (e.g. the VH-VL or VL-VH orientation) or V_{H}H, more preferably a scFv, and said proteins or peptides are coupled with their N-terminus to the C-terminus of the C-terminal fragment of the C4bp beta-chain. Accordingly, in such embodiments, the C-terminus of said protein or peptide having a biological function/activity located at its N-terminal end is located at the C-terminal end of the first and/or second polypeptide of the dimeric protein complex as taught herein. A linker, such as (SGGGGS)ₙ (SEQ ID NO: 7), wherein n is an integer from 1 to 11, may be present between the C-terminus of the C-terminal fragment of the C4bp beta-chain and the N-terminus of the protein or peptide having a biological function/activity located at its N-terminal end. Therefore, in such embodiments, the N-terminus of the protein or peptide having a biological function/activity located at its N-terminal end which is in its "natural" , unbound form freely accessible, is now fused to another protein or polypeptide.

In particular embodiments, the first, second, third and fourth functional components are not multimeric complexes *per se* but may form a complex with each other to form a multimeric complex.

In particular embodiments, the first, second, third and fourth functional components may be encoded by tandem-associated genes separated by linkers or spacers.

In particular embodiments, the functional component, such as the first and second functional component, comprises, consists essentially of or consists of at least 10, at least 15, at least 20, at least 30, at least 40, at least 50, at least 100, at least 200, at least 300, at least 400, at least 500, at least 600, at least 700, at least 800, at least 900 or at least 1000 amino acids.

In particular embodiments, the first and second polypeptides comprise a protein or polypeptide (e.g. comprising one or more functional components) of at least 10, at least 15, at least 20, at least 30, at least 40, at least 50, at least 100, at least 200, at least 300, at least 400, at least 500, at least 600, at least 700, at least 800, at least 900 or at least 1000 amino acids C-terminally of the C-terminal fragment of the C4bp beta-chain.

In particular embodiments, the functional component, such as the first and second functional component, does not comprise, consist essentially of or consists of, preferably does not consist of a tag. In particular embodiments, the functional component, such as the first and second functional component, does not comprise, consist essentially of or consists of, preferably does not consist of, a protein purification tag, such as a FLAG-tag, His-tag, HA-tag or Myc-tag

In particular embodiments, the first, second, third and/or fourth functional components, preferably the first and second functional components, are not the C4bp alpha or beta-chain or a fragment thereof.

In particular embodiments, the first, second, third and/or fourth functional components, preferably the first and second functional components, are polypeptides or proteins heterologous to the C-terminal fragment of the C4bp beta-chain. A functional component being heterologous to C4bp beta-chain means that the functional component is not naturally associated with the C-terminal fragment of the C4bp beta-chain to which it is coupled.

In particular embodiments, said first, said second, said third and/or said fourth functional component comprises, consists essentially of or consist of an enzyme, enzymatic activity regulating factors, receptor ligands, haptens, antigens, antibodies, antibody fragments, a prophylactic or therapeutic agent, an oncolytic agent, a cytotoxic agent, a cytokine, a recombinant viral structural protein, an antibody-like scaffold, an extracellular domain of a viral envelope protein, a cognate extracellular domain of a receptor or ligand for the antigen (e.g. a signaling molecule) or an antigen-binding portion of said receptor or ligand, a soluble receptor, or a synthetic receptor, preferably an enzyme, enzymatic activity regulating factors, receptor ligands, haptens, antigens, antibodies, antibody fragments, a prophylactic or therapeutic agent, a cytotoxic agent, a cytokine, a recombinant viral structural protein, an antibody-like scaffold, an extracellular domain of a viral envelope protein, a cognate extracellular domain of a receptor or ligand for the antigen (e.g. a signaling molecule) or an antigen-binding portion of said receptor or ligand, a soluble receptor, or a synthetic receptor.

Unless otherwise apparent from the context, reference herein to any peptide or polypeptide or protein, may generally also encompass fragments and/or variants of said peptide, polypeptide or protein.

Where the present specification refers to or encompasses fragments and/or variants of proteins, polypeptides or peptides, this preferably denotes variants and/or fragments which are "functional", i.e., which at least partly retain the biological activity or intended functionality of the respective proteins, polypeptides or peptides (e.g. antigen recognizing properties). Preferably, a functional fragment and/or variant may retain at least about 20%, e.g., at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared to the corresponding protein, polypeptide or peptide. For example, the extracellular domain of a viral envelope protein may also be a fragment thereof, the soluble receptor may also be a fragment thereof, or the synthetic receptor may also be a fragment thereof.

In particular embodiments, said first, said second, said third and/or said fourth functional component comprises, consists essentially of or consist of a binding domain (e.g. an antigen recognition domain).

The term "antigen" or "Ag" as used herein is defined as a molecule capable of being bound by an antigen recognition domain, such as capable being bound to an antibody or receptor (e.g. T-cell receptor). An antigen can be found on the cell surface of a target cell, such as a pathogen or tumor cell.

The term "antigen recognition domain" or "binding domain" or "antigen-specific binding domain" as used herein refers to the domain of the functional component that binds to a specific target molecule. An antigen recognition domain may bind to a single target molecule or to multiple target molecules. A binding domain includes any naturally occurring, synthetic, semi-synthetic, or recombinantly produced binding partner for a target molecule. A binding domain should not be considered to be limited to antibodies or fragments thereof, but may also include other molecules, such as receptors.Present inventors found that the use of short consensus repeats 1 and 2 (SCR1-2) (also known as complement control proteins 1 and 2 (CCP1-2)) from the C4bp alpha-chain, preferably from the human C4bp alpha-chain, as binding domain (e.g. antigen recognition domain) allows to target a wide variety of pathogens, such as those that evade complement-mediated killing through the recruitment of C4bp soluble complement regulatory proteins, including but not limited to, bacterial pathogens *(e.g. Bordetella pertussis* & *burgdorferi, Haemophilus influenza, Moraxella catarrhalis, Neisseria gonorrhoeae & meningitidis, Streptococcus pneumoniae* & *pyogenes, Yersinia enterocolitica and Staphylococcus aureus*) as well as fungi (*Candida dubliensis, Aspergillus fumigatus & terreus*)*.* Accordingly, a dimeric protein complex as taught herein comprising C-terminally of the C-terminal fragment of the C4bp beta-chain SCR1 and SCR2 from the C4bp alpha-chain and N-terminally of the C-terminal fragment of the C4bp beta-chain monomeric IgG acts as a multi-target pseudo-IgG with a high broad-spectrum therapeutic use. Preferably, the C4bp alpha-chain is the human C4bp alpha-chain.

In particular embodiments, the binding domain (e.g. antigen recognition domain) comprises, consists essentially of or consists of SCR1 and SCR2 (also known as CCP1 and CCP2) from the C4bp alpha-chain, preferably the human C4bp alpha-chain (e.g. human C4bp alpha-chain with Uniprot accession number P04003.2).

In particular embodiments, the binding domain (e.g. antigen recognition domain) comprises, consists essentially of or consists of an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 54 (SCR1) and an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 55 (SCR2). In particular embodiments, the binding domain (e.g. antigen recognition domain) comprises, consists essentially of or consists of an amino acid sequence having at least 70%, at least 75% , preferably at least 80%, at least 85%, at least 90%, more preferably at least 95%, such as at least 96%, at least 97%, at least 98%, at least 99%, or 100%, sequence identity to SEQ ID NO: 56 (SCR1-2).

In particular embodiments, the binding domain (e.g. antigen recognition domain ) does not comprise CCP3, CCP4, CCP5, CCP6, CCP7 and CCP8 , ... of the C4bp alpha-chain, preferably human C4bp alpha chain (e.g. human C4bp alpha-chain with Uniprot accession number P04003.2).

In particular embodiments, the antigen recognition domain (e.g. extracellular antigen recognition domain) is derived from an antibody or an antibody fragment. The term "antibody" is used herein in its broadest sense and generally refers to any immunologic binding agent, such as a whole antibody, including without limitation a chimeric, humanized, human, recombinant, transgenic, grafted and single chain antibody, and the like, or any fusion proteins, conjugates, fragments, or derivatives thereof that contain one or more domains that selectively bind to an antigen of interest. The term antibody thereby includes a whole immunoglobulin molecule, a monoclonal antibody, a chimeric antibody, a humanized antibody, a human antibody, or an immunologically effective fragment of any of these. The term thus specifically encompasses intact monoclonal antibodies, polyclonal antibodies, multivalent (e.g., 2-, 3- or more-valent) and/or multi-specific antibodies (e.g., bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced *in vitro,* in cell culture, or *in vivo.* The term "antibody fragment" or "antigen -binding moiety" comprises a portion or region of a full length antibody, generally the antigen binding or variable domain thereof. Examples of antibody fragments include Fab, Fab', F(ab)2, Fv, scFv fragments, single domain (sd)Fv, such as V_{H} domains , V_{L} domains and V_{H}H domains, diabodies, linear antibodies, single-chain antibody molecules, in particular heavy-chain antibodies; and multivalent and/or multispecific antibodies formed from antibody fragment(s), e.g., dibodies, tribodies, and multibodies. The above designations Fab, Fab', F(ab')2, Fv, scFv etc. are intended to have their art-established meaning.

A full-length antibody as it exists naturally is an immunoglobulin molecule comprising 2 heavy (H) chains and 2 light (L) chains interconnected by disulfide bonds. The amino terminal portion of each chain includes a variable region of about 100-110 amino acids primarily responsible for antigen recognition via the complementarity determining regions (CDRs) contained therein. The carboxy-terminal portion of each chain defines a constant region primarily responsible for effector function.

The CDRs are interspersed with regions that are more conserved, termed framework regions (FR). Each light chain variable region (LCVR) and heavy chain variable region (HCVR) is composed of 3 CDRs and 4 FRs, arranged from amino-terminus to carboxy-terminus in the following order: FR1, CDR1, FR2, CDR2, FR3, CDR3, FR4. The 3 CDRs of the light chain are referred to as "LCDR1, LCDR2, and LCDR3" and the 3 CDRs of the heavy chain are referred to as "HCDR1, HCDR2, and HCDR3." The CDRs contain most of the residues which form specific interactions with the antigen. The numbering and positioning of CDR amino acid residues within the LCVR and HCVR regions is in accordance with the well-known Kabat numbering convention, which refers to a system of numbering amino acid residues which are more variable (i.e., hypervariable) than other amino acid residues in the heavy and light chain regions of an antibody (Kabat, et al., Ann. NYAcad. Sci. 190:382-93 (1971 ); Kabat, et al., Sequences of Proteins of Immunological Interest, Fifth Edition, U.S. Department of Health and Human Services, NIH Publication No. 91-3242 (1991 )). The positioning of CDRs in the variable region of an antibody follows Kabat numbering or simply, "Kabat."

Light chains are classified as kappa or lambda, and are characterized by a particular constant region as known in the art. Heavy chains are classified as gamma, mu, alpha, delta, or epsilon, and define the isotype of an antibody as IgG, IgM, IgA, IgD, or IgE, respectively. IgG antibodies can be further divided into subclasses, e.g., IgG1, IgG2, IgG3, IgG4. Each heavy chain type is characterized by a particular constant region with a sequence well known in the art.

In certain embodiments, an antibody may be any of IgA, IgD, IgE, IgG and IgM classes, and preferably IgG class antibody.

In particular embodiments, the antigen recognition domain (e.g. extracellular antigen recognition domain) comprises, consists essentially of, or consists of the antigen-binding region of an antibody or an antibody fragment.

The antibody or antibody fragment may not only recognise a specific target, such as a specific receptor, enzyme, or toxin, but may also be able to activate (i.e. agonize) or inhibit (i.e. antagonize) said specific target.

For example, if one or more of functional components comprises, consists essentially of or consists of scFv anti*-SmaseD* from the venom from *Loxosceles intermedia* (Li), the dimeric protein complex may bind to the *SmaseD* and inhibit its activity.

For example, if one or more of functional components comprises, consists essentially of or consists of *SmaseD* from the venom from *Loxosceles intermedia* (Li) and one or more functional components comprise an antigen-binding portion targeting a cancer cell (e.g. scFv anti-HER2), the dimeric protein complex may bind to the target cell and kill the target cell by the accumulation of *SmaseD* at its cell surface, hence, exerting a tumor-suppressor activity. *SmaseD* transforms sphingomyelin to ceramide-1-phosphate. In situ ceramide-1-phosphate generation by *SmaseD* enzymatic activity alters the lateral structure and morphology of the target membrane, and can be considered a tumor-suppressor lipid.

The term "antigen-binding portion" or "antigen-binding region" refers to one or more fragments of an antibody, such as a particular site, part, domain or stretch of amino acid residues, that retain the ability to specifically bind to an antigen of interest. It has been shown that the antigen-binding function of an antibody may be performed by fragments of a full-length antibody. These may be bispecific, dual specific, or multi-specific formats; specifically binding to two or more different antigens. Examples of binding fragments encompassed within the term "antigen-binding portion" of an antibody include (i) a Fab fragment, a monovalent fragment consisting of the V_{L}, V_{H}, C_{L} and CHI domains; (ii) a F(ab')2 fragment, a bivalent fragment comprising two Fab fragments linked by a disulfide bridge at the hinge region; (iii) a Fd fragment consisting of the V_{H} and CHI domains; (iv) a Fv fragment consisting of the V_{L} and V_{H} domains of a single arm of an antibody, (v) a dAb fragment (Ward et al., Nature, 341 : 544-546 (1989); PCT publication WO 90/05144), which comprises a single variable domain; and (vi) an isolated complementarity determining region (CDR). Furthermore, although the two domains of the Fv fragment, V_{L} and VH, are coded for by separate genes, they may be joined, using recombinant methods, by a synthetic linker that enables them to be made as a single protein chain in which the V_{L} and V_{H} regions pair to form monovalent molecules (known as single chain Fv (scFv) (Bird et al., Science, 242: 423-426 (1988); and Huston et al., Proc. Natl. Acad. Sci., 85: 5879-5883 (1988)). Such single chain antibodies are also intended to be encompassed within the term "antigen-binding portion" of an antibody. Other forms of single chain antibodies, such as diabodies are also encompassed. Diabodies are bivalent, bispecific antibodies in which V_{H} and V_{L} domains are expressed on a single polypeptide chain, but using a linker that is too short to allow for pairing between the two domains on the same chain, thereby forcing the domains to pair with complementary domains of another chain and creating two antigen binding sites (Holliger, et al., Proc. Natl. Acad. Sci., 90: 6444-6448 (1993); Poljak, et al., Structure 2: 1121-1123 (1994)). Such antibody binding portions are known in the art (Kontermann and Dubel eds., Antibody Engineering (2001) Springer- Verlag. New York. 790 pp. (ISBN 3-540-41354-5). In addition, the term "sequence" as used herein (for example in terms like "variable domain sequence", "V_{H}H sequence" or "protein sequence"), should generally be understood to include both the relevant amino acid sequence as well as nucleic acid sequences or nucleotide sequences encoding the same, unless the context requires a more limited interpretation.

In particular embodiments, the antigen-binding region of an antibody or an antibody fragment specifically binds to an antigen of interest, such as a TSA or TAA, as described elsewhere in this specification.

The term "specifically bind" means that an agent (denoted herein also as "binding agent" or "specific-binding agent") binds to one or more desired targets (e.g., peptides, polypeptides, proteins, nucleic acids, or cells) substantially to the exclusion of other entities which are random or unrelated, and optionally substantially to the exclusion of other molecules that are structurally related. The term "specifically bind" does not necessarily require that an agent binds exclusively to its intended target(s). For example, an agent may be said to specifically bind to target(s) of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold, or at least about 1000-fold, or at least about 10⁴-fold, or at least about 10⁵-fold, or at least about 10⁶-fold or more greater, than its affinity for a non-target.

The binding or interaction between the agent and its intended target(s) may be covalent (i.e., mediated by one or more chemical bonds that involve the sharing of electron pairs between atoms) or, more typically, non-covalent (i.e., mediated by non-covalent forces, such as for example, hydrogen bridges, dipolar interactions, van der Waals interactions, and the like). Preferably, the agent may bind to or interact with its intended target(s) with affinity constant (K_{A}) of such binding K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A} ≥ 1×10¹⁰ M⁻¹ or K_{A} ≥ 1×10¹¹ M⁻¹, wherein K_{A} = [A_T]/[A][T], A denotes the agent, T denotes the intended target. Determination of K_{A} can be carried out by methods known in the art, such as for example, using equilibrium dialysis and Scatchard plot analysis.In particular embodiments, the antigen recognition domain (e.g. extracellular antigen recognition domain) comprises, consists essentially of, or consists of proteins or peptides having their biological function/activity located at their N-terminal end, such as an antibody fragment, like a scFv (e.g. the V_{H}-V_{L} or V_{L}-V_{H} orientation) or V_{H}H, preferably a scFv.

In preferred embodiments, the antigen recognition domain (e.g. extracellular antigen recognition domain)comprises, consists essentially of, or consists of a single chain variable fragment of an antibody (scFv).

In further embodiments, the antigen recognition domain (e.g. extracellular antigen recognition domain)may comprise, consist essentially of, or consist of divalent scFv. In dimeric protein complexes as taught herein comprising di-scFvs, two scFvs specific for each antigen are linked together by producing a single peptide chain with two V_{H} and two V_{L} regions, yielding tandem scFvs, such as described in Xiong, C.Y. et al., 2006, Protein Engineering Design and Selection 19 (8): 359-367; Kufer, P. et al., 2004, Trends in Biotechnology 22 (5): 238-244).

scFvs may be obtained using standard recombinant DNA techniques. For example, scFvs may be prepared by the isolation of the coding sequence from a hybridoma producing antibodies, identification of V-chain types and design of a nucleic acid encoding the scFv, as described in Köksal H. et al., 2019, Antibody Therapeutics, 2(2):56-63. The scFv may comprise, consist essentially of, or consist of, the VL sequence, the linker peptide, and the V_{H} sequence, wherein the V_{L} sequence is located N-terminally of the linker peptide, and the linker peptide is located N-terminally of the V_{H} sequence. Different scFv designs are possible, such as wherein the position of the V_{L} and V_{H} sequence is swapped.

In particular embodiments, the functional component may comprise, consist essentially of or consist of a scFv, such as
- a scFv specifically binding NKG2D. The scFv specifically binding NKG2D may be a RTX-hybrid (V_{L}-linker-V_{H}) codon-optimised human MS anti-NKG2D scFv derived from the whole IgG4 described in patent US 9.127.064-B2, which is incorporated herein by reference, spanned between Bgl2 (A/GATCT, amino acids RS) and BspEl (T/CCGGA, amino acids SG) restriction sites. Preferably, the scFv specifically binding *NKG2D* comprises, consists essentially of or consists of an amino acid sequence as defined by SEQ ID NO: 8 and/or a nucleic acid sequence as defined by SEQ ID NO: 9.
- a scFv specifically binding NKG2A. The scFv specifically binding NKG2A may be Z199 codon-optimised (V_{H}-linker-V_{L}) scFv anti-human NKG2A derived from the Z199 humanized anti-human NKG2A monoclonal antibody as described in International patent application WO2009092805 which incorporated herein by reference, a non-competitive antagonist of the CD94/NKG2A receptor, spanned between Bgl2 (A/GATCT, amino acids RS) and BspEl (T/CCGGA, amino acids SG) restriction sites. Preferably, the scFv specifically binding NKG2A comprises, consists essentially of or consists of an amino acid sequence as defined by SEQ ID NO: 10 and/or a nucleic acid sequence as defined by SEQ ID NO: 11.
- a scFv specifically binding KIR. The scFv specifically binding KIR may be a codon-optimised (V_{H}-linker-V_{L}) scFv derived from the fully human IgG4 anti-KIR2DL1/2L3 mAb (Lirilumab (IPH2102); Innate Pharma) spanned between Bgl2 (A/GATCT, amino acids RS) and BspEl (T/CCGGA, amino acids SG) restriction sites. Preferably, the scFv specifically binding KIR comprises, consists essentially of or consists of an amino acid sequence as defined by SEQ ID NO: 12 and/or a nucleic acid sequence as defined by SEQ ID NO: 13.

- a scFv specifically binding SLAMF7. The scFv specifically binding SLAM7 may be a scFv derived from Elotuzumab: RTX-hybrid reversed (V_{L}-linker-V_{H}) codon-optimised SLAMF7 (anti-CD319) scFv from humanized IgG1 spanned between Bgl2 (A/GATCT, amino acids RS) and BspEl (T/CCGGA, amino acids SG) restriction sites. Preferably, the scFv specifically binding SLAMF7 comprises, consists essentially of or consists of an amino acid sequence as defined by SEQ ID NO: 14 and/or a nucleic acid sequence as defined by SEQ ID NO: 15.
- a scFv specifically binding the CD20 antigen. The scFv specifically binding the CD20 antigen may be a codon-optimized (V_{L}-linker-V_{H}) scFv derived from Rituximab (RTX) specifically binding the CD20 antigen spanned between Bgl2 (A/GATCT, amino acids RS) and BspEl (T/CCGGA, amino acids SG) restriction sites. The scFv specifically binding CD20 may be a codon-optimized (V_{H}-linker-V_{L}) scFv derived from Ofatumumab (OFA) scFv pseudo-IgG specifically binding CD20 spanned between Bgl2 (A/GATCT, amino acids RS) and BspEl (T/CCGGA, amino acids SG) restriction sites. Preferably, the scFv specifically binding the CD20 antigen comprises, consists essentially of or consists of an amino acid sequence as defined by SEQ ID NO: 16 or 18 and/or a nucleic acid sequence as defined by SEQ ID NO: 17 or 19.
- a scFv specifically binding HER2. The scFv specifically binding HER2 may be a Trastuzumab-derived codon-optimized (V_{H}-linker-V_{L}) scFv spanned between Bgl2 (A/GATCT, amino acids RS) and BspEl (T/CCGGA, amino acids SG) restriction sites. The scFv specifically binding HER2 may be a codon-optimized 2D3 V_{H}H specifically binding HER2, as described in International patent application WO2009068625-A2, which is incorporated herein by reference, spanned between Bgl2 (A/GATCT, amino acids RS) and BspEl (T/CCGGA, amino acids SG) restriction sites. The scFv specifically binding HER2 may be a codon-optimized 47D5 V_{H}H specifically binding HER2 as described in International patent application WO2009068625-A2, which is incorporated herein by reference, spanned between Bgl2 (A/GATCT, amino acids RS) and BspEl (T/CCGGA, amino acids SG) restriction sites. Preferably, the scFv specifically binding HER2 comprises, consists essentially of or consists of an amino acid sequence as defined by SEQ ID NO: 20, 22, 24 or, and/or a nucleic acid sequence as defined by SEQ ID NO: 21, 23 or 25.
- a scFv specifically binding Psl of *Pseudomonas aeruginosa.* The scFv specifically binding Psl of *P. aeruginosa* may be a codon-optimized (V_{L}-linker-V_{H}) scFv specifically binding Psl of *P. aeruginosa,* comprising a sequence as defined in SEQ ID NO: 9 of International patent application WO2017095744 A1, which is incorporated herein by reference, and spanned between Bgl2 (A/GATCT, amino acids RS) and BspEl (T/CCGGA, amino acids SG) restriction sites. Preferably, the scFv specifically binding Psl of *P. aeruginosa* comprises, consists essentially of or consists of an amino acid sequence as defined by SEQ ID NO: 26 and/or a nucleic acid sequence as defined by SEQ ID NO: 27.
- a scFv specifically binding PcrV of *P. aeruginosa.* The scFv specifically binding PcrV of *P. aeruginosa* may be a codon-optimized (V_{L}-linker-V_{H}) scFv specifically binding PcrV of *Pseudomonas aeruginosa* as described in International patent application WO2017095744 A1, which is incorporated herein by reference, spanned between Bgl2 (A/GATCT, amino acids RS) and BspEl (T/CCGGA, amino acids SG) restriction sites or a codon-optimized RTX-hybrid reversed (V_{L}-Linker-V_{H}) scFv derived from Panobacumab (Aridis Pharmaceuticals, AR-101) specifically binding *Pseudomonas aeruginosa* spanned between Bgl2 (A/GATCT, amino acids RS) and BspEl (T/CCGGA, amino acids SG) restriction sites. Preferably, the scFv specifically binding PcrV of*P*. *aeruginosa* comprises, consists essentially of or consists of an amino acid sequence as defined by SEQ ID NO: 28 or 30 and/or a nucleic acid sequence as defined by SEQ ID NO: 29 or 31.
- a scFv specifically binding *SMaseD.* The scFv specifically binding *SMaseD* may be a scFv specifically binding *SMaseD* (scFv*Li7*) (V_{H}-Linker-V_{L}) from a mouse anti*-SMaseD* from *Loxosceles intermedia* mAb (LimAb7) as described in Karim-Silva, S., et al., Loxoscelism: Advances and Challenges in the Design of Antibody Fragments with Therapeutic Potential. Toxins (Basel), 2020. 12(4) or Karim-Silva, S., et al., Generation of recombinant antibody fragments with toxin-neutralizing potential in loxoscelism. Immunol Lett, 2016. 176: p. 90-6. The scFv specifically binding *SMaseD* may be codon-optimised scFv anti-*SMase* D (scFv*Li7*) (V_{L}-Linker-V_{H}) from a mouse anti*-SMaseD* from *Loxosceles intermedia* mAb (LimAb7) as described in Karim-Silva, S., et al., Loxoscelism: Advances and Challenges in the Design of Antibody Fragments with Therapeutic Potential. Toxins (Basel), 2020. 12(4) or Karim-Silva, S., et al., Generation of recombinant antibody fragments with toxin-neutralizing potential in loxoscelism. Immunol Lett, 2016. 176: p. 90-6, spanned between Bgl2 (A/GATCT, amino acid sequence RS) and BspEl (T/CCGGA, amino acid sequence SG) restriction sites and comprising the amino acid sequence QIVLSQSP (SEQ ID NO: 5) derived from RTX immediately C-terminally of the signal peptide. Preferably, the scFv specifically binding *SMaseD* comprises, consists essentially of or consists of an amino acid sequence as defined by SEQ ID NO: 58 (Codon-optimized mouse scFv-Li7 (VH-linker-VL) *anti-SmaseD* from *Loxosceles intermedia* (Li)) and/or a nucleic acid sequence as defined by SEQ ID NO: 57 (Codon-optimized mouse scFv-Li7 (VH-linker-VL) anti*-SmaseD* from *Loxosceles intermedia* (Li)).

It is known in the art that NK activation is improved when the NK activating component (e.g. antigen recognition domain specifically binding to and antagonising an activating NK cell receptor is present as a dimer. For instance, the NKG2D receptor consists of a homodimer of two disulfide-linked transmembrane proteins. Each NKG2D monomer interacts with different surfaces of their ligand MICA. Dimeric protein complexes comprising multiple, such as two, anti-NKGD2 scFv will bind to NKG2D-dimeric receptors with much higher efficacy than dimeric protein complexes comprising only one anti-NKGD2 scFv, leading to a greater NK activation. Alternatively, dimeric protein complexes comprising two different scFvs recognising two distinct NK receptors (e.g. NKG2D & SLAMF7) may be used and these scFvs can concomitantly bind to two NK receptors, activating for more efficiently NK cells.

In particular embodiments, the first and second functional components comprise, consist essentially of, or consist of a binding domain (e.g. an antigen recognition domain) specifically binding to an activating NK cell receptor such as a natural cytotoxicity receptor (NCR), natural-killer group 2, member D (NKG2D), NKG2A, killer immunoglobulin-like receptor (KIR), or SLAMF7, and the third and fourth functional components comprise, consist essentially of or consist of sIL15Ralpha. In more particular embodiments, the first and second functional components comprise, consist essentially of, or consist of a scFv anti-NKG2A or a scFv anti-KIR and the third and fourth functional components comprise, consist essentially of or consist of sIL15R-alpha. Preferably, the scFv anti-NKG2A is derived from the specific monoclonal antibody (mAb) Z199. Z199 is a non-competitive agonist of the human CD94/NKG2A receptor, as described in Carretero et al., The CD94 and NKG2-A C-type lectins covalently assemble to form a natural killer cell inhibitory receptor for HLA class I molecules. Eur J Immunol 1997 27:563. Such dimeric protein complexes allow to activate NK cells using a double-approach: the activation through the IL15 pathway and through NK-checkpoint inhibitors presented by two scFv either to NKG2A or to KIR. Therefore, such dimeric protein complexes act as NK superagonists. An equivalent approach is used here of the dimeric protein complexes for the NK cells than that of the checkpoint inhibitors for the CD8 T-cells. The dimeric protein complex inhibits the NKG2A pathway that is a pathway for exhaustion of NK cells through the scFv anti-NKG2A moiety. The IL15 moiety displays selective NK activation.

The dimeric protein complexes may be used to crosslink a NK cell and an unwanted cell (e.g. tumor cell) or a pathogenic microorganism, such as bacteria (e.g. *Pseudomonas aeruginosa*)*,* virus (e.g. SARS-CoV2) or fungus (e.g. Aspergillus), to kill the unwanted cell or pathogenic microorganism. For such purpose, the dimeric protein complexes may display (i) a binding domain (e.g. an antigen recognition domain) specifically binding to an activating NK cell receptor, such as a scFv directed against NKG2D or SLAM7, and (ii) a binding domain (e.g. an antigen recognition domain) specifically binding to a TSA, a TAA, or an antigen of a pathogenic microorganism (e.g. bacterial antigen, viral antigen, viral-associated antigen or fungal antigen).

For example, the dimeric protein complexes may be used to crosslink an NK cell and *Pseudomonas aeruginosa* to kill the *Pseudomonas aeruginosa.* For such purpose, the dimeric protein complexes may display (i) 2 different monomeric NK-activating scFvs directed against 2 NK natural cytotoxicity receptors (NCRs), NKG2D (CD314) and SLAMF7 (CS1, CD319, CRACC), and (ii) a dimeric PcrV or Psl scFv anti*-Pseudomonas aeruginosa* as therapeutics to activate NK cells towards *P. aeruginosa.* The scFv anti-NKG2D can act as an agonist of the NKG2D/DAP10/Grb2/Av1/PI3K/Akt pathway, whereas the scFv anti-SLAMF7 derived from Elotuzumab can act as an agonist of the SLAMF7/ITSM/PLC/Ca⁺⁺/ERK pathway, both pathways activate NK cells as well as enhance ADCC.

Accordingly, in particular embodiments, the first and second functional components comprise, consist essentially of, or consist of a scFv anti-PcrV of *Pseudomonas aeruginosa* or a scFv anti-Psl of *Pseudomonas aeruginosa,* such as described in International patent application WO2017095744 A1, the third functional component comprises, consists essentially of, or consists of a scFv anti-NKG2D and the fourth functional component comprises, consists essentially of, or consists of a scFv anti-SLAM7. Such dimeric protein complexes, as well as any dimeric protein complex comprising a binding domain (e.g. an antigen recognition domain) specifically binding to a TSA, TAA, a bacterial antigen, viral antigen or a virus-associated antigen, or a fungal antigen and further comprising two scFvs (e.g. V_{H}-V_{L} or V_{L}-V_{H} orientation) or V_{H}Hs, each with a different specificity for an activating NK cell receptor, may also be referred to herein as "TriKEs" as "Tri-specific Killer Engagers". Using two different pathways for NK activation dramatically enhances the overall NK-activating potential of TriKEs.

Further, dimeric protein complexes comprising a binding domain (e.g. an antigen recognition domain) specifically binding to a TSA, TAA, a bacterial antigen, viral antigen or a virus-associated antigen, or a fungal antigen and further comprising one scFv (e.g. V_{H}-V_{L} or V_{L}-V_{H} orientation) or V_{H}H with a specificity for an activating NK cell receptor, may also be referred to herein as "BiKEs" as "Bi-specific Killer Engagers".

When all functional components within the dimeric protein complex are scFv, a blocking scFv with a high affinity can be obtained as dimeric protein complexes as taught herein may comprise 4 valences of said scFv. An example of such a dimeric protein complex, is a dimeric protein complex in which the first and second functional component or the first, second, third and fourth functional components comprise a scFv anti*-sphingomyelinase D* (*SMase D*) from the spider venom *Loxosceles intermedia* (scFvLi7).

In preferred embodiments, the antigen recognition domain (e.g. extracellular antigen recognition domain) comprises, consists essentially of, or consists of a single domain variable fragment of a heavy chain antibody (V_{H}H) specific for the antigen or a Nanobody^{®}.

The terms "Nanobody^{®}" and "Nanobodies^{®}" are trademarks of Ablynx NV (Belgium). The term "Nanobody" is well-known in the art and as used herein in its broadest sense encompasses an immunological binding agent obtained (1) by isolating the V_{H}H domain of a naturally occurring heavy-chain antibody, preferably a heavy-chain antibody derived from camelids; (2) by expression of a nucleotide sequence encoding a naturally occurring V_{H}H domain; (3) by "humanization" of a naturally occurring V_{H}H domain or by expression of a nucleic acid encoding a such humanized V_{H}H domain; (4) by "camelization" of a naturally occurring V_{H} domain from any animal species, and in particular from a mammalian species, such as from a human being, or by expression of a nucleic acid encoding such a camelized V_{H} domain; (5) by "camelization" of a "domain antibody" or "dAb" as described in the art, or by expression of a nucleic acid encoding such a camelized dAb, for the term "dAb", reference is for example made to Ward et al. (Nature 1989 Oct. 12; 341 (6242): 544-6), to Holt et al., Trends Biotechnol., 2003, 21(11):484-490; as well as to for example WO 06/030220, WO 06/003388 and other published patent applications of Domantis Ltd, single domain antibodies or single variable domains can be derived from certain species of shark (for example, the so-called "IgNAR domains", see for example International patent application WO 05/18629); (6) by using synthetic or semi-synthetic techniques for preparing proteins, polypeptides or other amino acid sequences known *per se;* (7) by preparing a nucleic acid encoding a Nanobody using techniques for nucleic acid synthesis known *per se,* followed by expression of the nucleic acid thus obtained; and/or (8) by any combination of one or more of the foregoing. "Camelids" as used herein comprise old world camelids (*Camelus bactrianus* and *Camelus dromaderius*) and new world camelids (for example *Lama paccos, Lama glama* and *Lama vicugna*)*.*

The amino acid sequence and structure of a Nanobody can be considered - without however being limited thereto - to be comprised of four framework regions or "FR's", which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region l"or "CDR1"; as "Complementarity Determining Region 2" or "CDR2"; and as "Complementarity Determining Region 3" or "CDR3", respectively. The total number of amino acid residues in a Nanobody can be in the region of 110-120, and preferably 112-115. It should however be noted that parts, fragments, analogs or derivatives of a Nanobody are not particularly limited as to their length and/or size, as long as such parts, fragments, analogs or derivatives meet the further requirements outlined herein and are preferably suitable for the purposes described herein.

The variable domains present in naturally occurring heavy chain antibodies are also be referred to as "V_{H}H domains", in order to distinguish them from the heavy chain variable domains that are present in conventional 4-chain antibodies (which will be referred to herein as "V_{H} domains") and from the light chain variable domains that are present in conventional 4-chain antibodies (which will be referred to herein as "V_{L} domains"). V_{H}H domains have a number of unique structural characteristics and functional properties which make isolated V_{H}H domains (as well as Nanobodies based thereon, which share these structural characteristics and functional properties with the naturally occurring V_{H}H domains) and proteins containing the same highly advantageous for use as functional antigen-binding domains or proteins. In particular, and without being limited thereto, V_{H}H domains (which have been "designed" by nature to functionally bind to an antigen without the presence of, and without any interaction with, a light chain variable domain) and Nanobodies can function as a single, relatively small, functional antigen-binding structural unit, domain or protein. This distinguishes the V_{H}H domains from the V_{H} and V_{L} domains of conventional 4-chain antibodies, which by themselves are generally not suited for practical application as single antigen-binding proteins or domains, but need to be combined in some form or another to provide a functional antigen-binding unit (as in for example conventional antibody fragments such as Fab fragments; in ScFv's fragments, which consist of a V_{H} domain covalently linked to a VL domain, as described elsewhere in this specification).

For example, the functional component may comprise, consist essentially of or consist of a V_{H}H targeting HER2, such as the 2D3 V_{H}H or the 47D5 V_{H}H, e.g. the 2D3 V_{H}H or the 47D5 V_{H}H as described in International patent application WO2009068625 A2.

In further embodiments, the antigen-binding region is obtained from a multi-specific antibody or antibody fragment (such as a bispecific, trispecific, etc. antibody) comprising at least two (such as two, three, etc.) binding sites, each directed against a different antigen or antigenic determinant.

The antigen-binding region may be obtained from antibodies or antibody fragments originating from from or comprising one or more portions derived from any animal species, preferably vertebrate species, including, *e.g*., birds and mammals. Without limitation, the antibodies may be chicken, turkey, goose, duck, guinea fowl, quail or pheasant. Also without limitation, the antibodies may be human, murine (*e.g*., mouse, rat, etc.), porcine, donkey, rabbit, goat, sheep, guinea pig, monkey (e.g., cynomolus monkeys), camel (*e.g., Camelus bactrianus* and *Camelus dromaderius*) also including camel heavy-chain antibodies, llama (*e.g., Lama paccos, Lama glama or Lama vicugna*) also including llama heavy-chain antibodies, or horse.

In particular embodiments, the antigen-binding region may be obtained from a chimeric antibody or chimeric antibody fragment, such as a chimeric antibody or chimeric antibody fragment originating from at least two animal species. More specifically, the term "chimeric antibody" or "chimeric antibodies" refers to antibodies which comprise heavy and light chain variable region sequences from one species and constant region sequences from another species, such as for example antibodies having murine heavy and light chain variable regions linked to human, non-human primate, canine, equine, or feline constant regions. Chimeric antibodies comprise a portion of the heavy and/or light chain that is identical to or homologous with corresponding sequences from antibodies derived from a particular species or belonging to a particular antibody class or subclass, while the remainder of the chain(s) is identical to or homologous with corresponding sequences in antibodies from another species or belonging to another antibody class or subclass, as well as fragments of such antibodies, exhibiting the desired biological activity (See e.g., U.S. Pat. No. 4,816,567; and Morrison et al., Proc. Natl. Acad. Sci. USA 81 :6851-6855 (1984)). Chimeric antibodies are made through merging DNA encoding a portion, such as the Fv region, of a monoclonal antibody from one species, e.g. mouse or monkey, with the antibody-producing DNA from another species, e.g. human.

In particular embodiments, the antigen-binding region may be obtained from a fully human antibody or antibody fragment. As used herein, the term "fully human antibody" refers to an antibody of which the encoding genetic information is of human origin. Accordingly, the term "fully human antibody" refers to antibodies having variable and constant regions derived only from human germline immunoglobulin sequences. The term "fully human antibody" is thus not to include antibodies in which CDR sequences derived from the germline of other mammalian species, such as a mouse, have been grafted onto human framework sequences.

In particular embodiments, the antigen-binding region may be obtained from a humanized antibody or antibody fragment. More particularly, the term "humanized antibody" refers to antibodies which comprise heavy and light chain variable region sequences from a non -human species (e.g., a mouse) but in which at least a portion of the V_{H} and/or V_{L} sequence has been altered to be more "human-like", i.e., more similar to human germline variable sequences. One type of humanized antibody is a CDR-grafted antibody, in which non-human CDR sequences are introduced into human V_{H} and V_{L} sequences to replace the corresponding human CDR sequences.

A skilled person will understand that the antigen-binding region obtained from an antibody or antibody fragment can include one or more amino acid deletions, additions and/or substitutions (e.g., conservative substitutions), insofar such alterations preserve its binding of the respective antigen.

In particular embodiments, the functional component comprises, consists essentially of or consists of an antibody-like scaffold, an extracellular domain of a viral envelope protein, a cognate extracellular domain of a receptor or ligand for the antigen or an antigen-binding portion of said receptor or ligand, a soluble receptor, or a synthetic receptor.

In particular embodiments, the functional component comprises one or more antibody-like scaffolds.

The term "antibody-like scaffold" as used herein refers to a synthetic or natural binding molecules having a stable scaffold holding the molecule together and a variable arm binding to specific targets thereby mimicking the general structure and function of an antibody. Non-limiting examples of antibody-like scaffolds include designed ankyrin repeat proteins (DARPins), affimers and monobodies. For example, the functional component may comprise a Darpin directed to HER-2 with an amino acid sequence as defined by SEQ ID NO: 2294-2295 or a affibody directed to HER-2 with an amino acid sequence as defined by SEQ ID NO: 2297, as described in WO2017172981A2, which is incorporated herein by reference.

In particular embodiments, the functional component comprises a cognate extracellular domain of a receptor or ligand for the antigen or an antigen-binding portion of said receptor or ligand.

The term "cognate" as used herein with regard to the receptor or ligand refers to the receptor or ligand with which the target molecule preferentially interacts under physiological conditions, or under in vitro conditions substantially approximating physiological conditions. As used herein, the term "preferentially interacts" is synonymous with "preferentially binding" and refers to an interaction that is statistically significantly greater in degree relative to a control.

As described elsewhere in this specification for antibodies, the term "antigen-binding portion" or "antigen-binding region" refers to one or more fragments of a receptor or ligand that retain the ability to specifically bind to an antigen.

The receptor may be a soluble receptor, such as a soluble receptor capable of binding pathogenic microorganisms or toxins. For example, the functional component may comprise a soluble IL-15 receptor alpha chain (sIL15Ralpha) that is capable of binding IL-15, or a complement receptor type 1 (CR1, CD35). For example, a codon-optimized soluble version of the human IL15 receptor alpha-chain that binds human interleukin-15 [UniProtKB - Q13261.1 (I15RA_HUMAN) - Domain used: Residues 31-96] spanned between Bgl2 (A/GATCT, amino acids RS) and BspEl (T/CCGGA, amino acids SG) restriction sites, such as a soluble IL-15 receptor alpha chain with an amino acid sequence as defined by SEQ ID NO: 32 and a nucleic acid sequence as defined by SEQ ID NO: 33.

CR1 is a membrane-anchored complement regulatory protein (mCRP). CR1 is the most versatile mCRP. CR1 reduces the C3b and C4b depositions and deactivates bound C3b/C4b to C3d/C4d. Loss of CR1 expression on podocytes may contributes to complement-mediated damage in the kidney. A dimeric protein complex can be designed by cloning the gene encoding soluble CR1 upstream of the C-terminal fragment of the C4bp beta-chain, and by cloning a gene encoding a targeting moiety downstream of the C-terminal fragment of the C4bp beta-chain, in order to generate soluble complement inhibitor that selectively accumulates at a target membrane surface that could locally direct the complement activation. Such complexes may also be referred to as a "complement switch-off strategy" or protective cell targeting approach.

The ligand may be a cytokine, a growth factor, or a ligand that acts as an agonist or antagonist of receptors that are capable of modulating an immune response *in vivo.*

In particular embodiments, the functional component comprises, consists essentially of, or consists of an extracellular domain of a viral envelope protein.

In particular embodiments, the functional component comprises a synthetic receptor. The term "synthetic receptor" or "recombinant receptor" refers to a receptor that cannot be found in nature as such and is being artificially produced by man. For example, a polypeptide sequence can be intentionally modified by man in the laboratory.

When the functional component includes a single chain variable fragment of an antibody (scFv) or a single domain variable fragment of a heavy chain antibody (V_{H}H) specific for the antigen, an antibody-like scaffold, a cognate receptor or ligand for the antigen or an antigen-binding portion of said receptor or ligand, or a synthetic receptor known to specifically bind to an antigen of interest, such as a TSA or TAA or viral antigen or virus-associated antigen, the capacity of such functional component to bind to the antigen of interest is meaningfully similar or comparable to the ability of the scFv or V_{H}H specific for the antigen, the antibody-like scaffold, the cognate receptor or ligand for the antigen or the antigen-binding portion of said receptor or ligand, the soluble receptor or the synthetic receptor.

In particular embodiments, said binding domain (e.g. antigen recognition domain) specifically binds to a tumor antigen, a pathogen antigen, an activating NK cell receptor, a cytokine, a toxin or a contaminant. In particular embodiments , said binding domain (e.g. antigen recognition domain) specifically binds to a tumor specific antigen (TSA), a tumor associated antigen (TAA), a bacterial antigen, a viral antigen or a virus-associated antigen a fungal antigen, an activating NK cell receptor, a cytokine, a toxin, or a contaminant.

The term "tumor antigen" as used herein refers to an antigenic substance produced in tumor cells that is able to trigger an immune response in a host. The term "tumor specific antigen" refers to antigens that are present on tumor cells and not on any other cells. The term "tumor-associated antigen" refers to antigens that are present on some tumor cells and also on some normal cells. Non-limiting examples of TSA and/or TAA include CD19, CD319/CS1, ROR1, CD20, CD5, CD7, CD22, CD70, CD30, BCMA, CD25, NKG2D ligands, MICA/MICB, carcinoembryonic antigen (CEA), alphafetoprotein (AFP), CA-125, MUC-1, CO17-1A, melanoma-associated antigen (MAGE), mutated p53, mutated ras, HER2/Neu, ERBB2, folate binding protein, GD2, CD123, CD33, CD37, CD30, CD56, c-Met, mesothelin (MSLN), GD3, HERV-K, IL-11Rα, CSPG4,WT-1, EGFRvIII, TRAIL/DR4, VEGFR2, glycoprotein 100 (gp100/Pme117), NY-BR-1, NY-CO-58, NY-ESO-1, MART1, 5T4, αvβ6 integrin, B7-H3, B7-H6, CAIX, CD20, CD44, CD44v6, CD44v7/8, CD79a, CD79b, CD138, CD171, CEA, CSPG4, EGFR, EGFR family, EGP2, EGP40, EPCAM, EphA2, EpCAM, FAP, fetal AchR, FRα, 'Glypican-3 (GPC3), HLA-A1+MAGE1, HLA-A2+MAGE1, HLA-A3+MAGE1, HLA-A1+NY-ESO-1, HLA-A2+NY-ESO-1, HLA-A3+NY-ESO-1, IL-13Rα2, Lambda, Lewis-Y, Kappa, Mesothelin, Muc16, NCAM, PRAME, PSCA, PSMA, SSX, Survivin, TAG72, or TEMs.

Protein descriptions for the protein abbreviations used in the specification can be consulted via the protein database of U.S. government's National Center for Biotechnology Information (NCBI) (http://www.ncbi.nlm.nih.gov/) or via the homepage of UniProt (https://www.uniprot.org/).

In particular embodiments, the TSA or TAA is human epidermal growth factor receptor 2 (HER2). By means of example, HER2 is human HER2 as annotated under Uniprot (www.uniprot.org) accession number P04626.1.

In particular embodiments, the bacterial antigen is the PcrV or Psl of *Pseudomonas aeruginosa.*

In particular embodiments, the viral antigen or virus-associated antigen is selected from the group consisting of Human Cytomegalovirus (HCMV) antigens, gp160, Hepatitis C virus (HCV) antigens, Human papilloma virus (HPV) antigens, lysozymes (LYZ), pp50, Tat, VSV8, Epstein-Barr Virus (EBV) antigens, gp33, Hepatitis Delta Virus (HDV), Influenza virus, nef, pp65, tuberculosis antigens, gag, Hepatitis B virus (HBV) antigens, Human immunodeficiency virus (HIV) antigens, LMP2, p21 protein, severe acute respiratory syndrome (SARS)-CoV antigens, and vpr.

In particular embodiments, the fungal antigen is a *Aspergillus sp.* antigen.

In particular embodiments, said activating NK cell receptor is a natural cytotoxicity receptor (NCR), NKG2D, NKG2A, killer immunoglobulin-like receptor (KIR), or SLAMF7 (CRACC).

By means of example, the human NCR1 is as annotated under Uniprot (www.uniprot.org) accession number 076036.1 , the human NKG2D is as annotated under Uniprot (www.uniprot.org) accession number P26718.1, the human NKG2A is as annotated under Uniprot (www.uniprot.org) accession number P26715.2, the human KIR is as annotated under Uniprot (www.uniprot.org) accession number Q99706.3, Q8N743.2 or P43628.1, and the human SLAMF7 is as annotated under Uniprot (www.uniprot.org) accession number Q9NQ25.1.

In particular embodiments, said binding domain (e.g. antigen recognition domain) is capable of antagonizing (i.e. inhibiting) the activating NK cell receptor. A dimeric protein complex as taught herein comprising a functional component comprising, consisting essentially of or consisting of a binding domain (e.g. antigen recognition domain) specifically binding to and antagonizing an activating NK cell receptor may be used to activate NK cells.

In particular embodiments, the cytokine is IL-15.

In particular embodiments, the toxin is Sphingomyelinase-D (*SmaseD*) from *Loxosceles intermedia* or *Loxosceles reclusa.* A dimeric protein complex as taught herein comprising a functional component comprising, consisting essentially of or consisting of a binding domain (e.g. antigen recognition domain) specifically binding to a toxin may be used to neutralize the toxic activity of this toxin.

In particular embodiments, said first, said second, said third and/or said fourth functional component comprises, consists essentially of or consists of a recombinant viral structural protein, such as a recombinant viral structural protein for a virus-like particle (VLP).

In particular embodiments, said first, said second, said third and/or said fourth functional component comprises a cytotoxic agent, such as Sphingomyelinase-D (*SmaseD)* from *Loxosceles intermedia* or *Loxosceles reclusa.* A dimeric protein complex comprising as a functional component *SmaseD* from *Loxosceles intermedia* and as a further functional component a functional component comprising a binding domain (e.g. antigen recognition domain) could be used to kill a target cell as a result of the accumulation of the toxin at the cell surface of the target cell.

In particular embodiments, one of said first, said second, said third or said forth functional components comprise a binding domain (e.g. antigen recognition domain) specifically binding to an activating NK cell receptor, wherein binding of the binding domain (e.g. antigen recognition domain) to the activating NK cell receptor is capable of activating the NK cell; and wherein at least two, such as two or three, of said first, said second, said third or said forth functional components comprise a binding domain (e.g. an antigen recognition domain) specifically binding to a TSA, a TAA, a bacterial antigen, a viral antigen, a viral-associated antigen or a fungal antigen. Such dimeric protein complexes are referred to herein as "BiKEs".

In particular embodiments, two of said first, said second, said third or said forth functional components comprise a binding domain (e.g. antigen recognition domain) specifically binding to an activating NK cell receptor, wherein binding of the binding domain (e.g. antigen recognition domain) to the activating NK cell receptor is capable of activating the NK cell; and wherein two of said first, said second, said third or said forth functional components comprise a binding domain (e.g. antigen recognition domain) specifically binding to a TSA, a TAA, a bacterial antigen, a viral antigen, a virus-associated antigen or a fungal antigen. Preferably, each of said two binding domains (e.g. antigen recognition domains) specifically binding to an activating NK cell receptor are different, and more preferably each specifically bind a different activating NK cell receptor. Such dimeric protein complexes are referred to herein as "TriKEs".

In particular embodiments, three of said first, said second, said third or said forth functional components comprise a binding domain (e.g. antigen recognition domain) specifically binding to an activating NK cell receptor, wherein binding of the binding domain (e.g. antigen recognition domain) to the activating NK cell receptor is capable of activating the NK cell; and wherein one of said first, said second, said third or said forth functional components comprise a binding domain (e.g. antigen recognition domain) specifically binding to a TSA, a TAA, a bacterial antigen, a viral antigen, a viral-associated antigen or a fungal antigen. Preferably, each of said three binding domains (e.g. antigen recognition domains) specifically binding to an activating NK cell receptor are different, and more preferably each specifically bind a different activating NK cell receptor.

Said at least two, two or three binding domains (e.g. antigen recognition domains) specifically binding to a TSA, a TAA, a bacterial antigen, a viral antigen, a viral-associated antigen or a fungal antigen may each recognize different epitopes of the same ligand/receptor present on the surface of the same pathogen or tumor, distinct ligands/receptors on the same pathogen or tumor, or distinct ligands/receptors on distinct pathogens or tumors.

In particular embodiments, the first and/or second functional components of the dimeric protein complex as taught herein comprises, consists essentially of, or consists of a molecule or a fragment thereof, preferably a peptide, whose main biological active domain is located at its C-terminal end, such as a monomeric Fc. Preferably, the first and/or second functional components of the dimeric protein complex as taught herein do not comprises, consists essentially of, or consists of a molecule or a fragment thereof, preferably a peptide, whose main biological active domain is located at its N-terminal end, such as a glycoprotein. Alternatively, a flexible linker or a rigid spacer could be introduced between the molecule whose main biological active domain is located at its N-terminal end and the C-terminal part of the C4bp beta-chain, to move the biological active domain of the molecule away from the C-terminal part of the C4bp beta-chain.

As described elsewhere in the present specification, present inventors also found that the inclusion of a monomeric Fc, preferably monomeric Fc of IgG comprising the hinge, CH2 domain and CH3 domain of IgG, downstream (C-terminally) of the C-terminal fragment of the C4bp beta-chain of both the first and second polypeptide of the dimeric protein complex leads to an unexpectedly enhanced abilities of the dimeric Fc formed by two monomeric Fcs to (i) activate the complement system and C3b deposits to a target, to (ii) activate NK cells and antibody-dependent cell-mediated cytotoxicity (ADCC)/ complement-dependent cytotoxicity (CDCC) and to (iii) activate macrophages and macrophage-mediated phagocytosis of target cells compared to conventional therapeutic antibodies. These enhanced abilities are a result of the double hinge that is created (i.e. the presence of a Fc hinge and a C4bp beta-chain hinge N-terminally of the Fc hinge) in the dimeric protein complexes wherein the first and second functional components are monomeric Fc as taught herein. Furthermore, such dimeric protein complexes can be produced in a more simple way than antibodies. Antibodies are typically produced using two separate constructs, (i) one encoding the light chain (V_{L}, C_{L}) and (ii) one encoding the heavy chain (V_{H}, CH1, hinge, CH2, CH3), as for the dimeric protein complexes wherein the first and second functional components are monomeric Fc as taught herein a single construct encoding a polypeptide comprising the C-terminal part of the C4bp beta-chain can be used, and hence only a single transfection into a host cell is required. In addition, the dimeric protein complexes wherein the first and second functional components are monomeric Fc as taught herein as taught herein may have an overall size which is smaller than conventional IgG1 antibodies (e.g. about 130 kDa, while the MW of IgG1 antibodies is typically around 150 kDa).

In particular embodiments, said first, said second, said third and/or said fourth functional component comprises, consists essentially of or consists of monomeric Fc.

In particular embodiments, the first and second functional component comprise, consist essentially of or consist of monomeric Fc (also referred to herein as Fc domain); and wherein the hinge region of the monomeric Fc in the first polypeptide is connected to the hinge region of the monomeric Fc in the second polypeptide by at least two disulfide bonds; and
the third and/or fourth functional component comprises a binding domain (e.g. antigen recognition domain).

In particular embodiments, the first and second functional component comprise, consist essentially of or consist of an identical monomeric Fc.

In particular embodiments, the third and/or fourth functional component does not interfere with the biological function of the monomeric Fc.

In particular embodiments, the monomeric Fcs in the first and second polypeptide are coupled immediately C-terminally of the C-terminal part of the C4bp beta-chain, meaning that no linker is present between the monomeric Fc and the C-terminal part of the C4bp beta-chain.

The monomeric Fc may derived from known antibodies or fragments thereof, such as known therapeutic antibodies or fragments thereof. Accordingly, in particular embodiments, the dimeric protein complexes as taught herein allow to improve the biological functions of the Fc of antibodies or fragments thereof, without having to modify the sequence of the Fc. In particular embodiments, the monomeric Fc comprises the hinge (also referred to herein as the hinge region or hinge domain), the CH2 domain and the CH3 domain. In particular embodiments, the monomeric Fc is monomeric Fc of IgG comprising the hinge (also referred to herein as the hinge region or hinge domain), CH2 domain and CH3 domain of IgG. In preferred embodiments, the monomeric Fc is monomeric Fc of IgG1 comprising, consisting essentially of or consisting of the hinge (i.e. the hinge region), the CH2 domain and the CH3 domain of IgG1. For example, the monomeric Fc may comprise, consist essentially of or consist of the amino acid sequence

EPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEW(SEQ ID NO: 34).

In particular embodiments, the knob-into-hole technology as described in International patent application WO2013097430, which is incorporated herein by reference, may be applied to the monomeric Fc, for example such as shown in Figure 21. For example, the CH3 Fc IgG1 comprised by the first polypeptide of the dimeric protein complex may display 3 mutations (S354C, T366W and K409A), also known as Fc[knob] and the CH3 Fc IgG1 comprised by the second polypeptide of the dimeric protein complex may display 5 mutations (Y349C, T366S, L368A, F405K and Y407V). In particular embodiments, the third functional component comprises, essentially consists of or consists of a binding domain (e.g. antigen recognition domain) and the fourth functional component comprises, essentially consists of or consists of a binding domain (e.g. antigen recognition domain), wherein the binding domains (e.g. antigen recognition domains) are different, and therefore may be referred to as bispecific pseudo-IgGs. Bispecific pseudo-IgGs may allow to crosslink two different cell types, such as a target cell and an effector cell.

In particular embodiments, the first and second functional component comprise, essentially consist of or consist of monomeric Fc (also referred to herein as Fc domain); and wherein the hinge region of the monomeric Fc in the first polypeptide is connected to the hinge region of the monomeric Fc in the second polypeptide by at least two disulfide bonds; and the third and/or fourth functional component comprises, essentially consists of or consists of a scFv or V_{H}H as described elsewhere in the present specification, such as a scFv specifically binding the CD20 antigen (e.g. a scFv derived from Rituximab, or Ofatumumab), a scFv or a V_{H}H specifically binding HER2 (e.g. the 2D3 V_{H}H, the 47D5 V_{H}H anti-HER or a Trastuzumab-derived scFv), a scFv specifically binding *Pseudomonas aeruginosa* (e.g. scFv anti-*Pseudomonas* derived from Panobacumab, a scFv anti-PcrV or a scFv anti-Psl), a scFv specifically binding NKG2A, a scFv specifically binding SLAMF7, or a scFv specifically binding NKG2D. Numerous combinations can be made thereby creating bi-specific pseudo-IgGs as illustrated in Table 1 below.

**Table 1**

| **Bi-specific Pseudo IgGs** | HER2/ CD20 | NK/ HER2 | NK/ HER2 | NK/ *Pseudomonas* | NK/ *Neisseria* | *Pseudomonas* |
|---|---|---|---|---|---|---|
| scFv or V_{H}H anti-HER2 | X | X | X | | | |
| scFv anti-CD20 | X | | | | | |
| scFv anti-*Pseudomonas* | | | | X | | X (combination of scFv anti-Psl (*Pseudomonas*) and reversed Panobacumab (RP) scFv anti-*Pseudomonas*) |
| scFv anti-NKG2A | | X | | | | |
| scFv anti-SLAMF7 | | | X | | | |
| scFv anti-NKG2D | | | | X | X | |
| CPP1-2 C4bp alpha-chain | | | | | X | |

For example, the first and second polypeptide of the dimeric protein complex as comprised herein may comprise a codon-optimized RTX scFv pseudo-IgG specifically binding CD20, with an amino acid sequence: 
NSPVAAMGAGATGRAMDGPRLLLLLLLGVSLGGARSQIVLSQSPAILSASPGEKVTMTCR
ASSSVSYIHWFQQKPGSSPKPWIYATSNLASGVPVRFSGSGSGTSYSLTISRVEAEDAATYYCQ
QWTSNPPTFGGGTKLEIKGGSSRSSSSGGGGSGGGGQVQLQQPGAELVKPGASVKMSCKASG
 
YTFTSYNMHWVKQTPGRGLEWIGAIYPGNGDTSYNQKFKGKATLTADKSSSTAYMQLSSLT
SEDSAVYYCARSTYYGGDWYFNVWGAGTTVTVSASGAGGGGSSGAGGSAGGGGSSGGG
GSAGGGGSIQEAPKPECEKALLAFQESKNLCEAMENFMQQLKESGMTMEELKYSLELKKAE
LKAKLLEPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK.SRTSP
RASGR (SEQ ID NO: 35);
 and/or encoded by a nucleic acid sequence:

GAATTCACCGGTCGCCGCCATGGGCGCTGGCGCTACCGGAAGAGCCATGGACGGCC
CCAGGCTGCTGCTGCTGCTCCTGCTGGGCGTGTCCCTGGGCGGAGCTAGATCTCAGA
TCGTGCTGAGCCAGAGCCCCGCCATCCTGTCCGCCAGCCCAGGAGAGAAGGTGACAATG
ACATGTAGAGCCAGCTCCAGCGTGAGCTACATCCACTGGTTTCAGCAGAAGCCTGGCTCC
TCCCCTAAGCCCTGGATCTACGCCACATCCAACCTGGCCTCCGGCGTGCCCGTGAGGTTCT
CCGGTAGCGGCAGCGGCACCAGCTACAGCCTGACAATCTCCAGAGTGGAGGCCGAGGAC
GCCGCCACATACTACTGTCAGCAGTGGACAAGCAACCCCCCCACATTTGGCGGCGGCACA
AAGCTGGAGATCAAGGGCGGCTCCAGCAGGAGCAGCTCCAGCGGAGGCGGCGGATCCGG
TGGAGGAGGACAGGTGCAGCTGCAGCAGCCTGGCGCCGAGCTGGTGAAGCCCGGAGCTA
GCGTGAAGATGAGCTGCAAGGCCTCCGGCTACACCTTTACAAGCTACAATATGCACTGGG
TGAAGCAGACCCCCGGCAGAGGCCTGGAGTGGATCGGCGCTATCTACCCCGGCAACGGC
GATACATCCTACAACCAGAAGTTTAAGGGCAAGGCCACACTGACAGCCGATAAGAGCTC
CAGCACAGCCTACATGCAGCTGTCCAGCCTGACAAGCGAGGATAGCGCCGTGTACTACTG
CGCCAGGAGCACATACTACGGCGGCGACTGGTACTTTAATGTGTGGGGCGCCGGCACCAC
AGTGACAGTGAGCGCCTCCGGAGCCGGCGGAGGAGGAAGCAGCGGAGCTGGAGGCA
GCGCCGGAGGAGGAGGATCCTCCGGTGGCGGAGGCAGCGCTGGAGGAGGAGGTTC
CATCCAGGAGGCCCCTAAGCCTGAGTGTGAGAAGGCCCTGCTGGCCTTTCAGGAGAGCA
AGAACCTGTGCGAGGCCATGGAGAATTTTATGCAGCAGCTGAAGGAGAGCGGCATGACA
ATGGAGGAGCTGAAGTACAGCCTGGAGCTGAAGAAGGCCGAGCTGAAGGCCAAGCTGCT
GGAGCCTAAGAGCTGCGACAAGACCCACACATGTCCTCCCTGTCCTGCCCCTGAGCTGCTGG
GCGGACCTAGCGTGTTTCTGTTCCCTCCCAAGCCCAAGGATACACTGATGATCTCCAGAA
CACCCGAGGTGACATGCGTGGTGGTGGACGTGTCCCACGAGGACCCTGAGGTGAAGTTC
AATTGGTACGTGGATGGCGTGGAGGTGCACAACGCCAAGACCAAGCCTAGGGAGGAGCA
GTACAATTCCACCTACAGGGTGGTGAGCGTGCTGACCGTGCTGCACCAGGACTGGCTGAA
CGGCAAGGAGTACAAGTGTAAGGTGAGCAACAAGGCCCTGCCCGCCCCCATCGAGAAGA
CCATCAGCAAGGCCAAGGGCCAGCCCAGGGAGCCCCAGGTTTACACACTGCCTCCCT
CCAGAGATGAGCTGACAAAGAATCAGGTGAGCCTGACATGTCTGGTGAAGGGCTTT

TACCCCTCCGATATCGCCGTGGAGTGGGAGTCCAACGGCCAGCCCGAGAATAATTA
CAAGACCACCCCCCCTGTGCTGGACTCCGATGGCAGCTTCTTCCTGTACTCCAAGCT
GACAGTGGATAAGTCCAGGTGGCAGCAGGGCAACGTGTTCAGCTGCTCCGTGATGC
ACGAGGCCCTGCACAACCACTACACCCAGAAGAGCCTGAGCCTGTCCCCCGGCAAG
TGATCTAGAACTAGTCCTAGGGCTAGCGGCCGC (SEQ ID NO: 36):

The signal peptide (**first bold sequence** in the sequence defined by SEQ ID NO: 36) is cloned between EcoRI (GAATTC or NS) and Bgl2 (AGATCT or RS) restriction sites is the signal sequence from the tumour necrosis factor receptor superfamily member 16 (TNFR16) (UniProt no. P08138.1). The sequence encoding RTX scFv is between Bgl2 (AGATCT or RS) and BspE1 (TCCGGA or SG) restriction sites. The sequence encoding the linker is in between the BspE1 restriction site and the beginning of the C4bp C-terminal beta-chain (**second bold sequence** in the sequence defined by SEQ ID NO: 36). The sequence encoding the linker.C4bp(beta).Fc is between BspE1 and the stop codon **TGA** (.), followed by a multiple cloning sequence ending with NotI (GCGGCCGC). The sequence encoding the C-terminal part or fragment of the C4bp beta-chain dimerization scaffold is the first underlined sequence in the sequence defined by SEQ ID NO: 36. The sequence encoding the Hinge of Fc IgG1 is in italic. The sequence encoding CH2 Fc IgG1 is the second underlined sequence in the sequence defined by SEQ ID NO: 36. The sequence encoding the CH3 Fc IgG1 is the third bold sequence in the sequence defined by SEQ ID NO: 36.

Preferably, a "pseudo-IgG" as taught herein is encoded by a nucleic acid sequence comprising the cassette encoding the monomeric Fc spanned between EcoRI (G/AATTC) and NotI. The gene encoding the other functional component located N-terminally of the C-terminal part of the C4bp beta-chain of such exemplary pseudo-IgG is interchangeable, and preferably located between the restriction sites Bgl2 (A/GATCT) and BspE1, the restriction sites and the gene of interest being in the same open reading frame as depicted in the above sequence.

Present inventors also found that the previously described "knob-into-hole technology", such as described in European patent EP2235064B1 or International patent application WO2013097430, which are incorporated herein by reference, can be applied to the pseudo-IgGs as described herein. The "knob-into-hole technology" allows to generate 100% of a single molecular species consisting of dimeric protein complexes as taught herein without the use of a purification step for separating several different molecular species of dimeric protein complexes.

For example, the knob-into-hole technology may be used to generate a dimeric protein complex (Figure 21) comprising (i) a first polypeptide encoded by a nucleic acid sequence comprising a a codon-optimized sequence encoding the linker.C4bpβ.Fc between BspE1 (T/CCGGA) and the stop codon **TGA**, optionally followed by a multiple cloning site ending with NotI (GC/GGCCGC), whose encoded CH3 Fc IgG1 displays 3 amino acid mutations (S354C, T366W and K409A), also known as Fc[knob], comprising, consisting essentially of or consisting of a nucleic acid sequence as defined by SEQ ID NO: 38 and/or encoding an amino acid sequence as defined by SEQ ID NO: 37; and (ii) a second polypeptide encoded by a nucleic acid sequence comprising a codon-optimized sequence encoding the linker.C4bpβ.Fc between BspE1 and the stop codon **TGA**, optionally followed by a multiple cloning site ending with NotI, whose encoded CH3 Fc IgG1 displays 5 amino acid mutations (Y349C, T366S, L368A, F405K and Y407V), also known as Fc[hole], as described in the International patent application WO2013097430, comprising, consisting essentially of or consisting of an nucleic acid sequence as defined by SEQ ID NO: 40 and/or encoding an amino acid sequence as defined by SEQ ID NO: 39.

In particular embodiments, the functional component may be a tag.

In particular embodiments, the first and/or second polypeptide comprises a fifth and/or sixth functional component, wherein said fifth and/or sixth functional component is a tag. In particular embodiments, if the first and/or second polypeptide comprises two functional components N- and/or C-terminally of the C-terminal fragment of the C4bp beta-chain, one of these two functional components may be a tag.

In particular embodiments, the tag may be located just after the signal peptide, immediately N-terminal or C-terminal of the C-terminal fragment of the C4bp beta-chain or at the C-terminus of the first and/or second polypeptide.

A tag can be attached to proteins for various purposes, such as purification (e.g poly (His) tag), to assist proper protein folding (e.g. thioredoxin), separation techniques (e.g. FLAG-tag), enzymatic or chemical modifications (e.g. biotin ligase tags, FIAsH), or detection (e.g. tracking or visualization). Tags for detection can typically be visualized either directly or indirectly through detection with a labeled antibody or other protein or molecule binding or interacting with the tag. Examples of such tags are AviTag, Calmodulin-tag, polyglutamate tag, E-tag, EE-tag, EPEA/C-tag, FLAG-tag, HA-tag, His-tag, Myc-tag (e.g. c-myc-tag), HSV epitope, HAT, S-tag, SBP-tag, Softag 1, Softag 3, Strep tag, TC tag, V5 tag, VSV-tag, Xpress tag, Isopeptag, SpyTag, Biotin Carboxyl Carrier Protein, Glutathione-S-transferase (GST)-tag, Green fluorescent protein tag, Halo-tag, Maltose binding protein (MSB)-tag, Nus-tag, Thioredoxin-tag or Fc-tag, but is not limited thereto. The term "tag" as used herein also encompasses other tracking components such as a fluorescent protein (eGFP, eRFP, Cherry), a magnetic bead, biotin for staining with labelled avidin or streptavidin conjugate, an enzyme, a substrate, a cofactor, a chemiluminescent group (e.g. nanoluciferase), a chromogenic agent, a colorimetric label, a molecular imaging probe (e.g. .¹⁸F, ¹¹C, or ⁶⁴Cu, ^{99m}Tc, iron oxide nanoparticles, or luciferase).

Preferably the tag is a peptide, protein or polypeptide. In particular embodiments, the tag is a peptide, protein or polypeptide having an amino acid sequence of at most 10 amino acids. In particular embodiments, the tag is a protein purification tag or a protein separation tag. More preferably, the tag is a FLAG-tag, His-tag, HA-tag or Myc-tag.

In particular embodiments, the first and/or second polypeptide comprises a tag C-terminally of the first and/or second functional component.

For therapeutic use in humans, where the presence of a fluorescent moiety may not be advisable, a proteolytic cleavage site can be introduced N- or C-terminally of the tag, depending on the location of the tag within the first or second polypeptide, to remove said tag after purification of the dimeric protein complex.

In particular embodiments, a proteolytic cleavage site is included immediately N-terminally of the tag. The person skilled in the art will understand that as result thereof, the proteolytic cleavage site is located between the first or second functional component and the tag.

Example of such cleavage sites are well known in the art and include a Tobacco Etch Virus (TEV) protease cleavable site, such as comprising an amino acid sequence ENLYFQ/G (SEQ ID NO: 41), or a Human rhinovirus (HRV) 3C protease cleavable site, such as comprising an amino acid sequence LEVLFQ/GP (SEQ ID NO: 42), wherein '/' represents the peptide bond which will be cleaved), as are the methods to introduce them in the constructs of the invention or to use them for releasing protein moieties.

Preferably, the proteolytic cleavage site is a TEF protease cleavable site, such as comprising an amino acid sequence ENLYFQ/G (SEQ ID NO: 41), or a HRV 3C protease cleavable site, such as comprising an amino acid sequence LEVLFQ/GP (SEQ ID NO: 42).

A further aspect provides, a nucleic acid encoding the first polypeptide of the dimeric protein complex comprising a first functional component and a C-terminal fragment of the C4bp beta-chain, wherein said first functional component is coupled to the C-terminus of the C-terminal fragment of the C4bp beta-chain as defined herein.

A further aspect provides, a nucleic acid encoding the second polypeptide comprising a second functional component and a C-terminal fragment of the C4bp beta-chain, wherein said second functional component is coupled to the C-terminus of said C-terminal fragment of the C4bp beta-chain as defined herein.

In particular embodiments, the nucleic acid encoding the first polypeptide and the nucleic acid encoding the second polypeptide are in separate cistrons or in a multicistronic construct.

Accordingly, a further aspect provides, a nucleic acid encoding the first polypeptide of the dimeric protein complex comprising a first functional component and a C-terminal fragment of the C4bp beta-chain, wherein said first functional component is coupled to the C-terminus of the C-terminal fragment of the C4bp beta-chain and encoding a second polypeptide comprising a second functional component and a C-terminal fragment of the C4bp beta-chain, wherein said second functional component is coupled to the C-terminus of said C-terminal fragment of the C4bp beta-chain as defined herein.

By "encoding" is meant that a nucleic acid sequence or part(s) thereof corresponds to a particular amino acid sequence, e.g., the amino acid sequence of one or more desired proteins or polypeptides, or to another nucleic acid sequence in a template-transcription product (e.g. RNA or RNA analogue) relationship.

In particular embodiments, the nucleic acids encoding the first, second, third and/or fourth functional components are codon-optimized.

In particular embodiments, the nucleic acids encoding the first and second functional components, such as nucleic acids encoding a scFv, are spanned between Bgl2 (A/GATCT, amino acids RS) and BspE1 (T/CCGGA, amino acids SG) restriction sites.

In particular embodiments, the nucleic acid encoding the first polypeptide and the nucleic acid encoding the second polypeptide comprise a nucleic acid encoding a signal peptide.

In particular embodiments, the nucleic acid encoding the first polypeptide and the nucleic acid encoding the second polypeptide comprise a nucleic acid encoding a signal peptide 5' (or upstream) of the nucleic acid encoding the C-terminal fragment of the C4bp beta-chain. The person skilled in the art will understand that if the nucleic acid encoding the first polypeptide comprises a nucleic acid encoding a third functional component 5' of the nucleic acid encoding the C-terminal fragment of the C4bp beta-chain, the nucleic acid encoding the signal peptide is located 5' of said nucleic acid encoding said third functional component. Similarly, if the nucleic acid encoding the second polypeptide comprises a nucleic acid encoding a fourth functional component 5' of the nucleic acid encoding the C-terminal fragment of the C4bp beta-chain, the nucleic acid encoding the signal peptide is located 5' of said nucleic acid encoding said fourth functional component.

Furthermore, the person skilled in the art will also understand that if the nucleic acid encoding the first polypeptide comprises a nucleic acid encoding a tag 5' of the nucleic acid encoding a third functional component and/or 5' of the nucleic acid encoding the C-terminal fragment of the C4bp beta-chain, the nucleic acid encoding the signal peptide is located 5' of said nucleic acid encoding said tag. Likewise, if the nucleic acid encoding the second polypeptide comprises a nucleic acid encoding a tag 5' of a nucleic acid encoding a fourth functional component and/or 5' of the nucleic acid encoding the C-terminal fragment of the C4bp beta-chain, the nucleic acid encoding the signal peptide is located 5' of said nucleic acid encoding said tag.

The signal peptide may be a homologous or heterologous signal peptide, depending on the host cell used for production of the agent as taught herein. Preferably, the nucleic acid encodes a signal peptide having the amino acid sequence LNGFR (SEQ ID NO: 42).

After expression of the first and/or second polypeptide of the dimeric protein complex as taught herein, the signal peptide is typically cleaved off after having performed its function, typically resulting in the "mature" form of the first and/or second polypeptides. The first and/or second polypeptide comprising the signal peptide may be referred to as the precursor form of the first and/or second polypeptide, respectively.

The first and/or second polypeptide of the dimeric protein complex as taught herein may comprise one or more restriction sites between the signal peptide and the C-terminal fragment of the C4bp beta-chain.

To allow expression of the nucleic acid encoding the first and/or second polypeptide as defined herein, the nucleic acid may be inserted into a nucleic acid expression cassette and/or vector, as is well-known in the art.

A further aspect provides, an expression cassette comprising the nucleic acid encoding the first polypeptide of the dimeric protein complex or the second polypeptide of the dimeric protein complex as taught herein, preferably wherein the nucleic acid encoding the first polypeptide or the nucleic acid encoding the second polypeptide are operably linked to a promoter and/or transcriptional and translational regulatory signals.

The term "nucleic acid expression cassette" or "expression cassette" as used herein refers to nucleic acid molecules, typically DNA, to which nucleic acid fragments, preferably the recombinant nucleic acid molecule as defined herein, may be inserted to be expressed, wherein said nucleic acid molecules comprise one or more nucleic acid sequences controlling the expression of the nucleic acid fragments. Non-limiting examples of such more nucleic acid sequences controlling the expression of the nucleic acid fragments include promoter sequences, open reading frames and transcription terminators.

Preferably, the nucleic acid expression cassette may comprise one or more open reading frames (ORF) encoding said one or more polypeptides. An "open reading frame" or "ORF" refers to a succession of coding nucleotide triplets (codons) starting with a translation initiation codon and closing with a translation termination codon known *per se*, and not containing any internal in-frame translation termination codon, and potentially capable of encoding a protein, polypeptide or peptide. Hence, the term may be synonymous with "coding sequence" as used in the art.

An "operable linkage" is a linkage in which regulatory sequences and sequences sought to be expressed are connected in such a way as to permit said expression. For example, sequences, such as, *e.g.*, a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence. Hence, "operably linked" may mean incorporated into a genetic construct so that expression control sequences, such as a promoter, effectively control transcription / expression of a sequence of interest.

The precise nature of transcriptional and translational regulatory sequences or elements required for expression may vary between expression environments, but typically include a transcription terminator, and optionally an enhancer.

Reference to a "promoter" is to be taken in its broadest context and includes transcriptional regulatory sequences required for accurate transcription initiation and where applicable accurate spatial and/or temporal control of gene expression or its response to, *e.g.*, internal or external (e.g., exogenous) stimuli. More particularly, "promoter" may depict a region on a nucleic acid molecule, preferably DNA molecule, to which an RNA polymerase binds and initiates transcription. A promoter is preferably, but not necessarily, positioned upstream, *i.e.*, 5', of the sequence the transcription of which it controls. Typically, in prokaryotes a promoter region may contain both the promoter *per se* and sequences which, when transcribed into RNA, will signal the initiation of protein synthesis (*e.g.*, Shine-Dalgarno sequence). A promoter sequence can also include "enhancer regions", which are one or more regions of DNA that can be bound with proteins (namely the trans-acting factors) to enhance transcription levels of genes in a gene-cluster. The enhancer, while typically at the 5' end of a coding region, can also be separate from a promoter sequence, e.g., can be within an intronic region of a gene or 3' to the coding region of the gene.

In embodiments, promoters contemplated herein may be constitutive or inducible. A constitutive promoter is understood to be a promoter whose expression is constant under the standard culturing conditions. Inducible promoters are promoters that are responsive to one or more induction cues. For example, an inducible promoter can be chemically regulated (e.g., a promoter whose transcriptional activity is regulated by the presence or absence of a chemical inducing agent such as an alcohol, tetracycline, a steroid, a metal, or other small molecule) or physically regulated (e.g., a promoter whose transcriptional activity is regulated by the presence or absence of a physical inducer such as light or high or low temperatures). An inducible promoter can also be indirectly regulated by one or more transcription factors that are themselves directly regulated by chemical or physical cues. Non-limiting examples of promoters include T7, U6, H1, retroviral Rous sarcoma virus (RSV) LTR promoter, the cytomegalovirus (CMV) promoter, the SV40 promoter, the dihydrofolate reductase promoter, the β-actin promoter, the phosphoglycerol kinase (PGK) promoter, and the EF1α promoter.

The terms "terminator" or "transcription terminator" refer generally to a sequence element at the end of a transcriptional unit which signals termination of transcription. For example, a terminator is usually positioned downstream of, *i.e.*, 3' of ORF(s) encoding a polypeptide of interest. For instance, where a recombinant nucleic acid contains two or more ORFs, e.g., successively ordered and forming together a multi-cistronic transcription unit, a transcription terminator may be advantageously positioned 3' to the most downstream ORF.

In particular embodiments, the expression cassette comprises the nucleic acid encoding the first or second polypeptide as disclosed herein, operably linked to one or more promoters, enhancers, ORFs and/or transcription terminators.

A further aspect provides, an expression vector comprising the nucleic acid as taught herein (i.e. the nucleic acid encoding the first and/or second polypeptide of the dimeric protein complex) or the expression cassette as taught herein.

The terms "expression vector" or "vector" as used herein refers to nucleic acid molecules, typically DNA, to which nucleic acid fragments, preferably the recombinant nucleic acid molecule as defined herein, may be inserted and cloned, i.e., propagated. Hence, a vector will typically contain one or more unique restriction sites, and may be capable of autonomous replication in a defined cell or vehicle organism such that the cloned sequence is reproducible. A vector may also preferably contain a selection marker, such as, e.g., an antibiotic resistance gene, to allow selection of recipient cells that contain the vector. Vectors may include, without limitation, plasmids, phagemids, bacteriophages, bacteriophage-derived vectors, PAC, BAC, linear nucleic acids, e.g., linear DNA, transposons, viral vectors, etc., as appropriate (see, e.g., Sambrook et al., 1989; Ausubel 1992). Viral vectors may include inter alia retroviral vectors, lentiviral vectors, adenoviral vectors, or adeno-associated viral vectors, for example, vectors based on HIV, SV40, EBV, HSV or BPV. Expression vectors are generally configured to allow for and/or effect the expression of nucleic acids or open reading frames introduced thereto in a desired expression system, e.g., *in vitro,* in a cell, organ and/or organism. For example, expression vectors may advantageously comprise suitable regulatory sequences.

Factors of importance in selecting a particular vector include inter alia: choice of recipient cell, ease with which recipient cells that contain the vector may be recognised and selected from those recipient cells which do not contain the vector; the number of copies of the vector which are desired in particular recipient cells; whether it is desired for the vector to integrate into the chromosome or to remain extra-chromosomal in the recipient cells; and whether it is desirable to be able to "shuttle" the vector between recipient cells of different species.

Expression vectors can be autonomous or integrative. A nucleic acid can be in introduced into a cell in the form of an expression vector such as a plasmid, phage, transposon, cosmid or virus particle. The recombinant nucleic acid can be maintained extra-chromosomally or it can be integrated into the cell chromosomal DNA. Expression vectors can contain selection marker genes encoding proteins required for cell viability under selected conditions (e.g., URA3, which encodes an enzyme necessary for uracil biosynthesis, or LEU2, which encodes an enzyme required for leucine biosynthesis, or TRP1, which encodes an enzyme required for tryptophan biosynthesis) to permit detection and/or selection of those cells transformed with the desired nucleic acids. Expression vectors can also include an autonomous replication sequence (ARS). The ARS may comprise a centromere (CEN) and an origin of replication (ORI). For example, the ARS may be ARS 18 or ARS68.

Prior to introducing the vectors into a cell of interest, the vectors can be grown (e.g., amplified) in bacterial cells such as *Escherichia coli* (*E. coli*). The vector DNA can be isolated from bacterial cells by any of the methods known in the art, which result in the purification of vector DNA from the bacterial milieu. The purified vector DNA can be extracted extensively with phenol, chloroform, and ether, to ensure that no *E. coli* proteins are present in the plasmid DNA preparation, since these proteins can be toxic to mammalian cells.

A further aspect provides, expression vector comprising a nucleic acid encoding the C-terminal fragment of the C4bp beta-chain, a first insertion site for a nucleic acid located immediately 3' of the nucleic acid encoding the C-terminal fragment C4bp beta-chain; and optionally a second insertion site for a nucleic acid immediately 5' of the nucleic acid encoding the C-terminal fragment of the C4bp beta-chain.

In particular embodiments, the expression vector comprises a nucleic acid encoding the C-terminal fragment of the C4bp beta-chain, a first insertion site for a nucleic acid encoding a functional component located immediately 3' of the nucleic acid encoding the C-terminal fragment C4bp beta-chain; and optionally a second insertion site for a nucleic acid encoding a functional component immediately 5' of the nucleic acid encoding the C-terminal fragment of the C4bp beta-chain.

The insertion site allows a nucleic acid, such as a piece of DNA, to be inserted into that region. The insertion site may comprise one or more restriction sites for one or more restriction enzymes. Preferably the restriction sites are unique restriction sites, meaning that they occur only once within a given vector. In particular embodiments, the restriction site(s) in the first insertion site is/are different from the restriction site(s) in the second insertion site. The insertion site allows a nucleic acid, such as a piece of DNA, to be inserted into that region. For example, the insertion site may be a multiple cloning site (MSC), as known in the art, which is a short nucleotide segment comprising multiple (such as up to 20) restriction sites.

The dimeric protein complexes as taught herein may be prepared by easy cloning processes. Furthermore, the methods for preparing the dimeric protein complexes as taught herein allow to produce dimeric protein complexes with high expression yields, such as equal to or more than 20 mg/L of dimeric protein complexes.

A further aspect provides a method for preparing the dimeric protein complex as taught herein.

In particular embodiments, if the first and second functional components are proteins or polypeptides, the method for preparing the dimeric protein complex as taught herein comprises:
- providing one or more nucleic acids encoding the first and/or second polypeptide of the dimeric protein complex as taught herein, in an expressible format;
- introducing said one or more nucleic acids encoding the first and/or second polypeptide into host cells; and
- collecting the dimeric protein complexes from the host cells or their supernatants; and
- optionally purifying the collected dimeric protein complexes.

In particular embodiments, the method for preparing the dimeric protein complex as taught herein comprises a step of culturing the host cells into which the one or more nucleic acids encoding the first and/or second polypeptide are introduced under conditions which are suitable for expressing the one or more nucleic acids and suitable for the dimerization, preferably covalent association, of the first and second polypeptide.

In particular embodiments, if the first and second polypeptides of the dimeric protein complex are identical, the method for preparing the dimeric protein complexes as taught herein only comprises introducing one nucleic acid encoding the first or second polypeptide (which are identical in the case of homodimers), in an expressible format, into host cells.

In particular embodiments, if the first and second polypeptides of the dimeric protein complex are not identical, the method for preparing the dimeric protein complexes as taught herein comprises introducing one nucleic acid encoding the first polypeptide and one nucleic acid encoding the second polypeptide, in an expressible format, into host cells, such as by co-transfection.

Typically, dimeric protein complexes (or simply dimers) are formed by random association of the first and/or second polypeptides comprising the C-terminal part of the C4bp beta-chain, irrespective of whether or not these comprise additional features such as one or more components. Therefore, host cells into which a nucleic acid encoding a first polypeptide and a nucleic acid encoding a second polypeptide, wherein the first and second polypeptides are different, are introduced, will produce dimeric protein complexes of more than one molecular specifies. For example, if the first polypeptide comprises a functional component A and the second polypeptide comprises a functional component B, the host cells will produce three different molecular species of dimeric protein complexes, namely A:A, A:B, and B:B dimeric protein complexes.

Accordingly, after production and collection of the dimeric protein complexes, the dimeric protein complexes may be purified to end up with a population of dimeric protein complexes substantially consisting (e.g. by at least 95%; preferably by at least 99%, more preferably by at least 99.9%) of dimeric protein complexes of a single molecular species of interest, such as the A:A, A:B or B:B dimeric protein complexes.

In particular embodiments, the purification of the dimeric protein complexes may be performed by making use of one or more tags incorporated into the one or more nucleic acids encoding the first and/or second polypeptide. For example, for heteroDiBolos or heteroTetraBolos wherein the first polypeptide comprises a HIS tag and the second polypeptide comprises a FLAG tag, a two-step purification may be performed, such as comprising a first purification by a HIS-TRAP purification to first capture all dimers comprising the first polypeptide comprising the HIS tag, followed by second purification by a FLAG affinity chromatography to capture all dimers comprising the first polypeptide comprising the HIS tag and the second polypeptide comprising the FLAG tag (e.g. see Figure 2).

The term "host cell" as used herein, refers to the cell that has been introduced with one or more nucleotides, preferably DNA, such as by transfection. The host cell may be a eukaryotic cell, such as a yeast cell such as *S cerevisiae*, filamentous fungus cells such as *Aspergillus sp*, insect cells such as the S2 cells of Drosophila or *sƒ9* of Spodoptera, mammalian cells or plant cells. Most commonly used host cells for production of proteins are mammalian immortalized cell lines such as CHO and HEK 293 cells, preferably HEK 293 cells, which allow easy transfection and have a high proliferation rate which facilitates the protein production and increases the yield. Large-scale production of protein complexes typically requires host cells capable of growing in suspension, such as suspension-adapted CHO and HEK 293 cells, preferably suspension-adapted HEK 293 cells.

Methods for introducing nucleic acids into viable cells are known to the person skilled in the art, and may include calcium phosphate co-precipitation, electroporation, micro-injection, protoplast fusion, lipofection, exosome-mediated transfection, transfection employing polyamine transfection reagents, bombardment of cells by nucleic acid-coated tungsten micro projectiles, viral particle delivery, etc. Such introduction may also be referred to as delivery, transfection or transformation. Cell penetrating peptides (CPPs) may also be employed for delivering polypeptides or nucleic acids into cells. CPPs include but are not limited to Penetratin, Tat (48-60), Transportan, and (R-AhX-R4).

In particular embodiments, the produced dimeric protein complexes may be concentrated.

In particular embodiments, if the first, second, third and/or fourth functional component to be included in the dimeric protein complex is not a protein or polypeptide, the method of preparing the dimeric protein complex as taught herein comprises the steps of:
- preparing a protein complex comprising the dimerized C-terminal fragments of the C4bp beta-chain and the first, second, third and/or fourth functional components being a protein or polypeptide;
- adding the first, second, third and/or fourth functional component that is not a protein or polypeptide to the prepared protein complex, thereby obtaining the dimeric protein complex of interest.

Unless indicated otherwise, all methods, steps, techniques and manipulations that are not specifically described in detail can be performed and have been performed in a manner known *per se*, as will be clear to the skilled person.A further aspect provides a eukaryotic cell engineered to produce dimeric protein complexes as taught herein, wherein said cell comprises one or more nucleic acids encoding the first and/or second polypeptide of the dimeric protein complex as taught herein, in an expressible format. The eukaryotic cell is not a human embryo.

Depending on the type of functional components included into the dimeric protein complex as taught herein, the dimeric protein complexes as taught herein may be used for multiple applications, not being limited to therapeutic applications, and include applications in health care in general (e.g. molecular imaging), for environmental purposes, for cleansing applications, etc.

A further aspect provides, a pharmaceutical composition comprising the dimeric protein complex as taught herein, the nucleic acid as taught herein, the expression cassette as taught herein, or the expression vector as taught herein, and a pharmaceutically acceptable carrier.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives, antioxidants, tonicity controlling agents, absorption delaying agents, and the like. The use of such media and agents for pharmaceutical active substances is well known in the art. Except insofar as any conventional media or agent is incompatible with the active substance, its use in the therapeutic compositions may be contemplated.

Illustrative, non-limiting carriers for use in formulating the pharmaceutical compositions include, for example, oil-in-water or water-in-oil emulsions, aqueous compositions with or without inclusion of organic co-solvents suitable for intravenous (IV) use, liposomes or surfactant-containing vesicles, microspheres, microbeads and microsomes, powders, tablets, capsules, suppositories, aqueous suspensions, aerosols, and other carriers apparent to one of ordinary skill in the art.

Pharmaceutical compositions as intended herein may be formulated for essentially any route of administration, such as without limitation, oral administration (such as, e.g., oral ingestion or inhalation), intranasal administration (such as, e.g., intranasal inhalation or intranasal mucosal application), parenteral administration (such as, e.g., subcutaneous, intravenous (I.V.), intramuscular, intraperitoneal or intrasternal injection or infusion), transdermal or transmucosal (such as, e.g., oral, sublingual, intranasal) administration, topical administration, rectal, vaginal or intra-tracheal instillation, and the like. In this way, the therapeutic effects attainable by the methods and compositions can be, for example, systemic, local, tissue-specific, etc., depending of the specific needs of a given application.

In particular embodiments, the pharmaceutical composition is a neutralising anti-serum. Preferably, the pharmaceutical composition is a neutralising anti-serum for *SmaseD* from Li, and comprises a dimeric protein complex comprising a first and second functional component, wherein the first and second functional component comprise, consist essentially of or consist of scFv anti*-SmaseD* from the venom from *Loxosceles intermedia* (Li), or a dimeric protein complex comprising a first, second, third and fourth functional component, wherein the first, second, third and fourth functional component comprise, consist essentially of or consist of scFv anti-*SmaseD* from the venom from Li.

A further aspect provides, the dimeric protein complex as taught herein, the nucleic acid as taught herein, the expression cassette as taught herein, the expression vector as taught herein, or the pharmaceutical composition as taught herein, for use as a medicament, preferably for use in immunotherapy.

A further aspect provides, the dimeric protein complex as taught herein, the nucleic acid as taught herein, the expression cassette as taught herein, the expression vector as taught herein, or the pharmaceutical composition as taught herein, for use in the treatment of a neoplastic disease or an infectious disease. In other words, provided herein is a method of treating a neoplastic disease or an infectious disease in a subject in need of such a treatment, comprising administering a therapeutically effective amount of the dimeric protein complex as taught herein, the nucleic acid as taught herein, the expression cassette as taught herein, the expression vector as taught herein, or the pharmaceutical composition as taught herein.

Furthermore, also provided herein is the use of the dimeric protein complex as taught herein, the nucleic acid as taught herein, the expression cassette as taught herein, the expression vector as taught herein, or the pharmaceutical composition as taught herein, for the manufacture of a medicament for the treatment of a neoplastic disease or an infectious disease.

Furthermore, also provided herein is the dimeric protein complex as taught herein, the nucleic acid as taught herein, the expression cassette as taught herein, the expression vector as taught herein, or the pharmaceutical composition as taught herein, for use in the treatment of a venomous bite, from for example an insect, spider or snake. For example, as described elsewhere in the present specification, if one or more of functional components comprises, consists essentially of or consists of scFv anti-*SmaseD* from the venom from *Loxosceles intermedia* (Li), the dimeric protein complex may bind to the *SmaseD* and inhibit its activity, and therefore may treat a venomous bite from *Loxosceles intermedia.*

In particular embodiments, the dimeric protein complex as taught herein, the nucleic acid as taught herein, the expression cassette as taught herein, the expression vector as taught herein, or the pharmaceutical composition as taught herein is used to treat dermo-necrotic events following envenomation (i.e. the process by which venom is injected by the bite of a spider).

The terms "treat" or "treatment" encompass both the therapeutic treatment of an already developed disease or condition, such as the therapy of an already developed neoplastic disease or infectious disease, as well as prophylactic or preventive measures, wherein the aim is to prevent or lessen the chances of incidence of an undesired affliction, such as to prevent occurrence, development and progression of a neoplastic disease or an infectious disease. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "Treatment" can also mean prolonging survival as compared to expected survival if not receiving treatment.

Except when noted, the terms "subject" or "patient" can be used interchangeably and refer to animals, preferably warm-blooded animals, more preferably vertebrates, even more preferably mammals, still more preferably primates, and specifically includes human patients and non-human mammals and primates. Preferred subjects are human subjects. The terms "subject" or "patient" include subjects in need of treatment, more particularly subjects that would benefit from treatment of a given condition, particularly a neurovascular disorder or a central nervous system (CNS) disorder comprising neurovascular dysfunction. Such subjects may include, without limitation, those that have been diagnosed with said condition, those prone to develop said condition and/or those in who said condition is to be prevented.

The products and methods as taught herein allow to administer a therapeutically and/or prophylactically effective amount of the dimeric protein complex as taught herein, the nucleic acid as taught herein, the expression cassette as taught herein, the expression vector as taught herein, or the pharmaceutical composition as taught herein in subjects having a neoplastic disease or infectious disease which will benefit from such treatment. The term "therapeutically effective amount" as used herein, refers to an amount of active compound or pharmaceutical agent that elicits the biological or medicinal response in a subject that is being sought by a surgeon, researcher, veterinarian, medical doctor or other clinician, which may include inter alia alleviation of the symptoms of the disease or condition being treated. The term "prophylactically effective amount" refers to an amount of an active compound or pharmaceutical agent that inhibits or delays in a subject the onset of a disorder as being sought by a researcher, veterinarian, medical doctor or other clinician. Methods are known in the art for determining therapeutically and/or prophylactically effective doses of the dimeric protein complex as taught herein, the nucleic acid as taught herein, the expression cassette as taught herein, the expression vector as taught herein, or the pharmaceutical composition as taught herein.

The term "therapeutically effective dose" as used herein refers to an amount of the dimeric protein complex as taught herein, the nucleic acid as taught herein, the expression cassette as taught herein, the expression vector as taught herein, or the pharmaceutical composition as taught herein, that when administered brings about a positive therapeutic response with respect to treatment of a patient having a neoplastic disease or an infectious disease.

Appropriate therapeutically effective doses of the dimeric protein complex as taught herein, the nucleic acid as taught herein, the expression cassette as taught herein, the expression vector as taught herein, or the pharmaceutical composition as taught herein, may be determined by a qualified physician with due regard to the nature of the disease condition and severity, and the age, size and condition of the patient.

The term "neoplastic disease" generally refers to any disease or disorder characterized by neoplastic cell growth and proliferation, whether benign (not invading surrounding normal tissues, not forming metastases), pre-malignant (pre-cancerous), or malignant (invading adjacent tissues and capable of producing metastases). The term neoplastic disease generally includes all transformed cells and tissues and all cancerous cells and tissues. Neoplastic diseases or disorders include, but are not limited to abnormal cell growth, benign tumors, premalignant or precancerous lesions, malignant tumors, and cancer. Examples of neoplastic diseases or disorders are benign, pre-malignant, or malignant neoplasms located in any tissue or organ, such as in the prostate, colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, or urogenital tract.

As used herein, the terms "tumor" or "tumor tissue" refer to an abnormal mass of tissue that results from excessive cell division. A tumor or tumor tissue comprises tumor cells which are neoplastic cells with abnormal growth properties and no useful bodily function. Tumors, tumor tissue and tumor cells may be benign, pre-malignant or malignant, or may represent a lesion without any cancerous potential. A tumor or tumor tissue may also comprise tumor-associated non-tumor cells, e.g., vascular cells which form blood vessels to supply the tumor or tumor tissue. Non-tumor cells may be induced to replicate and develop by tumor cells, for example, the induction of angiogenesis in a tumor or tumor tissue.

As used herein, the term "cancer" refers to a malignant neoplasm characterized by deregulated or unregulated cell growth. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor). The term "metastatic" or "metastasis" generally refers to the spread of a cancer from one organ or tissue to another non-adjacent organ or tissue. The occurrence of the neoplastic disease in the other non-adjacent organ or tissue is referred to as metastasis.

Examples of cancer include but are not limited to carcinoma, lymphoma, blastoma, sarcoma, and leukemia or lymphoid malignancies. More particular examples of such cancers include without limitation: squamous cell cancer (e.g., epithelial squamous cell cancer), lung cancer including small-cell lung cancer, non-small cell lung cancer, adenocarcinoma of the lung, squamous carcinoma of the lung and large cell carcinoma of the lung, cancer of the peritoneum, hepatocellular cancer, gastric or stomach cancer including gastrointestinal cancer, pancreatic cancer, glioma, glioblastoma, cervical cancer, ovarian cancer, liver cancer, bladder cancer, hepatoma, breast cancer, colon cancer, rectal cancer, colorectal cancer, endometrial cancer or uterine carcinoma, salivary gland carcinoma, kidney or renal cancer, prostate cancer, vulvar cancer, thyroid cancer, hepatic carcinoma, anal carcinoma, penile carcinoma, as well as CNS cancer, melanoma, head and neck cancer, bone cancer, bone marrow cancer, duodenum cancer, esophageal cancer, thyroid cancer, or hematological cancer.

In certain embodiments, the tumor is a solid tumor. Solid tumors encompass any tumors forming a neoplastic mass that usually does not contain cysts or liquid areas. Solid tumors may be benign, pre-malignant or malignant. Examples of solid tumors are carcinomas, sarcomas, melanomas and lymphomas.

In certain embodiments, the neoplastic disease or cancer is a neoplastic disease or cancer comprising cells expressing one or more antigens targeted by the dimeric protein complex as taught herein.

In particular embodiments, if the disease to be treated is a neoplastic disease, the dimeric protein complex as taught herein at least one of the first, second, third, and fourth functional components is a binding domain (e.g. antigen recognition domain) specifically binding to a TSA or TAA. Accordingly, the person skilled in the art will understand that if the dimeric protein complex as taught herein comprises a functional component comprising, consisting essentially of or consisting of a binding domain (e.g. antigen recognition domain) specifically binding to HER2, the neoplastic disease may be a HER2-positive breast cancer.

In particular embodiments, the infectious disease may be any infectious disease known in the art, such as an infectious disease caused by a pathogen, like bacteria, virus or fungi. Non-limiting examples of such infectious disease are *P. aeruginosa* infection, *Bordetella pertussis* infection, *Bordetella burgdorferi* infection, *Borrelia recurrentis* infection, *Haemophilus influenza* infection, *Moraxella catarrhalis* infection, *Neisseria gonorrhoeae* infection, *Neisseria meningitidis* infection, *Streptococcus pneumoniae* & *pyogenes* infection, *Yersinia enterocolitica* and *Staphylococcus aureus* infection, *Candida dubliensis* infection, *E*. *coli* infection, *Pasteurella pneomotropica* infection, *Aspergillus fumigatus & terreus* infection, Human Cytomegalovirus (HCMV) infection, Hepatitis C virus (HCV) infection, Human papilloma virus (HPV) infection, Epstein-Barr Virus (EBV) infection, Hepatitis Delta Virus (HDV) infection, Influenza virus infection, tuberculosis infection, Hepatitis B virus (HBV) infection, Human immunodeficiency virus (HIV) infection, severe acute respiratory syndrome (SARS)-CoV infection, *Candida albicans* infection *Aspregillus fumigatus* infection, *Toxoplasma gondii* infection.

In particular embodiments, if the disease to be treated is an infectious disease, the dimeric protein complex as taught herein at least one of the first, second, third, and fourth functional components is a binding domain (e.g. antigen recognition domain) specifically binding to a viral antigen, a virus-associated antigen, a bacterial antigen or fungal antigen.

In particular embodiments, if the disease to be treated is an infectious disease caused by a pathogen that evades the human complement attack, the dimeric protein complex as taught herein at least one of the first, second, third, and fourth functional components comprise, consist essentially of or consist of SCR3, 4 and 5 from FHR1 as described elsewhere in the present specification.

In particular embodiments, if the disease to be treated is an infectious disease caused by a pathogen that evades complement-mediated killing through the recruitment of C4bp soluble complement regulatory proteins, the dimeric protein complex as taught herein at least one of the first, second, third, and fourth functional components comprise, consist essentially of or consist of CCP1 and CCP2 from the C4bp N-terminal alpha-chain as described elsewhere in the present specification. Non-limiting examples of pathogens that hijack C4bp through CCP1-2 binding include *Streptococcus pneomoniae and pyogenes*, *Staphylococcus aureus*, *Escherichia coli*, *Candida albicans*, *Aspregillus fumigatus*, and *Toxoplasma gondii.*

In particular embodiments, the disease to be treated is an infectious disease caused by a pathogen resistant to antibiotics, such as *Pseudomonas aeruginosa.*

A further aspect provides, the dimeric protein complex as taught herein, the nucleic acid as taught herein, the expression cassette as taught herein, the expression vector as taught herein, for use in a method of molecular imaging of a living body.

In particular embodiments, if the dimeric protein complex as taught herein is used in a method of molecular imaging of a living body, the dimeric protein complex comprises at least one functional component comprising a binding domain (e.g. antigen recognition domain) and further comprises at least one molecular imaging probe.

Non-limiting examples of molecular imaging probes are ¹⁸F, ¹¹C, or ⁶⁴Cu (for use in positron emission tomography (PET)), ^{99m}Tc (for use in single photon emission computed tomography (SPECT)), magnetically active elements such as iron oxide nanoparticles, and luciferase (for use in diagnostic molecular imaging).

The present application also provides aspects and embodiments as set forth in the following Statements:
Statement 1. A dimeric protein complex comprising
   a first polypeptide comprising a first functional component and the C-terminal fragment of the C4bp beta-chain, wherein said first functional component is coupled to the C-terminus of the C-terminal fragment of the C4bp beta-chain; and
   a second polypeptide comprising a second functional component and the C-terminal fragment of the C4bp beta-chain, wherein said second functional component is coupled to the C-terminus of the C-terminal fragment of the C4bp beta-chain;
   wherein said first and second polypeptides are identical or different, and
   wherein said first and second functional components are proteins or polypeptides.
Statement 2. The dimeric protein complex according to statement 1,
   wherein said first polypeptide further comprises a third functional component that is coupled to the N-terminus of said C-terminal fragment of the C4bp beta-chain; and
   wherein said second polypeptide further comprises a fourth functional component that is coupled to the N-terminus of said C-terminal fragment of the C4bp beta-chain;
   wherein said first, said second, said third and said fourth functional components are identical or different.
Statement 3. The dimeric protein complex according to statement 2, wherein said first, said second, said third and said fourth functional component is a protein or polypeptide, preferably a protein or polypeptide selected from the group consisting of an antigen recognition domain, a recombinant viral structural protein, an oncolytic agent, a cytotoxic agent, a cytokine, a receptor-binding peptide, or monomeric Fc.
Statement 4. The dimeric protein complex according to statement 3, wherein said antigen recognition domain is a single chain variable fragment of an antibody (scFv) or a single domain variable fragment of a heavy chain antibody (V_{H}H) specific for the antigen, an antibody-like scaffold, an extracellular domain of a viral envelope protein, a cognate extracellular domain of a receptor or ligand for the antigen or an antigen-binding portion of said receptor or ligand, a soluble receptor, or a synthetic receptor.
Statement 5. The dimeric protein complex according to statement 3 or 4, wherein said antigen recognition domain specifically binds to a tumor specific antigen (TSA), a tumor associated antigen (TAA), a bacterial antigen, a viral antigen or a virus-associated antigen, a fungal antigen, an activating NK cell receptor, a cytokine, a toxin or a contaminant.
Statement 6. The dimeric protein complex according to any one of statements 2 to 5, wherein two of said first, said second, said third or said forth functional components comprise an antigen recognition domain specifically binding to an activating NK cell receptor, wherein binding of the antigen recognition domain to the activating NK cell receptor is capable of activating the NK cell; and wherein two of said first, said second, said third or said forth functional components comprise an antigen recognition domain specifically binding to a TSA, a TAA, a bacterial antigen, a viral antigen, a virus-associated antigen, or a fungal antigen.
Statement 7. The dimeric protein complex according to any one of statements 2 to 6, wherein
   the first and second functional components are monomeric Fc, preferably monomeric Fc of IgG comprising the hinge, CH2 domain and CH3 domain of IgG; and wherein the hinge region of the monomeric Fc in the first polypeptide is connected to the hinge region of the monomeric Fc in the second polypeptide by at least two disulfide bonds; and
   the third and/or fourth functional component comprises an antigen recognition domain.
Statement 8. A nucleic acid
   encoding the first polypeptide of the dimeric protein complex comprising a first functional component and a C-terminal fragment of the C4bp beta-chain, wherein said first functional component is coupled to the C-terminus of the C-terminal fragment of the C4bp beta-chain as defined in any one of statements 1 to 7; and/or
   encoding the second polypeptide of the dimeric protein complex comprising a second functional component and a C-terminal fragment of the C4bp beta-chain, wherein said second functional component is coupled to the C-terminus of said C-terminal fragment of the C4bp as defined in any one of statements 1 to 7.
Statement 9. An expression cassette comprising the nucleic acid according to statement 8.
Statement 10. An expression vector comprising the nucleic acid according to statement 8 or the expression cassette according to statement 9.
Statement 11. An expression vector comprising a nucleic acid encoding the C-terminal fragment of the C4bp beta-chain, an insertion site for a nucleic acid encoding a functional component located immediately 3' of the nucleic acid encoding the C-terminal fragment C4bp beta-chain; and optionally an insertion site for a nucleic acid encoding a functional component immediately 5' of the nucleic acid encoding the C-terminal fragment of the C4bp beta-chain.
Statement 12. A pharmaceutical composition comprising the dimeric protein complex according to any one of statements 1 to 7, the nucleic acid according to statement 8, the expression cassette according to statement 9, or the expression vector according to statement 10 or 11, and a pharmaceutically acceptable carrier.
Statement 13. The dimeric protein complex according to any one of statements 1 to 7, the nucleic acid according to statement 8, the expression cassette according to statement 9, the expression vector according to statement 10 or 11, or the pharmaceutical composition according to statement 12, for use as a medicament, preferably for use in immunotherapy.
Statement 14. The dimeric protein complex according to any one of statements 1 to 7, the nucleic acid according to statement 8, the expression cassette according to statement 9, the expression vector according to statement 10 or 11, or the pharmaceutical composition according to statement 12, for use in the treatment of a neoplastic disease or an infectious disease.
Statement 15. The dimeric protein complex according to any one of statements 1 to 7, the nucleic acid according to statement 8, the expression cassette according to statement 9, the expression vector according to statement 10 or 11, for use in a method of molecular imaging of a living body.

The present application also provides aspects and embodiments as set forth in the following Statements' :
Statement 1'. A dimeric protein complex comprising
   a first polypeptide comprising a first functional component and the C-terminal fragment of the C4b binding protein (C4bp) beta-chain, wherein said first functional component is coupled to the C-terminus of the C-terminal fragment of the C4bp beta-chain; and
   a second polypeptide comprising a second functional component and the C-terminal fragment of the C4bp beta-chain, wherein said second functional component is coupled to the C-terminus of the C-terminal fragment of the C4bp beta-chain;
   wherein said first and second functional components are monomeric Fc; and wherein the hinge region of the monomeric Fc in the first polypeptide is connected to the hinge region of the monomeric Fc in the second polypeptide by at least two disulfide bonds;
   wherein said first polypeptide further comprises a third functional component that is coupled to the N-terminus of said C-terminal fragment of the C4bp beta-chain; and
   wherein said second polypeptide further comprises a fourth functional component that is coupled to the N-terminus of said C-terminal fragment of the C4bp beta-chain;
   wherein said third and/or fourth functional component comprises a binding domain; and
   wherein said first and second polypeptides are identical or different
Statement 2'. The dimeric protein complex according to statement 1', wherein said binding domain is a single chain variable fragment of an antibody (scFv) or a single domain variable fragment of a heavy chain antibody (VHH) specific for the antigen, an antibody-like scaffold, an extracellular domain of a viral envelope protein, a cognate extracellular domain of a receptor or ligand for the antigen or an antigen-binding portion of said receptor or ligand, a soluble receptor, or a synthetic receptor.
Statement 3' . The dimeric protein complex according to statement 1' or 2', wherein said binding domain specifically binds to a tumor specific antigen (TSA), a tumor associated antigen (TAA), a bacterial antigen, a viral antigen or a virus-associated antigen, a fungal antigen, an activating NK cell receptor, a cytokine, a toxin or a contaminant.
Statement 4'. The dimeric protein complex according to statement 3', wherein said third and said fourth functional components comprise complement control protein (CCP) 1 and 2 of the C4bp alpha-chain.
Statement 5'. The dimeric protein complex according to any one of statements 1' to 4', wherein
   the first and second functional components are monomeric Fc of IgG comprising the hinge, CH2 domain and CH3 domain of IgG.
Statement 6'. A nucleic acid
   encoding the first polypeptide of the dimeric protein complex as defined in any one of statements 1' to 5'; and/or
   encoding the second polypeptide of the dimeric protein complex as defined in any one of statements 1' to 5'.
Statement 7'. An expression cassette comprising the nucleic acid according to statement 6'.
Statement 8'. An expression vector comprising the nucleic acid according to statement 6' or the expression cassette according to statement 7'.
Statement 9'. A pharmaceutical composition comprising the dimeric protein complex according to any one of statements 1' to 5', the nucleic acid according to statement 6', the expression cassette according to statement 7', or the expression vector according to statement 8', and a pharmaceutically acceptable carrier.
Statement 10'. The dimeric protein complex according to any one of statements 1' to 5', the nucleic acid according to statement 6', the expression cassette according to statement 7', the expression vector according to statement 8', or the pharmaceutical composition according to statement 9', for use as a medicament, preferably for use in immunotherapy.
Statement 11'. The dimeric protein complex according to any one of statements 1' to 5 ', the nucleic acid according to statement 6', the expression cassette according to statement 7', the expression vector according to statement 8', or the pharmaceutical composition according to statement 9', for use in the treatment of a neoplastic disease or an infectious disease.

The present application also provides aspects and embodiments as set forth in the following Statements":
Statement 1". A dimeric protein complex comprising
   a first polypeptide comprising a first functional component and the C-terminal fragment of the C4b binding protein (C4bp) beta-chain, wherein said first functional component is coupled to the C-terminus of the C-terminal fragment of the C4bp beta-chain; and
   a second polypeptide comprising a second functional component and the C-terminal fragment of the C4bp beta-chain, wherein said second functional component is coupled to the C-terminus of the C-terminal fragment of the C4bp beta-chain;
   wherein said first polypeptide further comprises a third functional component that is coupled to the N-terminus of said C-terminal fragment of the C4bp beta-chain;
   wherein said second polypeptide further comprises a fourth functional component that is coupled to the N-terminus of said C-terminal fragment of the C4bp beta-chain;
   wherein said first, second, third and fourth functional components are proteins or polypeptides; wherein two of said first, second, third and/or fourth functional components comprise a binding domain;
   wherein said first and second polypeptides are identical or different; and
   wherein the C-terminal fragment of the C4bp beta-chain is the 194-252 fragment of the C4bp beta-chain. The "194-252" refers to the amino acid positions of the C4bp beta-chain protein. Statement 2". The dimeric protein complex according to statement 1", wherein the C-terminal fragment of the C4bp beta-chain consists of the amino acid sequence as set forth in SEQ ID NO: 1, or an amino acid sequence having at least 95% sequence identity to SEQ ID NO: 1.
Statement 3". The dimeric protein complex according to statement 1" or 2", wherein said third and fourth functional components comprise a binding domain.
Statement 4". The dimeric protein complex according to any one of statements 1" to 3", wherein said binding domain is a single chain variable fragment of an antibody (scFv) or a single domain variable fragment of a heavy chain antibody (VHH) specific for the antigen, an antibody-like scaffold, an extracellular domain of a viral envelope protein, a therapeutic agent, a cognate extracellular domain of a receptor or ligand for the antigen or an antigen-binding portion of said receptor or ligand, a soluble receptor, or a synthetic receptor.
Statement 5". The dimeric protein complex according to any one of statements 1" to 4", wherein said binding domain specifically binds to a tumor specific antigen (TSA), a tumor associated antigen (TAA), a bacterial antigen, a viral antigen or a virus-associated antigen, a fungal antigen, an NK cell receptor (preferably an activating NK cell receptor), a cytokine, a toxin or a contaminant.
Statement 6". The dimeric protein complex according to any one of statements 1" to 5", wherein two of said first, said second, said third or said forth functional components comprise a binding domain specifically binding to an NK cell receptor (preferably an activating NK cell receptor); and wherein two of said first, said second, said third or said forth functional components comprise a binding domain specifically binding to a TSA, a TAA, a bacterial antigen, a viral antigen, a viral-associated antigen or a fungal antigen.
Statement 7". The dimeric protein complex according to statement 6",
   wherein the third and fourth functional components comprise a scFv specifically binding NKG2A or a scFv specifically binding KIR; optionally wherein the first and second functional components comprise a soluble IL-15 receptor alpha chain (sIL15Ralpha) or a complement receptor type 1.
Statement 8". The dimeric protein complex according to statement 6", wherein the third and fourth functional components comprise a scFv specifically binding PsI or PcrV of *P. Aeruginosa*; optionally wherein the first and/or second functional components comprise a scFv specifically binding NKG2D and/or SLAMF7.
Statement 9". The dimeric protein complex according to any one of statements 1" to 5", wherein two of said first, said second, said third or said forth functional components comprise SCR3, 4 and 5 from factor H-related protein 1 (FHR1); and wherein two of said first, said second, said third or said forth functional components comprise a binding domain specifically binding to a TSA, a TAA, a bacterial antigen, a viral antigen, a virus-associated antigen, or a fungal antigen. Statement 10". The dimeric protein complex according to statement 9", wherein the third and fourth functional components comprise SCR 3, 4 and 5 from FHR1; and optionally wherein the first and/or second functional components comprise a scFv specifically binding PsI. Statement 11". The dimeric protein complex according to any of statements 1" to 5", wherein the first, second, third and/or fourth functional components comprise a scFv specifically binding sphingomyelinase D from *Loxosceles,* preferably *Loxosceles intermedia* or *Loxosceles reclusa*, more preferably *Loxosceles intermedia .*
Statement 12". A dimeric protein complex comprising
   a first polypeptide comprising a first functional component and the C-terminal fragment of the C4b binding protein (C4bp) beta-chain, wherein said first functional component is coupled to the C-terminus of the C-terminal fragment of the C4bp beta-chain; and
   a second polypeptide comprising a second functional component and the C-terminal fragment of the C4bp beta-chain, wherein said second functional component is coupled to the C-terminus of the C-terminal fragment of the C4bp beta-chain;
   wherein said first polypeptide further comprises a third functional component that is coupled to the N-terminus of said C-terminal fragment of the C4bp beta-chain;
   wherein said second polypeptide further comprises a fourth functional component that is coupled to the N-terminus of said C-terminal fragment of the C4bp beta-chain;
   wherein said first, second, third and fourth functional components are proteins or polypeptides; wherein two of said first, second, third and/or fourth functional components comprise a binding domain;
   wherein said first and second polypeptides are identical or different;
   wherein the C-terminal fragment of the C4bp beta-chain is the 194-252 fragment of the C4bp beta-chain; and
   wherein two of said first, said second, said third or said forth functional components comprise a binding domain specifically binding to an activating NK cell receptor, wherein binding of the binding domain to the activating NK cell receptor is capable of activating the NK cell; and wherein two of said first, said second, said third or said forth functional components comprise a binding domain specifically binding to a TSA, a TAA, a bacterial antigen, a viral antigen, a viral-associated antigen or a fungal antigen.
Statement 13". A nucleic acid encoding the first polypeptide of the dimeric protein complex as defined in any one of statements 1" to 12"; and/or encoding the second polypeptide of the dimeric protein complex as defined in any one of statements 1" to 12".
Statement 14". An expression cassette comprising the nucleic acid according to statement 13". Statement 15". An expression vector comprising the nucleic acid according to statement 13" or the expression cassette according to statement 14".
Statement 16". A pharmaceutical composition comprising the dimeric protein complex according to any one of statements 1" to 12", the nucleic acid according to statement 13", the expression cassette according to statement 14", or the expression vector according to statement 15", and a pharmaceutically acceptable carrier.
Statement 17". The dimeric protein complex according to any one of statements 1" to 12", the nucleic acid according to statement 13", the expression cassette according to statement 14", the expression vector according to statement 15", or the pharmaceutical composition according to statement 16", for use as a medicament.
Statement 18". The dimeric protein complex according to any one of statements 1" to 12", the nucleic acid according to statement 13", the expression cassette according to statement 14", the expression vector according to statement 15", or the pharmaceutical composition according to statement 16", for use in the treatment of a neoplastic disease or an infectious disease. Statement 19". The dimeric protein complex according to any one of statements 1" to 12", the nucleic acid according to statement 13", the expression cassette according to statement 14", the expression vector according to statement 15", for use in a method of molecular imaging of a living body.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and scope of the appended claims.

The herein disclosed aspects and embodiments of the invention are further supported by the following non-limiting examples.

### EXAMPLES

### 1. Example 1. Bifunctional heterodimers (TetraBolos) capable of activating NK cells

### 1.1. Introduction

NKG2A (CD159a) is a type II transmembrane protein always co-expressed with CD94 which forms a disulphide-linked NKG2A/CD94 heterodimeric complex. NKG2A is a member of the Ca⁺⁺-dependent (C-type) lectin family. The CD94/NKG2A dimer is an inhibitory NK receptor for HLA class I histocompatibility antigen, α-chain E (*HLA-E*). The cytoplasmic domain of NKG2A contains two characteristic ITIM motifs (Immunoreceptor tyrosine based inhibition motif) involved in the transduction of the inhibitory signal. HLA-E has a very specialized role in self and non-self-cell recognition by NK cells. HLA-E molecules are expressed at low levels in most tissues, whereas, it is commonly overexpressed on the surface of a variety of different cancers. High HLA-E⁺ tumors thus deliver a potent inhibitory signal upon NKG2A/CD94/HLA-E interaction that leads to inhibition of NK cell function. Z199 is a mouse monoclonal antibody (IgG2b) which is a non-competitive antagonist of the human NKG2A (CD159a) receptor. Present inventors have generated a scFv that is derived from the Z199 mAb as a blocker of human NK inhibitory pathways, comprising an amino acid sequence as defined by SEQ ID NO: 10 and a nucleic acid sequence as defined by SEQ ID NO: 11, which may be referred to in the present specification as "anti-NKG2A scFv".

IL-15 is a regulatory cytokine from the common γc chain family comprising IL-2, IL-4, IL-7, IL-9 and IL-21. IL-15 in its heterotrimeric receptor with IL-2 use the cytokine-specific receptor α-chains IL-15Rα (CD215). IL-2 and IL-15 stimulate proliferation of T cells, generation of cytotoxic lymphocytes, and stimulate the expansion of natural killer (NK) cells. In contrast to IL-2, IL-15 inhibits IL-2-mediated Activated Induced Cell Death and does not activate functional Tregs. IL-15 predominately acts as a cell-surface molecule as part of an immunological synapse with IL-15Rα on antigen-presenting cells providing IL-15 in trans to mononuclear cells such as NK and CD8 memory cells. IL-15 alone is very hard to produce, due to its short half-life, low solubility and strict transcriptomic regulation.

The IL15/sIL15Rα soluble complexes are much more stimulatory than soluble IL15 alone (Rubinstein, Kovar et al. 2006). The complex may prevent IL15 internalization, thereby strengthening signaling through βγ_{c} receptor. IL15Rα might also prevent the degradation of IL15 when complexed IL15Rα soluble receptor.

In the present example, presented inventors aimed at developing bifunctional heterodimers that associate (i) the IL-15/IL-15Rα soluble complex to (ii) the Z199 scFv anti-NKG2A using the 58 amino acid C4bp C-terminal β-chain dimerization scaffold (C4bpβ) to activate NK cells by delivering IL-15 at the surface of NK cells. The gene encoding the soluble IL-15 receptor α-chain (sIL-15Rα) is located upstream of the C4bpβ, while the gene encoding the Z199 scFv is located downstream of the C4bpβ as shown in Figure 3A. Each of the two functions is dimeric; HeteroTetraBolo "IL-15/sIL-15Rα.C4bpβ.scFv anti-NKG2A.His8x" concomitantly activates two distinct NK pathways through IL-15/sIL-15Rα complexes and the blockade of NKG2A receptors.

### 1.2. Genes used and cassette design

### Gene used:

- Signal peptide (SP) from the tumor necrosis factor receptor superfamily member 16 (NGFR): UniProtKB - P08138.1 TNR16_HUMAN, amino acids 1-28;
- Soluble recombinant human IL-15 receptor α-chain (sIL-15Rα) : UniProt n°Q13261.1, amino acids 31-205);
- Human C4b binding protein C-terminal β-chain (C4bpβ) dimerization scaffold: UniProtKB - P20851.1 C4BPB_HUMAN, amino acids 194-252. 3x(SGGGGS) (SEQ ID NO: 3) linkers are cloned on both ends of the C-terminal part of C4bpβ;
- Z199 (V_{H}-V_{L}) anti-NKG2A scFv, such as described in International patent applicationWO2009/092805, which is incorporated here by reference;
- Human interleukin 15 (hIL-15): UniProtKB - P40933.1 IL-15_HUMAN, amino acids 49-162. IL-15 is preceded by the optimized 35 amino acid tPA signal peptide for secretory expression in eukaryotic cells from Proteogenix (MDAMKRGLCCVLLLCGAVFVSPSQEIHARFRRGAR (SEQ ID NO: 44)). IL-15 gene was cloned in pcDNA3.1 (containing G418 resistance gene for stable expression) by Proteogenix;
- pEF-IRESpac is the bicistronic expression recipient vector used driven by the human polypeptide chain elongation factor 1α promoter to generate stable mammalian cell lines that express high levels of recombinant proteins. The plasmid contains the puromycin resistant gene (*pac*) for stable expression of recombinant proteins.

### The cassettes for the heteroTetraBolos:

- SP-sIL-15Rα-[3x(SGGGGS)]-C4bpβ-[3x(SGGGGS)]-Z199 scFv anti-NKG2A-His8x cloned in *pEF-IRESpac*
- tPA SP.hIL-15, cloning in pcDNA3.1.

The cDNA encoding the cassette (sIL-15Rα.C4bpβ.scFv anti-NKG2A) spanned between the Bgl2 *&* NotI restriction sites were codon-optimized for expression in human cells, synthesized and cloned into pEF-IRESp by Proteogenix (Illkirsch, Strasbourg). Figure 3 depicts the steps of transfections of the constructs: bifunctional HeteroTetraBolos (Figure 3A, 3B).

### 1.3. Transfection in HEK293F cells, cloning/screening, production, scaled-up cultures, purification using immobilized metal affinity chromatography (IMAC) technology with 1 or 5 ml Ni-sepharose Excel columns, FPLC

One day before the transfection, 1.3 × 10⁶ HEK293F cells were plated in 6-well cell culture plate and cultured in DMEM complete medium. Two hours before the transfection, complete DMEM was replaced by pre-warmed optiMEM. HEK293F cells either were transfected with the expression vectors coding the heteroTetraBolos alone or were co-transfected together with pcDNA3.1-hIL-15 using lipofectamine 3000 according to the manufacturer protocol (Thermo Fisher, catalog number L3000001). 4 µg of DNA were transfected with 5 µl of lipofectamine and 4 µL of reagent. A DNA ratio 1: 1 was used for the co-transfection. 1ml of complete DMEM medium was added 24h after the transfection. Forty-eight hours later, cells were transferred in 10-cm cell culture dish and cultured in complete DMEM medium supplemented with 5-20 µg/ml puromycin for the single transfection, or with 5-20 µg/ml of puromycin and 100-500 µg/ml Geneticin (G418) for the co-transfection. About 2 weeks later, antibioticresistant growing single clones were individually transferred into 96-well plates. About a week later, when the clones reach a certain density and the culture medium turns yellowish, supernatants from single isolated clones were screened for heteroTetraBolo expression using anti-IL-15/anti-HIS and anti-HIS/anti-HIS sandwich ELISA to identify the highest bifunctional heteroTetraBolo expression levels. Then, the selected cell clones with the best protein expression were expanded. Subsequently, cells were amplified in large culture flasks and transferred into 5-chamber cell stacks and left for 24h in 500 ml complete DMEM medium. The next day, DMEM was replaced by 500 ml optiMEM and left for 48h as first round of production. Then, a boost in complete DMEM was performed for 24h, followed by a second round of production for 48h in 500 ml fresh optiMEM. Then, the optiMEM was loaded onto the His-Trap column: the 2 productions were pooled (making 1L), filtered using 1000 ml *Nalgene*^{™} Rapid-Flow^{™} vacuum filter units. The supernatants were passed through a 5 ml excel column for 5 days in a closed loop at 2 ml/min flow rate to immobilize the bifunctional HeteroTetraBolos. The column was then connected to a FPLC. After washing, the molecules were eluted using stepwise imidazole gradients. The eluates were concentrated using Amicon^{®} Ultra-15 centrifugal filter units with 30-kDa molecule weight cut off (MWCO). The molecules were then dialyzed against PBS to remove the imidazole.

### 1.4. Western blotting analysis of the bifunctional HeteroTetraBolos

The following purified molecules were analyzed using Western blotting (WB) under non-reducing or reducing conditions:
1. Bifunctional HeteroTetraBolos: sIL-15Rα.C4bpβ.scFv anti-NKG2A.His8x + huIL-15
2. Bifunctional HeteroTetraBolos: sIL-15Rα.C4bpβ.scFv anti-NKG2A.His8x

One µg of purified HeteroTetraBolos from transient transfections or 1 µg purified molecules from stable transfections were mixed to 4X Laemmli sample buffer without or with 10% (v/v) β2-mercaptoethanol (reducing conditions) and were electrophoresed using 4-15% Mini-Protean^{®} Tris-Glycin eXtended (TGX^{™}) precast protein gel (Bio-Rad) and SDS-polyacrylamide gel electrophoresis (SDS-PAGE) using XT MES running buffer (Bio-Rad). After electrophoresis, gel was electroblotted onto a low-fluorescence background PVDF membrane (activated in methanol prior to use) using TTB (25 mM Tris, 192 mM glycine, 0.01% (v/v) SDS, 20% (v/v) methanol, pH 8.8) blotting buffer. Protein transfer was carried out using a Bio-Rad Mini *Trans-*Blot^{®} electrophoretic transfer cell. After blocking with PBS-5% (w/v) low-fat milk, the membrane was incubated for 1h in the same buffer with 1µg rabbit anti-His 6x-tag pAb. After three washings with TBS-Tween buffer, pH 7.2, the membrane was incubated for 1h with a goat anti-rabbit IgG AF647-conjugated antibody in PBS-1% (w/v) low-fat milk. After three washings, the membrane was dried-off and analyzed using an Amersham^{™} Typhoon^{™} biomolecular imager using the adapted filter for AF647 (APCC) fluorochrome (Cy5). The scanned images were processed using the open source image-processing program "Image J". The WB shows that the in non-reducing conditions, the heteroTetraBolos are present as dimers and have a size of about 120 kDa (data not shown). Under reducing conditions, dimers are reduced into monomers with a size of 60 kDa (data not shown).

### 1.5. Characterization of the purified HeteroTetraBolos using anti-HIS or anti-IL-15 ELISA

Different ELISA set ups were used to evaluate the presence of the molecules in the crude supernatant or after purification. In this purpose, either purified molecules, mouse anti-human IL-15 (clone CT2NU; Invitrogen) or 6-His Tag rabbit anti-his polyclonal (Bethyl, A190-114-P) antibodies were coated on MaxiSorp^{™} 96-well flat-bottom polystyrene 96-well *ELISA* plate for 12h (in case of direct molecule coating) or for 72h when coated with the antibodies (100ng/100uL PBS/well). All ELISA washings were done with 1% PBS/BSA 5 times and all incubations were done for 1h at 4 degrees. After the first wash, the plate was blocked with 5% PBS/BSA, washed again and incubated with 100-200ul of the supernatants, purified molecules or rhuIL-15. After another washing, 100ng of mouse anti-HIS HRP-conjugated detection monoclonal antibody (SIGMA) per well was used for detection of the His tag. The IL-15 detection was done in two steps: first with 100ng per well of mouse anti-IL-15 and then with 100ng per well of rabbit anti-mouse conjugated to HRP (sigma; whole molecule). The revelation was done with 1× citrate buffer supplemented with chromogen substrate OPD and H₂O₂. The reaction was stopped with 0.5N H₂SO₄. Absorbance was read on the Polarstar plate reader at 492nm and 630nm.

### 1.5.1. Demonstration of the rhIL-15/sIL-15Rαα formed complexes in the purified sIL-15Rα.C4bpβ.scFv anti-NKG2A.His8x HeteroTetraBolos

The protein/anti-His ELISA demonstrated that the purified HeteroTetraBolos with or without IL-15 have a similar pattern of dose response binding to the ELISA plate. The Protein/anti-IL-15 ELISA demonstrated that only the sIL-15Rα.C4bpβ.scFv anti-NKG2A.His8x + hIL-15 display a dose-response signal specific to the presence of IL-15 complexed to the molecules (data not shown).

### 1.5.2. Demonstration of the hIL-15/sIL-15Rα formed complex on the HeteroTetraBolos after the purification process

To determine whether huIL-15 stays bound to the molecule after multiple steps of purification and dialysis, a sandwich ELISA coated with anti-IL-15 and revealed with anti-His was performed. It was demonstrated that a proper complex formation between hIL-15 and its co-receptor IL-15Rα on the HeteroTetraBolos occurs (data not shown). The sIL-15Rα.C4bpβ.scFv.anti-NKG2A.His8x without hIL-15 showed only background signal, indicating the absence of IL-15 in this construct (data not shown).

### 1.5.3. Demonstration of the hIL-15 saturation within the sIL-15Rα.C4bpβ.scFv.anti-NKG2A.His8x + hIL-15 HeteroTetraBolo

In order to show (i) the specific binding of rhuIL-15 to sIL-15Rα.C4bpβ.scFv anti-NKG2A bifunctional HeteroTetraBolos and (ii) to evaluate the rhuIL-15 saturation levels of His-Trap purified sIL-15Rα.C674bpβ.scFv anti-NKG2A bifunctional HeteroTetraBolos expressed from HEK293T cells that were co-transfected with a rhuIL-15 expression vector [sIL-15Rα.C4bpβ.scFv anti-NKG2A+rhuIL-15], we have set-up an ELISA. We used His-Trap purified sIL-15Rα.C4bpβ.scFv anti-NKG2A counterpart bifunctional HeteroTetraBolos expressed from HEK293T cells that were not co-transfected with a rhuIL15 expression vector [sIL-15Rα.C4bpβ.scFv anti-NKG2A] as a control molecules in this ELISA. An ELISA plate was coated with 6 µg/100µl/well [sIL-15Rα.C4bpβ.scFv anti-NKG2A+rhuIL-15] or [sIL-15Rα.C4bpβ.scFv anti-NKG2A]. After washing, the plate was incubated with a serial dilution of commercial rhuIL-15 (starting concentration 20 µg/ml), and revealed with a mouse anti-human IL-15 detection pAb followed by a rabbit anti-mouse IgG HRP-conjugated. After washing, the ELISA was revealed with 1× citrate buffer supplemented with OPD chromogenic substrate for HRP and H₂O₂. The reaction was stopped with 0.5N H₂SO₄. Absorbance was read using a spectrophotometer at 492nm and 630nm.
- Regarding the sIL-15Rα.C4bpβ.scFv anti-NKG2A bifunctional HeteroTetraBolos, the O.D varies from 0,05 to 2. The results of ELISA show that the 50% rhuIL-15 saturation for the sIL-15Rα.C4bpβ.scFv anti-NKG2A bifunctional HeteroTetraBolos is 0,15 ng (O.D.=1). The full saturation is reached between 10-100 ng rhuIL-15 (O.D. = 2).
- Regarding the sIL-15Rα.C4bpβ.scFv anti-NKG2A+rhuIL-15 bifunctional HeteroTetraBolos, the O.D varies from 1.55 to 1.95. We can consider that the rhuIL-15 saturation in the absence of additional commercial rhuIL-15 is about 75-80% saturation. Additional rhuIL-15 leads to a full saturation.

This experiment shows that the sIL-15Rα.C4bpβ.scFv anti-NKG2A+rhuIL-15 is already saturated with at least 75% rhuIL-15 when co-expressed with the rhuIL-15 expression vector and that the unoccupied rhuIL-15-free remaining valences are still able to capture additional rhu-IL-15 until full saturation.

### 1.5.4.Demonstration of the binding of the HeteroTetraBolos to NK cells from PBMCs or to the NK92MI cell line

To demonstrate the proper binding of the HeteroTetraBolos to target NK cells, the molecules were incubated either with PBMCs or with NK92MI cell lines and stained for IL-15 and His. On Figure 4 both HeteroTetraBolos showed an anti-His signal on NK cells from PBMCs and NK92MI cells, indicating a specific binding of the molecules to the target cells. In contrast, only cells incubated with sIL-15Rα.C4bpβ.scFv anti-NKG2A.His8x + hIL-15 were positively stained for anti-IL-15. These results indicate that the scFv anti-NKG2A cloned in a non-natural location downstream of the C4bp C-terminal β-chain dimerization scaffold, is able to bind to NK cells from PBMCs or to NK92MI cell line expressing NKG2A. Furthermore, it shows that the HeteroTetraBolos form a sIL-15Rα/IL-15 complexes that are robust and non-affected during the purification process.

### 1.5.5.Profile of CD107a/IFNγ expression on NK-cell pre-incubated with HeteroTetraBolos or rhuIL-15 and stimulated with RAJI target cells or latent HIV-1 infected ACH2 cells

The biological function and ability of the HeteroTetraBolos to activate NK cells from PBMCs in the presence of RAJI target cells (human B lymphoblastoid cell line from a patient with Burkitt lymphoma) and ACH-2 target cells (acute lymphoblastic leukemic T-cell line A3.01 infected with the HIV-1 strain LAI) was investigated. The Raji cell line is known for its resistance to NK cells through its expression of multiple major histocompatibility complexes, including the NKG2A ligand HLA-E. The latent ACH-2 cell line is a model for chronic HIV-1 infection that possesses a single integrated copy of HIV-1. The profiles of CD107a positive NK-cell - a marker that reflect the degranulation status of NK cells- and intracellular IFN-gamma expression - that reflects their cytolytic activities - were investigated. 1.5 × 10⁶ PBMCs were incubated in RPMI medium for 4 or 48 hours with 3 µg/ml of each molecule in a 24 well plate. PBMCs were further incubated with Raji cells and with ACH-2 cells at an E:T ratio of 10:1 in the presence of anti-CD107a- BV421. After 1h of incubation, GolgiStop^{™} and GolgiPlug^{™} (BD Biosciences) were added for another 4h, then washed and stained for extracellular markers (anti-CD3/BUV496, CD14/PE-Cy5, CD16/BUV737, CD19/PE-Cy5, CD56/BV786 and CD8-BV711 and L/D). Cells were permeabilized for 20 minutes following the cytoperm/cytofix manufacturer protocol (BD Biosciences, ref. 554714) and stained for intracellular IFN-γ/FITC.

After 48h pre-incubation with sIL-15Rα.C4bpβ.scFv.anti-NKG2A.His8x + hIL-15 or commercial rhIL-15, the CD107a positive NK cells were significantly increased compared to an incubation with medium alone. The data clearly indicate that the dimeric sIL-15Rα/hIL-15 complex has the same effect on NK cells than rhIL-15 when it comes to CD107a mediated degranulation after being stimulated by Raji cells but significantly enhanced NK cell degranulation as compared to rhIL-15 after being stimulated by latent HIV-1 infected ACH2-cells. The increase of CD107a positive NK cells after incubation with the HeteroTetraBolos without hIL-15 was not significant as compared to the medium control when stimulated with Raji cells but was significant when stimulated with latent HIV-1 infected ACH-2 cells. This indicates that blocking the NKG2A receptor with a scFv had only a small effect on the degranulation of NK cells. Furthermore, HeteroTetraBolos+hIL-15 elicited 60% IFNy-positive NK cells after 4h of pre-incubation with Raji cells and 25% IFNy-positive NK cells with ACH-2 cells, whereas HeteroTetraBolos without hIL-15 and the commercial rhIL-15 elicited only 2% IFNgamma-positive NK cells against Raji cells and 10% against ACH-2 cells. Regarding IFN-gamma-expression by NK cells, divalent sIL-15Rα/huIL-15 complex was more efficient than rhIL-15 alone (Figure 5).

### 1.5.6.Cytotoxic activity of NK cells against Raji cells after incubation with the HeteroTetraBolos or HeteroTetraBolos + rhuIL-15

In order to determine the ability of NK cells stimulated with the HeteroTetraBolos to lyse target cells, a cytotoxicity experiment was performed. 1.5 × 10⁶ PBMCs were incubated for 48 hours with 3µg of each molecule in 1ml of RPMI medium in a 24 well plate. Raji cells and ACH-2 cells stained with cell tracer violet (ThermoFisher, Ref. C34557) following the manufacturer's protocol were added at E:T ratio 10: 1 for another 24h. Cells were stained with live/dead (ThermoFisher, Ref. L34975) and analyzed by flow cytometry. Raji cells and ACH-2 cells incubated without NK cells were used as a negative control. Cytotoxicity was calculated as follows: ([control viable cell %]-[co-incubation viable cell %]) / (control viable cell %).

Figure 6 shows that sIL-15Rα.C4bpβ.scFv.anti-NKG2A.His8x + huIL-15 was able to significantly increase the cytotoxic activity of NK cells against resistant Raji cells and ACH-2 cells, whereas sIL-15Rα.C4bpβ.scFv.anti-NKG2A.His8x and commercial rhuIL-15 did not have an effect compared to the medium control when stimulated with Raji cells or showed a significantly lower effect than sIL-15Rα.C4bpβ.scFv.anti-NKG2A.His8x + huIL-15 when stimulated with ACH-2 cells.

### 2. Example 2. Redirected optimized NK-mediated killing of Pseudomonas aeruginosa using Trifunctional HeteroTetraBolos as trispecific killer engagers ("TriKEs")

### 2.1. Introduction

NK cells are innate lymphoid cells that exhibit the following functions:
- Cytokine production and cytotoxic response via perforin/granzyme release through granule exocytosis in the extracellular milieu.
- NK cells patrol in the circulation and in tissues to sense and differentiate normal or abnormal cells (infected or tumor cells) through activating and inhibitory receptors.
- NK cells play a key role in immunity against bacteria and tumors through (i) cytokine production and (ii) the release of cytotoxic granules.

NK cells sense their environment through inhibitory or activating receptors. NK cell response thus depends on the balance between activating and inhibitory signals, and the cytotoxic response of NK cells is consequently driven by an imbalance between inhibitory and activating ligands. *Pseudomonas aeruginosa* infection alters the membrane expression of activating receptors on the NK cell. NKG2D is involved in antitumor as well as antiviral and antibacterial immunity. NKG2D has been reported to participate in the bacterial clearance in a murine model of *P. aeruginosa* pneumonia. The expression of NKG2D significantly decreased after *P. aeruginosa* infection. As a result, the reduction of NKG2D expression could account for the altered cytotoxicity of NK cells after *P. aeruginosa* infection. SLAMF7 is a member of the SLAM family. Like most SLAM receptors, SLAMF7 is a self-ligand. i.e., it recognizes as ligand another SLAMF7 molecule on another cell. SLAMF7 is found on natural killer (NK) cells, activated T cells, most B cells and myeloid cells. In NK cells, SLAMF7 is a positive regulator of NK cell activation. This activity requires expression of the SAP family adaptor Ewing's sarcoma-associated transcript 2 (EAT-2). SLAMF7 receptor as an important regulator of IFN-α-driven innate immune responses.

Resistance to multiple antibiotics is an increasingly urgent problem in health care all over the world. However, it appears that the pharmaceutical industry is not focused anymore on the development of new classes of antibiotics, as this costs a lot but is not very profitable, in contrast to new immunotherapies for cancer patients. Therefore, the risk that medical caregivers face infections with bacteria resistant to all known antibiotics is steadily increasing, with dramatic consequences for the prognosis of the patients. New therapeutic antibacterial avenues are therefore mandatory if science and medicine do not want to lose the battle against these pathogens. Some, such as *Klebsiella pneumoniae*, *Acinetobacter baumannii* and *Pseudomonas aeruginosa,* are considered as especially dangerous by the World Health Organisation.

In the cancer field, various immunotherapies have emerged in the last decade and are considered as the new biggest hope in the fight against tumors. Among them, the "Trispecific Killer Engagers" (TriKEs), as being referred to herein, first developed by Jeffrey Miller from the University of Minnesota, USA, are a promising approach. They are a combination of single chain variable fragments (scFv) derived from three immunoglobulins (antibodies) directed against an activating receptor on NK cells (frequently CD16) and two antigens on cancer cells, or alternatively human interleukin-15 (IL-15) replacing one of the tumor antigens and thus allowing a better survival and activation of the NK cells.

Present inventors have used the TriKes principle (Felices M et al. Methods Mol Biol, 2016, 1441:333-346) to create dimeric protein complexes for use in the treatment of bacterial infections, such as *P. aeruginosa* infections. Such dimeric protein complexes comprise two scFv fragments directed against the NK cell activating receptors NKG2D (CD314) and SLAMF7 (or CD319). This is based on the knowledge that generally NK cells are optimally stimulated when two activating receptors are engaged instead of only one. The dimeric protein complexes further comprise a third functional component, being a scFv derived from anti-bacterial antibodies, for example directed at a surface structure of *P. aeruginosa,* such as dimeric PcrV or Psl scFv anti-P. *aeruginosa* (scFv anti-PcrV or scFv anti-Psl, such as described in International patent application WO2017095744-A1, which is incorporated herein by reference). Present inventors hypothesized that crosslinking of the NK cells with the bacteria, via the TriKE-based dimeric protein complexes (also referred to herein as "TriKEs")s, will lead to a degranulation of the NK cells. It is known that human cytotoxic lymphocytes contain cytolytic granules filled with apoptosis inducers (Perforin, Granzymes) and the directly bactericidal molecule Granulysin. After degranulation, the bacteria will be killed, should they be resistant to antibiotics or not. Thus, present inventor's approach is new and original, as the TriKE dimeric protein complexes are in fact not acting as antibiotics by interfering with the bacterial metabolism, but as direct inducers of cell death. Autologous NK cells in the infected tissues and organs would be activated *in situ* and degranulate there.

### 2.2. Genes used and cassette design (Figure 7)

- Construct A': MS scFv [(RTX V_{L} 10-first amino-acids / MS (V_{L}-V_{H})] anti-NKG2D.**C4bpβ**.scFv (V_{L}-V_{H}) anti-Psl.**His8x**

The MS anti-NKG2D scFv (as disclosed in European patent application EP2769993-A1, which is incorporated herein by reference) is an agonist of the NKG2D/DAP10/Grb2/Avl/PI3K/Akt pathway, whereas the ELO scFv anti-SLAMF7 (derived from Elotuzumab) is an agonist of the SLAMF7/ITSM/PLC/Ca⁺⁺/erk pathway, both pathways activate NK cells.

Psl scFv anti-P. *aeruginosa* is scFv anti-Psl as described in International patent application WO2017095744-A1.
- Construct B': ELO scFv [(RTX V_{L} 10-first amino-acids / ELO (V_{L}-V_{H})] anti-SLAMF7.**C4bpβ**.scFv (V_{L}-V_{H}) anti-Psl.**FLAG**

The anti-NKG2D and anti-SLAMF7 scFv are located upstream of the C-terminal part of the C4bpβ-chain, whereas the scFv anti-Psl are located downstream (C-terminally) of the C-terminal part of the C4bpβ-chain. The cassettes were cloned in the pEF-IRESpac expression vector.

Three clones were selected for further experiments for: A'(BiKE), B' (BiKE) and A'B' (TriKE) (see Figure 8A for schematic representation hereof), wherein BiKEs are dimeric protein complexes as taught herein wherein the first and second polypeptides are identical and TriKEs are dimeric protein complexes as taught herein wherein the first and second polypeptides are different from each other. The purified BiKEs A' and TriKEs A'B' were verified using SDS-PAGE and SYPRO analysis under non-reducing conditions (data not shown). A stepwise purification with sequential increase of imidazole concentration allowed isolating the dimeric form, while getting rid of the molecular species with lower (monomers) or higher (tetramers) valences.

### 3.2. Binding of the BiKEs and TriKEs displaying the scFv anti-PslI to Pseudomonas aeruginosa using ELISA with coated bacteria

Bacteria were grown overnight in TBS medium to an optical density of 1.0 measured at 600 nm. 1 O.D. unit represents 2 ×10⁸ CFU/ml. Bacteria were centrifuged for 10 minutes 2500 rpm. The pellet was resuspended in 10 ml PBS. After centrifugation, bacteria were resuspended in 4 ml PBS with 1% (v/v) formaldehyde and left for 2h at room temperature. Bacteria were diluted 10x in PBS (10×: 2 × 10⁷ CFU/ml). Bacteria were immobilized (200 µl/well= 4 × 10⁶ CFU/well) on a NUNC MaxiSorp^{™} 96-well flat-bottom polystyrene ELISA plate overnight at 4°C. After washing with PBS-1% (w/v) BSA the plates were blocked with 100 µl PBS-5% (w/v) BSA for 1 hour at 4°C. The wells were incubated with 100 µl BiKE- and TriKE-containing crude cell culture supernatants for 1h at 4°C. After 5x washings with PBS-1%BSA, the wells were incubated with either a goat anti-FLAG/HRP-conjugated pAb (Bethyl) or a mouse anti-HIS HRP-conjugated mAb (SIGMA) for 1h at 4°C. After 5x washings, the ELISA plate was revealed with o-phenylenediamine dihydrochloride (ODP)/H₂O₂ or tetramethylbenzidine (TMB)/H₂O₂ chromogenic substrate and the reaction was stopped with 0.5N H₂SO₄ when the colour is suitable for a readout at 450/492 nm using the spectrophotometer.

The data of binding of the BiKEs and TriKEs HeteroTetraBolos to *Pseudomonas aeruginosa* show that the scFv anti-PslI bound to 1 out of 4 strains coming from patients with mucoviscidosis, and 5 out of 5 strains coming from secretions from patients with endotracheal tubes. The scFv anti-PslI also bound to the RP73 strain. This example shows that a scFv anti-Pseudomonas that is located C-terminally of the C-terminal fragment of the C4bpβ-chain is functional, able to bind its target (Figure 8B).

### 3.3. ELISA and FACS analysis of the binding and cross-linking of the BiKEs, TriKEs to Pseudomonas/ NK92MI, and killing of bacteria

NK92MI and *P. aeruginosa* (strain RP73) were at first stained with violet cell tracer and PKH26, respectively. BiKEs anti-NKG2D/anti-Psl (A'), anti-SLAMF7/anti-Psl (B') and TriKEs anti-NKG2D/anti-SLAMF7/anti-Psl (A'B') were then incubated with NK92MI alone, Pseudomonas aeruginosa alone, or with both NK92MI *& P.aeruginosa* for 1h at 4°C. As negative control, cells/bacteria were incubated with either DMEM supernatant from HEK293 cells or with PBS/10%FBS.

Cells were analyzed using flow cytometry and the percentage of colocalisation NK92MI/*P*.*aerugonisa* was measured for all conditions (results FACS analysis see **Table 2 and** **Figure 8C**).

**Table 2**

| **% colocalization (violet⁺PHK26⁺)** | **BiKEs A'** | **BiKEs B'** | **TriKEs A'B'** | **Supernatant HEK** | **PBS/10%FBS** |
|---|---|---|---|---|---|
| **NK92 MI (violet⁺)** | 0.14 | 0.24 | 0.24 | 0.06 | 0.23 |
| ***P. Aeruginosa* (PKH26⁺)** | 0.07 | 0.05 | 0.07 | 0.1 | 0.06 |
| **NK + *P. aeruginosa*** | 0.43 | 1.85 | 0.9 | 0.29 | 0.31 |

BiKE anti-SLAMF7/anti-Psl (B') and TriKEs anti-NKG2D/anti-SLAMF7/anti-Psl (A'B') resulted in cells stained with the violet cell tracer and PKH26, demonstrating that the 2 scFv in the TetraBolos are functional, allowing binding and cross-linking NK cells and target *P*. *aeruginosa.*

The expression of the different scFvs on BiKEs and TriKEs were checked using anti-HIS or/and anti-Flag antibodies by ELISA. The BiKE NKG2D (A') was purified on an HIS-TRAP column, the BiKE SLAMF7 (B') was purified using a FLAG-affinity chromatography while His and FLAG tags in the structure of the TriKE (A'B') allow a 2-step purification method to isolate clones bearing both scFvs anti-NKG2D and anti-SLAMF-7. As shown in Figure 8D, each BiKE and TriKE express scFVs for their respective targeted NKG2D and/or SLAMF-7. In Figure 8E, present inventors used the luminescent Pseudomonas PAO1-lux strain to assess the killing of the bacteria induced by NK cells after binding with BiKEs and TriKEs. 2.10⁵ NK92-CD16 cells were plated together with Pseudomonas PAO1-lux strain at a final E/T ratio 1/3 in 200 µl complete RPMI medium without antibiotics and cultured at 37°C. Molecules were added at T₀ and bacterial growth was measured in triplicates over time with a luminescence microplate reader during 9 hours. The TriKE anti-NKG2D/anti-SLAMF7/anti-PSl (A'B') decreased by two-fold the bacterial growth of PAO1-lux at the concentration of 3 µg and completely prevented its growth after 9 hours of incubation with NK cells when provided at the concentration of 6 µg. The BiKE anti-NKG2A/anti-Psl (A') and the BiKE anti-SLAMF7/anti-Psl (B') did not have a significant effect on bacterial growth as compared to the NK cells incubated with PAO1-lux without any molecules.

### 3. Example 3: "Two-hinged long-neck pseudo-Immunoglobulin G (pseudo-IgG)" with enhanced Fc-biological functions using the C4bp C-terminal β-chain dimerization scaffold and the Fc (hinge+CH2+CH3) from IgG downstream of the dimerization scaffold

### 3.1. Design and features of the pseudo-IgGs

Present inventors have generated two-hinged long-neck pseudo-immunoglobulin G1 (LNPIgG1) as "pseudo-IgGs" (Figure 9). Like the known scFv-Fc antibody-like molecules, the pseudo-IgGs are generated as a single-cassette cloned into an expression vector for eukaryotic cells.

The pEF-IRESpac bicistronic expression vector is a vector suitable for the stable expression of recombinant therapeutic glycoproteins in mammalian cells. The pEF-IRES*pac* contains an "Internal Ribosome Entry Site" (IRES) downstream of the multiple cloning site (MCS), followed by the Puromycin resistance gene (*pac*). The protein expression is driven by the strong human polypeptide chain elongation factor 1α promoter for high protein expression. The signal peptide, from the tumor necrosis factor receptor superfamily member 16 (TNFR16, UniProt no. P08138.1) was cloned between the restriction sites ECoRI and Bgl2. The cDNA encoding the cassette for pseudo-IgG (scFv or V_{H}H.Linker.C4bp(beta).Fc) is spanned between the Bgl2 and NotI restriction sites were codon-optimized for human cells, synthesized and cloned into pEF-IRESp by Proteogenix (Illkirsch, Strasbourg). The C4bp C-terminal β-chain (UniProt no. P20851.1, aa: 137-252) comprises (3x SGGGGS (SEQ ID NO: 3)) linkers on its upstream ends - beginning with the BspE1 restriction site - and is followed by the hinge, CH2 and CH3 of the Fc from human IgG1 and ends with a stop codon and the NotI restriction site (Figure 10).

Pseudo-IgGs comprising the (V_{L}-V_{H}) scFv derived from MabThera or rituximab (RTX) typically have a molecular weight of about 140 kDa under non-reducing conditions.

The pseudo-IgG is a dimeric molecule displaying an N-terminal recognition domain upstream of the C-terminal part of the C4bp beta-chain dimerization scaffold, with a 3x(SGGGGS) (SEQ ID NO: 3) linker in-between. The targeting moiety can be an antibody fragment (scFv, nanobody), but also a soluble recombinant receptor, or a ligand. Downstream of the dimerization scaffold is located the Fc from IgG1, consisting of (i) the hinge, (ii) CH2 and (iii) CH3. There is no linker between the dimerization scaffold and the Fc. The C4bpβ is responsible for the covalent dimeric association of the 2 chains, whereas the hinge of the Fc allows a dimeric Fc association leading to a fully functional Fc-dimer.

The transfection into eukaryotic cells such as HEK293T cells of a single mono-cistronic construct comprising either a third or fourth functional component N-terminally of the C-terminal fragment of the C4bp beta-chain and monomeric Fc of IgG1 C-terminally of the C-terminal fragment of the C4bp beta-chain leads to the secretion into the cell culture supernatants of a single molecular species monospecific pseudo-IgG (**Figure 10A**).

The co-transfection of 2 constructs, wherein one construct comprises a third functional component N-terminally of the C-terminal fragment of the C4bp beta-chain and a monomeric Fc of IgG1 C-terminally of the C-terminal fragment of the C4bp beta-chain and a second construct comprises a fourth functional component N-terminally of the C-terminal fragment of the C4bp beta-chain and a monomeric Fc of IgG1 C-terminally of the C-terminal fragment of the C4bp beta-chain, wherein the third and fourth functional components are different leads to the generation of a bispecific 3, 4 pseudo-IgG, along with the 2 monospecific 3 and 4 counterparts (**Figure 10A**).

The combination of the known "knob-into-hole" technology as described in International patent application WO2013097430, which is incorporated herein by reference, or as described by Ridgway J B and P Carter L G P, 'Knobs-into-holes' engineering of antibody CH3 domains for heavy chain heterodimerization, Protein Eng., 1996, Jul 9 (7): 617-21, with the pseudo-IgG technology as taught herein allows the expression of 100% bispecific pseudo-IgGs (**Figure 10B**).

The particularity of the pseudo-IgG lies in a double-hinged "long-neck" Fc, conferring particularly enhanced biological properties to activate the complement system on the target surface when compared to conventional antibodies. The ability to activate FcyRs is also augmented, leading to enhanced NK activation as well as phagocytic activity of macrophages for targets. The accessibility for C1q is increased in the two-hinged long-neck pseudo-IgGs, and according to the *in vitro* data of present inventors, this configuration may also favor - over conventional IgG1 - the concomitant engagement of the Fc for C1q binding and subsequent complement activation and complement-mediated cytotoxicity (CDC) as well as complement-dependent cell-mediated cytotoxicity/ phagocytosis (CDCC/CDCP) and the Fc engagement to FcyRs of immune effector cells (ADCC/ADCP).

### 3.2. Cloning & screening of the pseudo-IgG-containing single clone crude supernatants using ELISA or flow cytometry analysis

### 3.2.1. Screening of the monospecific & "knob-into-hole" bispecific pseudo-IgG-containing supernatants using symmetrical Fc/Fc ELISA

HEK293T cells were used to express the different molecules. HEK293T cells were plated at densities 1.3×10⁶ cells/well into a 6-well cell culture plate and complete DMEM medium the day prior to transfection. The next day, the complete DMEM medium was replaced by optiMEM medium. Cells were transfected with the expression vectors using the lipofectamine 3000 kit according to the instruction datasheet (ThermoFisher, catalog number L3000001). Forty eight hours after transfection, cells were transferred into a 10-cm cellule culture dish and a DMEM medium supplemented with Pen/Strep antibiotics (P/S), Glutamine (Gln), 10% (v/v) fetal bovine serum (FBS) and 5 to 20 µg/ml puromycin (PURO20) selection antibiotic (depending on the construct). After 2 weeks, growing clones that are resistant to puromycin were individually manually picked up and transferred into 96-well plates. About a week later, supernatants from single isolated clones were screened for TetraBolos/pseudo-IgGs expression levels using a symmetrical anti-HIS or anti-human IgG1 ELISA.

The screening of the individual clone supernatants was established using a home-made Fc ELISA. A goat anti-human IgG pAb (ABCAM ab97221) was coated onto a NUNC MaxiSorp 96-well ELISA plate at 1 µg/ml in PBS (100 ng/well) for 48h at 4°C. After blocking with PBS-5%BSA for 1h at 4°C, the plate was incubated with 200 µl crude supernatants from individual cell clones for 1h at 4°C. The dilutions of the supernatants have to be adjusted to avoid saturating the signal. Generally, a dilution of 1/200 is required. The plate was then revealed with a goat anti-human Fc pAb HRP-conjugated (ABCAM ab997225) dilution 1/1000. The ELISA was then revealed with OPD/H₂O₂ chromogenic substrate for HRP and read at 492 nm and 620 nm. The best clones (i.e. expressing the highest amounts of pseudo-IgGs) were selected and then expanded for scaling up productions.

### 3.2.2. Double screening of the bispecific pseudo-IgG-containing supernatants using FACS analysis and 2 target cells

This is an example of screening of RTX scFv / anti-HER2 V_{H}H bi-specific pseudo-IgG-containing supernatants from individually picked-up cell clones using target cells and flow cytometry analysis. Supernatants were tested in parallel on HER2-positive BT474 and CD20-positive DAUDI cells. BT474 and DAUDI cells (1,5.10⁵ cells/well) were incubated with 150 µl crude supernatants from transfected individual clones for 30 min at 4°C. After washing (PBS/1%FBS), cells were incubated with a goat anti-human IgG AF647-conjugated pAb. After fixation with 1% paraformaldehyde in PBS, cells were analysed using flow cytometry. Cells of which the supernatants showed the strongest staining on both cell type (i.e. clones D1, D12 and E1) (data not shown) were expanded for scaling-up production.

### 3.3. Large scale production, protein-G purification and characterization of the pseudo-IgGs 3.3.1.Large scale production

The clone with the highest pseudo-IgG expression as result of the screening was selected and transferred from the 96-well cell culture plate to a 25 cm² (T25) flask. After reaching confluence the cells were first transferred into a 75 cm² (T75) flask and then to a 175 cm² (T175) flask. The cells from 7 confluent T175 flasks were transferred into a 5-chamber format polystyrene CellSTACK^{®} in complete medium (DMEM with 10% (v/v) FBS, Penicillin/Streptomycin and L-Glutamine). After 24 hours the complete DMEM medium was replaced by OptiMEM completed with Penicillin/Streptomycin and L-Glutamine. After 48 hours the OptiMEM medium was collected and the process was repeated (24 hours of complete DMEM followed by 48 hours of OptiMEM). The collected OptiMEM medium (1 liter) was centrifuged in 50 ml falcon tubes (20 minutes, 4000 rpm) and filtered using 0.22µm PVDF 1L Millipore vacuum filter units.

### 3.3.2.G-protein-based purification of the pseudo-IgGs

The filtered OptiMEM medium was incubated with 1 ml of Protein G Sepharose^{®} 4 Fast Flow (GE healthcare, GE17-0618-01) for 48 hours at 4°C with agitation. The Protein G beads were collected by centrifugation and transferred to an empty 1 ml disposable column (Qiagen) connected to a peristatic pump. After washing the beads with 50 ml PBS, the pseudo-IgG were eluted using 20 ml elution buffer (phosphate citrate buffer, pH 2.7 supplemented with 10% (v/v) Glycerol). Collected fractions are immediately neutralized using a neutralization buffer (Bi-carbonate buffer pH 9 supplemented with 10% (v/v) glycerol) at ratio 4/5 elution buffer + 1/5 neutralization buffer to reach a final pH7.2. The elution solution was then concentrated using Amicon^{©} 30 kDa MWCO centrifugal filter devices. The concentration of purified molecules was measured using a NanoDrop^{™} micro-volume spectrophotometer, aliquoted and were frozen.

### 3.3.3. Molecular pattern analysis by SDS page electrophoresis followed by SYPRO Ruby staining

One µg of purified pseudo IgG was mixed with 4 µl-s of 4X Laemmli sample buffer without (non-reducing conditions) or with 10% (v/v) β2-mercaptoethanol (reducing conditions). The samples were loaded and electrophoresed using 4-15% Mini-Protean^{®} Tris-Glycin eXtended (TGX^{™}) precast protein gels (Bio-Rad) and XT MES running buffer (Bio-Rad). The gel was then fixed with 2x 100 ml 50% (v/v) methanol + 7% (v/v) acetic acid for 30 minutes. After fixing the gel was incubated with 30 ml of SYPRO Ruby gel stain overnight at 4°C. The next day the gel was washed for 30 minutes with 100 ml 10% (v/v) methanol + 7% (v/v) acetic acid for 30 minutes. Finally, the gel was analyzed using an Amersham^{™} Typhoon^{™} biomolecular imager with the Cy5 filter.

Molecular pattern analysis of the RTX anti-CD20 pseudo-IgG, the 47D5 V_{H}H anti-HER2 pseudo-IgG, and the bispecific CD20/HER2 pseudo-IgG demonstrated that in the knob-into-hole generation, there is 100% of bispecific pseudo-IgGs. Furthermore, a size difference was observed between the 2D3 and 47D5 anti-HER V_{H}H, the apparent MW of 47D5 V_{H}H being smaller than that of the 2D3 V_{H}H.

### 3.3.4. FACS analysis of the binding of RTX scFv.C4bp(beta).Fc pseudo-IgG versus RTX scFv.C4bp(alpha).Fc and MabThera (rituximab) to Daudi cells

The binding of purified RTX scFv.C4bp(alpha).Fc (Multi-Di-Fc), RTX scFv.C4bp(beta).Fc (pseudo-IgG or mono-Di-Fc), MabThera (RTX) or no molecule (negative control) to Daudi cells was analyzed using flow cytometry. Daudi cells (1.5 × 10⁵/well) were incubated with saturating concentration of molecules (18 µg/ml) for 30 min at 4°C. After washing, cells were incubated with either a goat anti-human IgG/AF647 or Protein A/AF647 for 30 min. at 4°C.

It was demonstrated that the RTX scFv is functional in both scaffolds [C4bp(alpha).Fc and C4bp(beta).Fc], with similar binding patterns as observed with both revelation systems (anti-human IgG or protein A) (data not shown).

### 3.4. Comparison of fluid-phase complement activation of RTX scFv.C4bp(alpha).Fc, RTX scFv.C4bp(beta).Fc pseudo-IgG versus MabThera (RTX) using CHso assay

Present inventors compared the molecule-mediated fluid-phase complement activation of the RTX scFv.C4bp(beta).Fc pseudo-IgG to MabThera. For that purpose, present inventors used the classic CH₅₀ assay. The CH₅₀ assay tests the functional capability of complement components of the classical pathway from a normal human serum (NHS) to lyse sheep red blood cells (SRBC) that were previously sensitized with rabbit anti-SRBC antibody (haemolysin). Hemolysis is mediated by the formation of the membrane attack complexes (MAC).

In order to calibrate the CH₅₀ assay, present inventors first established a serial dilution of normal human serum (NHS): Eighty µl of serum were at first added to 20 µl PBS, corresponding to the working serum solution. A serial dilution of the working serum solution was incubated (30 min. at 37°C) with sensitized SRBC. SRBC were then centrifuged, the hemoglobin release in the supernatants was measured using a spectrophotometer at 418 nm. A hemolysis calibration curve was thus established. The serum dilution that elicits about 75% hemolysis, corresponding to the near top of the linear hemolysis curve before reaching a plateau was used final serum dilution for the assay.

Serial dilutions of the 3 molecules were performed in PBS. Twenty µl of the molecule serial dilutions were mixed to 80 µl NHS and incubated for 1h at 37°C. Then, hemolysis was tested on sensitized SRBC (30 min. at 37°C) using the serum dilution established during the calibration. If the molecules consumes the fluid-phase complement in the NHS, the ability of NHS to hemolysis will drop.

**Figure 11** depicts the data of CH₅₀ assay. CH₅₀, representing the molecule concentration that inhibits 50% hemolysis, was:
- 41 mg/l for RTX scFv.C4bp(beta).Fc
- 4 mg/l for MabThera (RX).

These data indicates the RTX pseudo-IgG (or "Pseudo-RTX") - displaying a single dimeric-Fc - presents similar features to MabThera in term of fluid-phase complement activation, with CH₅₀ concentrations around 4 mg/l. In contrast, the CH₅₀ concentration for the 2 multi-Di-Fc is 4 times lower. The hemolysis curve starts dropping at concentration 28 and 12 mg/l for RTX pseudo-IgG and MabThera, respectively. The results of this assay show that up to 45 mg/l, fluid-phase complement consumption by pseudo-IgGs is lower than that by conventional antibodies (RTX). Conventional antibodies and pseudo-IgGs start consuming fluid-phase complement at concentrations 12 mg/l and 28 mg/l, respectively. In conclusion, pseudo-IgGs offer better reliability over Multi-Di-Fc counterparts, whose fluid-phase complement activation features are similar to that of conventional therapeutic antibodies. Most antibodies, in order to show a therapeutic effect, have to be injected at high doses to reach a serum concentration between 10 and 100 mg/l. From our assay, pseudo-IgGs could be used with greater safety than conventional IgGs up to 45 mg/l.

### 3.5. Flow cytometry analysis of the bi-specific anti-CD20/anti-HER2 pseudo-IgG-mediated crosslinking of Daudi lymphoma with BT474 breast tumour cells

Present inventors tested the ability of the bispecific 47D5 anti-HER2 V_{H}H.C4bp(beta).Fc / RTX anti-CD20 scFv.C4bp(beta).Fc pseudo-IgG (i) to bind CD20-positive Daudi lymphoma cells AND HER2-positive BT474 breast tumor cells and (ii) to crosslink the 2 cell-types. As control, the 47D5 anti-HER2 and RTX anti-CD20 monospecific counterpart pseudo-IgGs were used.

Daudi cells were stained with CFSE and BT474 cells with KPH26 cell tracers. Daudi and BT474 cells were mixed at ratio Daudi:BT474 = 2:1. Cells were incubated with (i) purified bispecific anti-HER2/anti-CD20 pseudo-IgG, (ii) monospecific 47D5 V_{H}H anti-HER2 pseudo-IgG, (iii) monospecific RTX scFv anti-CD20 pseudo-IgG at saturating concentrations, or no molecule as control. After washing, cells were incubated with a goat anti-human IgG/AF647 pAb. After washing, cells were then analyzed using flow cytometry. It was demonstrated that the bispecific pseudo-IgG binds the 2 cell types and lead to their crosslinking (**Table 3**). In contrast, 47D5 pseudo-IgG was shown to only bind to BT474 cells, whereas RTX pseudo-IgG was shown to only bind to Daudi cells, both were unable to crosslink the 2 cell types. This experiment validates the functionality of both scFv anti-CD20 and V_{H}H anti-HER2 in the bispecific pseud-IgGs.

**Table 3**

| | **Bispecific 47D5 / RTX** | **47D5 V_{H}H anti-HER2** | **RTX scFv anti-CD20** | **Control (no molecules)** |
|---|---|---|---|---|
| % double positive CFSE⁺ PKH26⁺ | 10.1 | 0.9 | 0.86 | 0.83 |

In a second experiment, the bispecific [knob-into-hole] RTX anti-CD20 scFv.C4bp(beta).Fc[knob] / 47D5 anti-HER2 V_{H}H.C4bp(beta).Fc[hole] pseudo-IgG was compared to RTX, Trastuzumab or the 2 combined mAbs for Daudi and BT474 binding and crosslinking. Present inventors clearly demonstrated that neither RTX, Trastuzumab, nor the combined mAbs were able to crosslink both cell types, even when put together. In contrast, the bispecific [knob-into-hole] pseudo-IgG is able to (i) bind each cell type, and (ii) to crosslink the 2 cell types. The double positive population Violet/PKH26 also displays a strong anti-Fc signal (**Table 4**). Present inventors thus validated that the 2 associated recognition moieties in the bispecific pseudo-IgG are functional in the [knob-into-hole] bispecific construct.

**Table 4**

| | **RTX scFv / 47D5 V_{H}H** | **RTX (MabThera)** | **Trastuzumab (Herceptin)** | **RTX + Trastuzumab** | **Control** |
|---|---|---|---|---|---|
| % double positive ^{+ +} Violet /PKH26 | 19 | 2 | 2.1 | 2.2 | 2.2 |

All together, these two experiments validate the bispecific pseudo-IgG associated or not to the knob-into-hole technology.

### 3.6. Analysis using flow cytometry of the CDC and C3b depositions on Daudi cells following incubation with serial dilutions of pseudo-RTX (RTX scFv.C4bpβ.Fc) or MabThera reference mAb in the presence of 25% normal human serum

In this experiment, Daudi cells were incubated with 2-fold serial dilutions of molecules (starting concentration was 20 µg/ml) of (i) scFv.C4bp(beta).Fc (RTX pseudo-IgG), MabThera or no molecule. Then, cells were incubated with 25% normal human serum (NHS) in GVB⁺⁺ for 30 min. at 37°C. After washing, cells were then stained with a mouse anti-human C3b mAb (clone 7C12 from Cedarlane) for 30 min. at 4°C, and then with a goat anti-Mouse IgG AF647-conjugated secondary pAb, together with a live/dead. Cells were fixed with 1% (v/v) paraformaldehyde in PBS, then were analyzed using flow cytometry. **Figure 12A** shows that the RTX pseudo-IgG elicits higher cytotoxicity compared to the RTX MabThera for all concentrations used: At 5 µg/ml molecules, the % dead cells was 1.7-fold higher for pseudo-RTX (38%) than for RTX (22%). Only at the highest concentration used (20 µg/ml) RTX shows similar efficacy as pseudo-RTX. In **figure 12B**, pseudo-RTX-mediated C3b depositions are higher of factors 3.5x, 2.2x, 2.14x and 1.55x at concentrations 2,5, 5, 10 and 20 µg/ml when compared to RTX, respectively. RTX is a therapeutic antibody whose CDC represents a substantial part of its biological activity. Expressing the scFv from RTX in the pseudo-IgG scaffold enhances the Fc function of RTX.

### 3.7. Design of pseudo-RTX & comparison of efficacy of C3b depositions relative to Fc densities on DAUDI cells between RTX & pseudo-RTX

**Figure 13A** depicts the structural difference between the RTX pseudo-IgG (or pseudo-RTX or RTX scFv.C4bpβ.Fc) compared to RTX:
- RTX scFv.C4bp(beta).Fc or Pseudo-IgG or "Pseudo-RTX" is on the left
- RTX or MabThera is on the right

**Figure 13B** represents C3b depositions relative to the Fc densities on Daudi at the different concentrations used. The RTX pseudo-IgG rapidely stands out from RTX and displayed a higher C3b activation efficacy at same Fc densities. This is in line with the comparative dose-response analysis between pseudo-RTX and RTX for cytotoxicity and C3b depositions reported in **figure 12****.** **Figure 13B** shows that for the same MFI anti-Fc (12,200), C3b deposition was 5.6-fold superior for the pseudo-RTX when compared to RTX. This experiment validates the C4bp(beta).Fc scaffold for design and expression of "pseudo-IgGs", whose Fc displays enhanced activity for complement activation and CDC on target surfaces.

### 3.8. FACS analysis of pseudo-IgG-mediated complement activation on BT474 and NK activation (% CD107-positive NKs)

Present inventors wanted to explore the biological function of 2 bispecific "knob-into-hole" pseudo-IgGs:
- Z199 scFv anti-NKG2A.C4bp(beta).Fc[knob] / 2D3 V_{H}H anti-HER2.C4bp(beta).Fc[hole]; abbreviation: Z199/2D3
- Z199 scFv anti-NKG2A.C4bp(beta).Fc[knob] / Trastu scFv anti-HER2.C4bp(beta).Fc[hole]; abbreviation: Z199/T

For (i) NK activation from PBMCs, and (ii) complement activation on BT474 cells upon incubation with 20% C5-deficient human serum as source of complement. The experimental protocol is briefly detailed in **Figure 14A**.

BT474 were stained with CFSE, then incubated with 5-fold serial dilutions (20, 4, 0,8 µg/ml) of bispecific pseudo-IgG (Z199/2D3 or Z199/Trastu), or Trastuzumab, or no molecule. BT474 cells were incubated either with 20% C5-deficient human serum in GVB⁺⁺ (30 min at 37°C/5% CO₂) or with complete RPMI medium prior to co-culture with PBMCs for 5h at 37°C/5% CO₂. Cells were stained with an anti-humCD107/BV421 (after 1h co-incubation), and then with anti-human IgG/AF647 pAb, as well as with anti-C3b/PE, anti-CD3/BUV496, CD14/PE-Cy5, CD16/BUV737, CD19/PE-Cy5, CD56/BV786 mAbs. The gating strategy settings were CD3⁻/CD14⁻CD19⁻/CD16⁺/CD56⁺ in order to access NK staining for Fc, knowing that BT474 cells are CSFE-positive.

**Figure 14B** shows that Z199/2D3 bispecific pseudo-IgG activated NK cells slightly better than Trastuzumab in the presence of complement, and its activity was similar to Trastuzumab in the absence of complement. Z199/T activity was slightly weaker than Trastuzumab, with or without complement, but the presence of complement on BT474 cells slightly enhanced Z199/T-mediated NK activation.

Bi-specific pseudo-IgGs but not trastuzumab clearly strongly recruit complement towards target cells. Z199/T displays enhanced NK activation in the presence of complement. NK activation takes places through 2 mechanisms, (i) the interaction FcyRIIIA (CD16)/Fc and (ii) the interaction complement breakdown products (such as iC3b, C3dg, C3d) with CD11b (CR3) and CD11c (CR4). Trastuzumab has no complement activation activity. **Figure 14C** shows a C3b deposition only with the bispecific pseudo-IgGs but not Trastuzumab. Z199/T activated complement better than Z199/2D3. For both pseudo-IgGs coated to BT474, the additional presence of complement tended to enhance their ability to activate NK cells, whereas it seemed to have a negative effect on Trastuzumab. These data show that introducing a scFv derived from Trastuzumab in the C4bp(beta).Fc pseudo-IgG scaffold leads to the generation of a pseudo-IgG with dramatically enhanced complement activation biological activity compared to the original Trastuzumab.

Te Z 199/2D3 bispecific pseudo-IgG were shown to cross-link NK92MI and BT474, as for the bispecific CD20/HER2 capable of cross-linking Daudi and BT474 cells (data not shown). We validate here the bispecific pseudo-IgG in a NK / Tumour cell model.

### 3.9. Comparative analysis using an Andor spinning-disc confocal microscopy of the pseudo-IgG vs RTX-mediated phagocytosis of Daudi cells by monocyte-derived matured macrophages

**Experimental setting.** Daudi were stained with CFSE and macrophages with KPH26.

Daudi cells were then incubated with saturating concentration (20 µg/ml) of:
- MabThera (RTX)
- RTX scFv.C4bp(beta).Fc (pseudo-RTX)
- No molecule

After washing, Daudi cells were then incubated with 15% C5-deficient human serum (ΔC5-HS) in GVB⁺⁺, and then co-cultured for 18h at 37°C/5%CO₂. Cells are then fixed with 1% (v/v) formaldehyde, stained with DAPI, and analysed using confocal microscopy (magnification 40x). The NIKON NIH-elements software was used for the analysis. Large pictures were assembled from 50 photographed fields in 3 colours [DAPI (blue), CFSE (green), and PKH26 (red)] and bright light. A minimum of 3 large pictures for each condition are analysed to establish the statistical analysis (mean and SD). After thresholding, a highly accurate automated counting of macrophages and Daudi was performed, and automated co-localisation was then performed using Pearson's coefficient.

**Figure 15** depicts the histograms summarizing the analysis. Percentage of phagocytic macrophages in the presence (left) or in the absence (right) of complement. The presence of ΔC5-HS allow complement deposits without subsequent CDC. Using Δ□5-HS, ADCP+CDCP are assessed, whereas using decomplemented ΔC5-HS, only ADCP is assessed. In the presence/absence of complement on Daudi, percentage of phagocytic macrophages were:
- MabThera (RTX): **24.1% / 8.6%; drop of factor 2.8**
- RTX scFv.C4bp(beta).Fc (pseudo-RTX): **52% / 34.1%; drop of factor 1.52**
- No molecule: **5% / 1.8%**

In the absence of complement, the loss of percentage of phagocytic macrophages was 33% for the pseudo-IgGs and 66% for RTX, when compared to the results phagocytosis obtained in the presence of complement. RTX-mediated cytotoxic activity lies more in complement-mediated cytotoxicity (CDC) when compared to pseudo-RTX. An explanation could be that for the pseudo-IgG, the binding of C1q to the Fc, leading to a further involvement of the CD11b/iC3b axis, affects less a concomitant engagement of the Fc with the FcyRIIIA receptor on macrophage surface. Pseudo-RTX is superior to RTX by factors 2.16 & 4 in the presence & in the absence of complement, respectively, to elicit phagocytosis of macrophages of lymphoma Daudi target. This experiment validates the pseudo-IgG scaffold, showing the producing a pseudo-IgG using a scFv from RTX dramatically enhances the biological activity of the pseudo-RTX when compared to the RTX gold standard.

The biological activity of RTX is more dependent on Complement (CDCP) than for the RTX pseudo-IgG. For pseudo-RTX and RTX, phagocytosis drops by factors 1.52 and 2.8 in the absence of complement, while overall activity of pseudo-RTX is higher than RTX in the absence of complement. This means that concomitant ADCP & CDCP is more efficient for the pseudo-RTX than for RTX. This could be explained by the fact that the binding of C1q to Fc (engaging complement classical pathway activation and subsequent CDCC/CDCP) affects less the ability of the Fc to subsequently bind to CD16 (and engage ADCC/ADCP) for the pseudo-RTX when compared to RTX.

The pseudo-IgG technology can thus improve the overall therapeutic activity of current therapeutic mAbs, and in particular concomitant ADCC/CDCC & ADCP/CDCP activities.

### 4. Example 4: Generation of anti-Pseudomonas aeruginosa pseudo-IgGs

### 4.1. Design of the cassettes / Expression vectors (Figure 16)

The recombinant cDNA construct for the expression of the pseudo IgGs was synthesized by ProteoGenix SAS, Schiltigheim:
- scFv KBPA-101 (anti-O11 scFv V_{L}-V_{H} orientation derived from Panobacumab, as described in International patent application WO2006/084758A1)_hu C4bp C-terminal β chain (UniProt nr. P20851.1, aa 137-252)_hu IgG H chain constant gamma (UniProt nr. P01857.1)
- scFv anti-Ps1 (derived from MEDI3902, as described in International patent application WO2017095744A1)_hu C4bp C-terminal β chain (UniProt nr. P20851.1, aa 137-252)_hu IgG H chain constant gamma (UniProt nr. P01857.1)
- (V_{L}-V_{H}) scFv Panobacumab.(3xSGGGGS).**C4bp(beta)**.hinge.CH2.CH3
- (V_{L}-V_{H}) scFv anti-Psl.(3xSGGGGS).**C4bp(beta)**.hinge.CH2.CH3
- (V_{L}-V_{H}) scFv anti-PcrV.(3xSGGGGS).**C4bp(beta)**.hinge.CH2.CH3
- (V_{L}-V_{H}) scFv Panobacumab.(3xSGGGGS).hinge.CH2.CH3
- (V_{L}-V_{H}) scFv Panobacumab.(3xSGGGGS).**C4bp(beta)**.hinge.CH2.CH3[knob]/ (V_{L}-V_{H}) scFv anti-Psl.(3xSGGGGS).**C4bp(beta)**.hinge .CH2.CH3 [hole]

The expression cassettes was cloned between the restriction enzymes BglII and BspEI of the multiple cloning site of a bi-cistronic pEF-IRESpac expression vector

### 4.2. Transfection & clone selection

The production of Panobacumab scFv (V_{L}-V_{H}).C4bpβ.Fc Pseudo IgG and anti-Psl scFv.C4bpβ.Fc Pseudo IgG molecules was performed in a similar way as described above in Example 3. Briefly, HEK293T cells were transfected with the recombinant plasmid DNA using the Lipofectamine 3000 kit. Two days following the transfection the cells were trypsinized and transferred into a 10 cm cell culture dish in complete medium (DMEM with 10% (v/v) FBS, Penicillin/Streptomycin and L-Glutamine) supplemented with selection antibiotic (40 µg/ml Puromycin). The successfully transfected cells were transferred into single chambers of a 96 well plate when the diameter of the clone reached 0.5-1 mm. The protein concentration in the supernatants from the isolated clones were analysed using symmetrical anti-Fc ELISA.

### 4.3. Measurement of protein concentration using symmetrical anti-Fc ELISA

A goat anti-human IgG Fc (Abcam, ab97221) was coated onto a NUNC MaxiSorp^{™} 96-well flat-bottom polystyrene plate at a concentration of 1 µg/ml diluted in PBS (100 ng/well in 100 µl PBS). After 48 hours of incubation at 4°C the plate was washed 5 times with 150 µl PBS-1% BSA and blocked for 1h with 100 µl PBS-5% BSA at 4°C. After washing steps 2 µl of cell culture supernatant + 198 µl of PBS was added (100x dilution). The plates were incubated for 1h at 4°C. The goat anti-human IgG Fc (HRP) (Abeam, ab97225) was added at the same concentration as the coating antibody (100 ng/well) and incubated for 1 h at 4°C. After the final washing step the plate was revealed with 50 µl TMB/H₂O₂ chromogenic substrate, and the staining reaction was stopped with 0.5N H₂SO₄. The plate was read at 450 nm using a spectrophotometer.

### 4.4. Large scale production

The clone with the highest absorbance (anti-Fc ELISA) was selected and transferred from the 96-well cell culture plate to a 25 cm² (T25) flask. After reaching confluency the cells were first transferred into a 75 cm² (T75) flask and then to a 175 cm² (T175) flask. The cells from 7 confluent T175 flasks were transferred into a 5-chamber format polystyrene CellSTACK^{®} in complete medium (DMEM with 10% FBS, Penicillin/Streptomycin and L-Glutamine). After 24 hours the complete DMEM medium was replaced by OptiMEM completed with Penicillin/Streptomycin and L-Glutamine. After 48 hours the OptiMEM medium was collected and the process was repeated (24 hours of complete DMEM followed by 48 hours of OptiMEM). The collected OptiMEM medium (1 liter) was centrifuged in 50 ml falcon tubes (20 minutes, 4000 rpm) and filtered using 0.22µm PVDF 1L vacuum filter units.

### 4.5. Protein purification with Protein G affinity chromatography

The filtered OptiMEM medium was incubated with 1 ml of Protein G Sepharose^{®} 4 Fast Flow (GE healthcare, GE17-0618-01) for 48 hours at 4°C with agitation. The Protein G beads were collected by centrifugation and transferred to an empty 1 ml disposable column (Qiagen) previously washed with 25% ethanol and PBS using a peristaltic pump. After washing the beads with 50 ml PBS elution buffer (phosphate citrate buffer, pH 2,7) was added. 1600 µl-s of eluate was collected in a 2 ml Eppendorf tube already containing 400 µl-s of neutralization buffer (Bi-carbonate buffer pH 9). 15 2 ml tubes were filled with the eluate and mixed in a 50 ml falcon. The elution solution was then concentrated using Amicon^{©} 30 kDa MWCO centrifugal filter devices. The concentration of purified molecules was measured using a NanoDrop^{™} micro-volume spectrophotometer.

### 4.6. Molecular pattern analysis by SDS page electrophoresis followed by SYPRO Ruby staining

One µg of purified Panobacumab scFv (V_{L}-V_{H}).C4bpβ.Fc Pseudo IgG molecules were mixed with 4 µl-s of 4X Laemmli sample buffer without (non-reducing conditions) or with 10% β2-mercaptoethanol (reducing conditions). The samples were loaded and electrophoresed using 4-15% Mini-Protean^{®} Tris-Glycin eXtended (TGX^{™}) precast protein gels (Bio-Rad) and XT MES running buffer (Bio-Rad). The gel was then fixed with 2x 100 ml 50% methanol + 7% acetic acid for 30 minutes. After fixing the gel was incubated with 30 ml of SYPRO Ruby gel stain overnight at 4°C. The next day the gel was washed for 30 minutes with 100 ml 10% methanol + 7% acetic acid for 30 minutes. Finally, the gel was analyzed using an Amersham^{™} Typhoon^{™} biomolecular imager with the Cy5 filter.

Three purification products of Panobacumab scFv (V_{L}-V_{H}).C4bpβ.Fc Pseudo IgG were analyzed and shown to have a MW of about 140 kDa for a non-reduced form and about 70 kDa for the reduced form (data not shown).

### 4.7. Dose-dependent binding of Panobacumab scFv (V_{L}-V_{H}).C4bpβ.Fc Pseudo IgG and anti-Psl scFv.C4bpβ.Fc Pseudo IgG to Pseudomonas aeruginosa - Whole-cell ELISA

Bacterial cells (reference strains PAO1 and O11 and clinical isolate IPP6247290) were grown overnight in 4 ml TBS medium to an optical density of 1.0 at 600 nm. The cells were transferred to a 15 ml falcon tube and centrifuged for 10 minutes 2500 rpm. The supernatant was discarded and the pellet was resuspended in 10 ml PBS. After centrifugation the bacteria were resuspended in 1% (v/v) PFA-PBS solution (fixation). 4 × 10⁶ CFU/well were then immobilized overnight onto a NUNC MaxiSorp^{™} 96-well flat-bottom polystyrene ELISA plate. After washing five times with PBS-1% BSA the plates were blocked with 100 µl PBS-5% BSA solution for 1 hour at 4°C. Different concentration of purified pseudo IgG molecules [Pano scFv.C4bp(beta).Fc or Psl scFv.C4bp(beta).Fc] were added (3 times serial dilution starting from 1000 ng/well) for 1 hour 4°C. After washing goat anti-human IgG Fc (HRP) was added at a concentration of 1 µg/ml (100 ng/well) and incubated for 1 h at 4°C. The plate was revealed with 100 µl OPD/H₂O₂ chromogenic substrate. The staining reaction was stopped with 0.5N H₂SO₄. The plate was read at 492 nm using a spectrophotometer. Data are means ± SD derived from three experiments.
- **Reference strain PAO1** is specifically recognized by the anti-Psl scFv (EC₅₀=6ng/well) but not by Pano scFv.C4bp(beta).Fc (**figure 17C****, left**).
- **ATCC33358 Serotype O11:** Pano scFv.C4bp(beta).Fc pseudo IgG specifically binds *P. aeruginosa* International Antigenic Typing System (IATS) serotype O11 recognizing the O-polysaccharide moieties of the lipopolysaccharide (LPS) (EC₅₀=110 ng/well), whereas the recognition by Psl scFv.C4bp(beta).Fc is far weaker (EC₅₀ was not reached) (**figure 17C****, middle**). Serotype O11 accounts for ~20% of *P. aeruginosa* infections.
- **Clinical isolate (IPP6247290)** was isolated from a patient with tracheostomy and is recognized by both pano scFv.C4bp(beta).Fc (EC₅₀=5 ng/well) and Psl scFv.C4bp(beta).Fc (EC₅₀=110 ng/well), pano scFv.C4bpβ.Fc having an affinity 22-fold superior to Psl scFv.C4bpβ.Fc (**Figure 17C****, right**). As a negative control present inventors used coated bacteria, but with no pseudo-IgG and the use of the secondary anti-human IgG pAb revelation system.

**Figure 17A** depicts the direct killing activity of Psl scFv.C4bpβ.Fc pseudo IgG mediated through complement activation against reference strain PAO1-*Luciferase* preventing bacterial growth, which led to a decay of luciferase signal between 4 and 5 hours. In the presence of Psl scFv.C4bpβ.Fc and decomplemented human serum, there is no effect on bacteria growth compared to the controls (25% NHS non-decomplemented or decomplemented). This experiment shows evidence of pseudo-IgG-mediated complement-dependent bacteria killing.

**Figure 17B** depicts the mechanism of action of the Pano & Psl pseudo-IgGs, recruiting C1q once bound to *Pseudomonas*, which engaging the complement classical pathway activation, leading to C3b depositions, formation of membrane attack complexes and finally bacteria killing through pore formation and membrane disruption.

### 4.8. Pano scFv.C4bp(beta).Fc & Psl scFv.C4bp(beta).Fc pseudo-IgGs mediate complement activation (C3b depositions) on Pseudomonas aeruginosa in a dose-response manner using ELISA with coated bacteria

Bacterial cells (PAO1, O11 reference strains or clinical isolate IPP6247290) were coated onto NUNC MaxiSorp^{™} 96-well flat-bottom polystyrene ELISA plates as described in section 4.7. After blocking, 3-fold serial dilutions of pseudo-IgGs [Pano scFv.C4bp(beta).Fc or Psl scFv.C4bp(beta).Fc] in PBS/1% BSA were added (starting concentration 100 ng/well) and incubated for 1h. As control, no pseudo-IgG was added to determine the background for C3b deposition when normal human serum (NHS) was applied afterwards. After washing, the plate was incubated for 30 minutes at 37°C with 0,5% non-decomplemented or decomplemented human serum (NHS versus ΔNHS, respectively), diluted in GVB⁺⁺ buffer to a final volume of 100 µl. After washing, the plate was incubated with a mouse anti-human C3/C3b/iC3b mAb (clone 7C12) (Cedarlane, CL7636AP) for 1 hour at 4°C. The plate was then revealed with 100 ng/well goat anti-mouse IgG HRP-conjugated pAb (Biolegend, 405306). Finally, the plate was revealed with 100 µl OPD/H₂O₂ chromogenic substrate. The staining reaction was stopped using 0.5N H₂SO₄. The O.D. was read at 492 nm and 605 nm using a spectrophotometer.

The data of pseudo-IgG-mediated C3b depositions on bacteria depicted in **figure 18** are consistent with the data of binding of the pseudo-IgGs observed in **figure 17C**:
- Psl pseudo-IgG, but not Pano pseudo-IgG, directs strong C3b deposition on PAO1 reference strain (EC₅₀=111 ng/well) **(****Figure 18****, left)**
- Pano pseudo-IgG directs strong C3b deposition on O11 ATCC33358 reference strain (EC₅₀=300 ng/well), and in a far lesser extent Psl pseudo-IgG (EC₅₀ was not reached). O.D. are 3 versus 0.7 for the first and second, respectively, at same pseudo-IgG concentrations (1000 ng/well).
- Pano pseudo-IgG & Psl pseudo-IgG direct strong C3b depositions on clinical isolate IPP627290 (EC₅₀=111 ng/well for both). The weaker binding of Pano pseudo-IgG over Psl pseudo-IgG does not affect the dose-response C3b deposition efficacy of the earliest.

The presence of 0,5% NHS does not lead to C3b deposition in the absence of pseudo-IgG.

### 4.9. Pano scFv.C4bp(beta).Fc & Psl scFv.C4bp(beta).Fc pseudo-IgGs mediate complement activation (C5b9 membrane attack complex formation) on Pseudomonas aeruginosa in a dose-response manner using ELISA with coated bacteria

Strain PAO1 **(****figure 19** **left),** reference strain O11 from ATCC **(****figure 19** **middle)** or clinical isolate IPP6247290 **(****figure 19** **right)** were coated onto NUNC MaxiSorp^{™} 96-well flat-bottom polystyrene ELISA plates as described in section 4.7. As a negative control non-coated wells were used. After blocking with 100 µl PBS-5% BSA the plates were incubated with 3-fold serial dilutions (starting from 1000 ng/well) of either Pano scFv.C4bp(beta).Fc or Psl scFv.C4bp(beta).Fc pseudo-IgGs for 1 hour at 4°C. After 5 time washing with 150 µl of PBS-1% BSA, the plates were then incubated for 30 minutes at 37°C with 2% normal human serum (NHS) or decomplemented normal human serum (ΔNHS) diluted in GVB⁺⁺ buffer to a final volume of 100 µl. After washing, the plates were incubated with a mouse anti-human C5b9 mAb (clone aE11) (Abeam, ab66768) for 1 hour at 4°C. The plate was incubated with a goat anti-mouse IgG HRP-conjugated pAb (Biolegend, 405306) 100 ng/well. Finally, the plate was revealed with 100 µl OPD/H₂O₂ chromogenic substrate. The staining reaction was stopped using 0.5N H₂SO₄. The O.D. was read at 492 nm. Data are means ± SD derived from three experiments.

The data of pseudo-IgG-mediated C5b9 MAC formation on bacteria depicted in **figure 19** are consistent with the data of binding of the pseudo-IgGs observed in **figure 17C** as well as with the data of pseudo-IgG-mediated C3b depositions observed in **figure 18****:**
- Psl pseudo-IgG, but not Pano pseudo-IgG, directs MAC formation on PAO1 reference strain **(****Figure 19****, left).** At highest pseudo-IgG concentration (1000 ng/well), O.D. measured were 2,5 and 1 for Psl pseudo-IgG and Pano pseudo-IgG, respectively. O.D. background was 0,5 in the absence of pseudo-IgG.
- Pano pseudo-IgG directs MAC formation on O11 ATCC33358 reference strain, and in a much lesser extent Psl pseudo-IgG as well At highest pseudo-IgG concentration (1000 ng/well), O.D. measured were 1,5 and 0,9 for Pano pseudo-IgG and Psl pseudo-IgG, respectively.
- Pano pseudo-IgG & Psl pseudo-IgG direct strong MAC formation on clinical isolate IPP627290 (EC₅₀=12 & 37 ng/well for Psl pseudo-IgG & Pano pseudo-IgG, respectively).

The presence of 2% NHS does not lead to MAC formation in the absence of pseudo-IgG.

Altogether, **figures 17-19** show that the 2 pseudo-IgGs *anti-Pseudomonas aeruginosa* are strong complement activators. Pano pseudo-IgG recognized 9% and 26% of cystic fibrosis and tracheostomy isolates, respectively, whereas Psl pseudo-IgG recognized 45% and 100% of cystic fibrosis (11 isolates) and tracheostomy (15 isolates) isolates, respectively (data not shown). Psl pseudo-IgG seems thus to have a broader *Pseudomonas* recognition spectrum compared to Pano pseudo-IgG.

Present inventors further explored whether the C3b depositions lead to the formation of terminal complement complexes (TCC), also called membrane attack complexes (MAC) that create major membrane damages and pore formation that is the origin of the Complement-dependent cytotoxicity (CDC) creating direct lysis, but also tag targets to facilitate recognition by immune effector cells, phagocytosis and cytotoxic cell activation.

### 4.10. Complement-dependent killing assay

Bacterial cells (SPAO1, reference strain O11, clinical isolate IPP6247290) were grown in Tryptic soy broth (TSB) medium at 37°C to an optical density at 600 nm of 1.0. Cells were washed and diluted in PBS to reach a concentration of 10⁶ cells/ml. 5 × 10³ cells were added per well (5 µl of bacterial suspension + 45 µl of GVB⁺⁺ buffer) and incubated for 2 hours at 4°C with or without Pseudo IgG molecules (5 µg/well). Thereafter 50 µl-s of 10% (v/v) normal human serum (NHS) or heat inactivated human serum (ΔNHS) was added for 30 minutes at 37°C. Subsequently, the mixture was diluted in 10x and 100x in PBS and plated on Trypric Soy Agar (TSA) plates for enumeration of CFU after overnight incubation. Data are means ± SD derived from three experiments.

The direct killing capacity of Panobacumab scFv (V_{L}-V_{H}).C4bpβ.Fc Pseudo IgG and anti-Psl scFv.C4bpβ.Fc Pseudo IgG molecules was assessed by complement-dependent killing assay. Bacterial cells (PAO1, reference strain O11, clinical isolate IPP6247290) were incubated with active (NHS) or decomplemented human serum (ΔNHS) in the presence or absence of Pseudo IgG molecules and plated onto TSA agar plates for CFU enumeration after overnight incubation. **Figure 20** shows that when bound, both pseudo-IgG-s facilitate complement activation and bacterial killing resulting in decreased number of Colony Forming Units (CFUs)/plate compared to NHS. Anti-Psl pseudo IgG reduced PAO1 CFU-count by 35% and IPP6247290 CFU-count by 31%. Pano Pseudo IgG reduced ATCC33358 O11 CFU-count by 26%.. This experiment shows that both Pano and Ps1 pseudo IgG-mediated C3b and C5b9 depositions has a bactericidal effect on *Pseudomonas aeruginosa.* PAO1-*Luciferase Pseudomonas* reference strain is however far straightforward to demonstrate pseudo-IgG-mediated complement-directed killing effect and further inhibition on bacterial growth **(****figure 17A****).**

### 4.11. Flow cytometry analysis of the Panobacumab scFv.C4bp(beta).Fc pseudo-IgG-mediated cross-linking of NK92^{humCD16} cell line with Pseudomonas aeruginosa

Present inventors have investigated the ability of the Panobacumab (V_{L}-V_{H}) scFv.C4bp(beta).Fc pseudo-IgG to cross-link *Pseudomonas aeruginosa* and NK cells. NK92^{humCD16} cell line was stained with violet cell tracer, whereas *P. aeruginosa* O11 ATCC strain was stained with PKH26. One µg pseudo-IgG (or no molecule as control) was incubated with (i) violet-NK92^{humCD16} alone, (ii) PKH26-O11 alone or (iii) with both cells and bacteria (ratio 1:5) for 1h at 4°C. After washing, the cells, bacteria or cell/bacteria were incubated with a goat anti-human IgG AF647-conjugated. Cells were analyzed using flow cytometry (Quanteon).

Flow cytometry analysis of the Pano scFv pseudo-IgG-mediated capture of *P. aeruginosa* by NK92^{humCD16} cell line demonstrated that the pseudo-IgG binds to *P. aeruginosa* and that the pseudo-IgG does not bind to NK92^{humCD16} alone (data not shown). In the absence of pseudo-IgG, there was no cross-linking between P. *aeruginosa* and NK92^{humCD16} (data not shown). There was a population that is violet-positive AND Fc-positive only when the pseudo-IgG is present and there was a population that is PKH26-positive AND Fc-positive only when the pseudo-IgG is present (data not shown). It was further confirmed that only when the pseudo-IgG is present, there is a double positive population for cell violet tracer and PKH26 (data not shown). In conclusion, the complexes pseudo-IgG and *P. aeruginosa* can be captured by NK92^{humCD16} cells through interactions between CD16 and the Fc. In contrast, free pseudo-IgGs are not captured by NK92^{humCD16}. This experiment shows that pseudo-IgGs are fully functional, and recruit NK effector cells when bound to their bacterial targets.

### 5. Example 5. Bifunctional heterodimers (TetraBolos) capable of killing Pseudomonas aeruginosa

### 5.1 Introduction

The opportunistic pathogen *Pseudomonas aeruginosa* (*P. aeruginosa*) causes life-threatening infections in humans, and is a major cause of hospital-acquired infections especially in immunocompromised patients. The complement system plays a crucial role in the early clearance of bacterial infection. At the contact with body fluids, *P. aeruginosa* evades human complement attack through the human Factor H (FH), the main inhibitor of the complement alternative pathway. FH protein family includes the major alternative pathway regulator FH, its splice variant FH-like protein 1 (FHL-1), and five FH-related proteins (FHRs). Recent evidence indicates a role of FHR-1 in the regulation or modulation of complement activation. FHR-1 is composed of five CCPs that are homologous to CCPs 6 and 7 and CCPs 18-20 of FH that are responsible for host surface recognition and contain an important C3b binding site, respectively. More particularly, the C-terminal SCR3-5 domains of FHR-1 are 100%/97%, 100% and 98% identical to FH/SCR18-20, respectively, and bind C3b and C3d. FH, that controls the amplification reaction at the level of C3, can bind to bacterial cells via complement control protein (CCP) domains 6-7 and 19-20. FHR1 compete with FH, acting as a positive regulator of complement attack. Importantly, FHR-1 is recruited to microbial surfaces, and thereafter the increasing concentrations of FHR-1 therein outcompete factor H, resulting in the down regulation of C3 convertase and increased complement activation. The activation of the complement alternative pathway subsequently amplifies the recruitment, priming, & activation of neutrophils and monocytes, thereby creating a self-amplifying inflammatory loop that ultimately results in destructive cell/bacteria targeting.

Present inventors have generated a bifunctional heterotetrabolos using an scFv anti-psl that is derived from an anti-bacterial antibody, for example directed at a surface structure of *P. aeruginosa,* such as dimeric PcrV or Psl scFv anti-*P*. *aeruginosa* (scFv anti-PcrV or scFv anti-PstI, such as described in International patent application WO2017095744-A1, which is incorporated herein by reference) to target the exopolysaccharide psl of *P aeruginosa* bacterium and the last 3 Short Consensus Repeats (SCR3-5) from (FHR1). Present inventors used the fusion protein consisting of (i) the Psl scFv (V_{L}-V_{H}) and (ii) SCR3 to SCR5 from FHR1, cloned upstream and downstream of C4bp C-terminal beta-chain, respectively **(****figure 22A****)**. Psl/FHR1(SCR3-5) construct binds to *P. aeruginosa* through its Psl binding moiety. Therefore, at the targeted bacteria surface, FHR1(SCR3-5) C-terminal effector moiety of the HeteroTetraBolos competes with hijacked bound factor H (FH) for C3b, which deregulated FH-mediated complement decay and leads locally to complement alternative pathway (AP) activation and subsequent bacterial lysis, as sketched in **figure 22B****.**

### 5.2 Genes used and cassette design

BspE1-(SGGGGS)₅-C-terminal part of C4bpβ-(SGGGGS)₅-MluI-FHR1(SCR3-5)-His8x-Stop-NotI

### Gene used:

- Psl scFv anti-P. *aeruginosa* is the scFv anti-PstI as described in International patent application WO2017095744-A1.
- SCR3-SCR5 of Complement factor H-related protein 1 (CFHR1): UniProtKB - Q03591 (FHR1 HUMAN)
- All other components are described elsewhere in Example 1

### 5.3 Demonstration of the binding and complement activation of the heterotetrabolos Psl/FHR1(SCR3-5) on P. aeruginosa

The dose-dependent binding of the HeteroTetraBolos Psl/FHR1(SCR3-5) to PAO1 *P. aeruginosa* cells was confirmed by ELISA using 1.5.10⁵ bacteria/well immobilized onto 96-well ELISA plates. The bound molecules were revealed using a mouse anti-HIS HRP-conjugated mAb **(****figure 22C****).** To measure C3b deposition on bacteria, present inventors used the same ELISA in presence of 0.5% of normal human serum (NHS) or decomplemented human serum (ΔNHS) for 30 minutes. C3b was detected with a mouse anti-human C3/C3b/iC3b mAb followed by a goat anti-mouse IgG HRP-conjugated revelation antibody. The addition of Psl scFv/FHR1(SCR3-5) HeteroTetraBolos increased C3b deposition thereby activating the complement on the surface of PAO1 *P. aeruginosa* cells, only in the presence of non-decomplemented serum, C3b deposition being completely abrogated when ΔNHS **(****Figure 22D****).**

### 5.4 Demonstration of the killing of P. aeruginosa by the HeteroTetraBolos Psl/FHR1(SCR3-5)

The direct killing of Psl/FHR1(SCR3-5) was shown using the PAO 1-Luciferase *P. aeruginosa* strain after 5 hours of incubation with the HeteroTetraBolos. Bacteria growth was measured (luciferase signal in RLU) at different time points (5 hours). Psl scFv/FHR1(SCR3-5) HeteroTetraBolos, in the presence of 50% NHS, induced a direct killing of the bacteria preventing their growth throughout the duration of the experiment (5 hours). The last three SCRs (SCR3-5) from FHR1 containing the binding domain for C3b that activate the complement alternative pathway are sufficient to elicit locally an efficient FH deregulation. NHS alone has a weak intrinsic bactericidal effect (background). This innovative construct directs selectively and locally the activation of the complement alternative pathway (AP) at the targeted membrane surface, which represents a novel disruptive destructive cell targeting approach.

### Example 6. Generation of multi target CCP1-2 pseudo-IgGs

### 6.1. Introduction

The complement system is a complex innate immune surveillance system responsible for the defense against invading pathogens, inflammation and homeostasis of the host. The complement system discriminates between self, modified-self (e.g. senescent, apoptotic or necrotic cells) and non-self, and is able to tag the last 2 but not the first type of cells/pathogens, as danger signal, which leads to (i) direct complement-mediated cytotoxicity (CDC), or recruit and activate immune effector cells (e.g. NK cells, macrophages) for (ii) complement-mediated cell cytotoxicity (CDCC) or phagocytosis (CDCP). The complement system is the first line of defense against invading microbes, and is responsible for the elimination of the vast majority of bacterial infections. Selective pattern recognition molecules such as Mannan-binding lectin (MBL), C1q, properdin or antibodies are the circulating molecules responsible for this self/modified-self/non-self discrimination. Four membrane-anchored complement regulatory proteins (mCRPs such as CD35, CD46, CD55 and CD59) are expressed on self-cells to control unwanted complement activation towards self-cells. Moreover, 3 soluble complement regulatory proteins (sCRPs such as C4b binding protein or C4bp, factor H or C1-inhibitor) bind to self-cells and protect them from complement-destruction. Factor H negatively regulates the complement alternative pathway (AP), whereas C4bp negatively regulates the complement classical & lectin pathways (CP & LP). C1-inhibitor inhibits C1r, C1s, MASP1 and MASP-2, which are the involved in the activation of the lectin pathway. Additional soluble complement regulatory proteins such as clusterin & vitronectin inhibit the downstream formation of the complement terminal complex C5b9 also called membrane attack complex (MAC).

Pathogenic invading microorganisms have developed several mechanisms to interfere with the complement system in order to survive in the host. Most of pathogenic bacteria indeed evade complement-mediated killing by hijacking soluble complement regulatory proteins such as C4b binding protein and/or factor H. Multiple bacteria can bind the C4bp alpha-chain at different sites to hijack C4bp. C4bp alpha-chain contains 8 complement control proteins (CCPs), also called short consensus repeats (SCRs). The majority of bacteria that hijack C4bp (but not all) bind the first two N-terminal CCPs (CCP1-2) of C4bp alpha-chain. This is the case for *Neisseria gonorrhoeae* and *meningitidis* that recruit factor H and C4bp. *Ngonorrhoeae* binds C4bp alpha-chain through N-terminal CCP1-2. This CCP1-2 domain can be used as targeting moiety, combined to a complement activation function as effector moiety, in order to generate multi-target biologics that direct complement-mediated killing towards the pathogens that bind CCP1-2 from C4bp alpha-chain, such as *Bordetella pertussis, Bordetella burgdorferi, Haemophilus influenza, Moraxella catarrhalis, Neisseria gonorrhoeae & meningitidis, Streptococcus pneumoniae & pyogenes, Yersinia enterocolitica* and *Staphylococcus aureus*) as well as fungi (*Candida dubliensis, Aspergillus fumigatus & terreus*).

The first two N-terminal SCRs of C4bp alpha-chain (CCP1-2) are the binding domain for these bacteria & fungi. Anna Blom et al. have designed a CCP1-2 oligomeric biologics (C4BP-IgM) using an IgM scaffold (S. Bettoni et al., JCI Insight. 2019. 4:23: e131886) as therapeutic approach to treat *Neisseria gonorrhoeae* infections.

In the present example, present inventors have cloned CCP1-2 domain in their pseudo-IgG scaffold to generate CCP1-2.C4bpβ.Fc multi-target pseudo-IgGs **(****figure 23A****).**

### 6.2. Cloning, transfection, cell culture and sub-cloning

The 372 bp cDNA encoding the 124 residues of CCP1-2 (amino acids 49-172) from C4bp N-terminal alpha-chain (UniProtKB - P04003 - C4BPA_HUMAN) were codon-optimized and synthetized between the restriction sites Bgl2 and BspE1 (in the open reading frame) by the company Proteogenix. The synthesized cDNA was cloned between the Bgl2 and BspE1 restriction sites of the pseudo-IgG cassette in the final pEF-IRESpac expression vector. The vector was amplified and then transfected in HEK293T cells. Transfection was stabilized using Puromycin selection antibiotic. Stable single clones resisting to Puromycin were manually picked-up and cultured individually in a 96-well cell culture plate. Supernatants from individual clones were quantitatively analyzed using Fc-Fc homemade symmetrical ELISA for CCP1-2.C4bpβ.Fc pseudo-IgG (data not shown).

### 6.3. Production

The best-expressing clone was expanded for scaled-up culture production in 5-chamber cell stacks. For large production, cells were cultured in cell-stack in serum-free optiMEM for 48h, then were boosted for 24h with complete DMDM medium. Up to 4 cycles of production were performed, putting new optiMEM for 48h for each cycle.

### 6.4. Purification, SDS-PAGE / Sypro Ruby staining analysis

CCP1-2 pseudo-IgG-containing optiMEM supernatant was incubated with Protein G Sepharose 4 Fast Flow beads (1 ml/liter, Cytiva) for 2-3 days using roller bottle. Beads were collected and packed to 1ml polypropylene column (Qiagen). After washing, the column was connected to a peristatic pump and elution was performed at pH 2.7, and immediately neutralized to a final pH 7.2. Purified CCP1-2 pseudo-IgG-containing eluate was concentrated using 30 WMCO centrifugal PVDF filter device. Purified CCP1-2 pseudo-IgG was analyzed using SDS-PAGE followed by an overnight staining with SYPRO Ruby Protein Gel Stain. After washing, the gel was analyzed using an Amersham Thyphoon biomolecular imager using the appropriate filter. The apparent MW of CCP1-2 pseudo-IgG is 120 kDa and 60 kDa under non-reducing and reducing conditions, respectively **(****figure 23A****).**

### 6.5. Binding testing assay to Neisseria gonorrhoeae

*Neisseria gonorrhoeae* were incubated with CCP1-2 pseudo-IgG (10 µg/ml) for 30 min. at 37°C. Bacteria were then stained with a goat anti-human IgG AF488 conjugated pAb (Thermofisher ref. A11013). Bacteria were fixed and analyzed by FACS. The assay was made in triplicate. In the absence of CCP1-2 pseudo-IgG, no signal was observed. This assay shows evidence for a specific binding of CCP1-2 pseudo-IgG to Neisseria gonorrhoeae **(****figure 23B****).**

### 6.6. Terminal complement complex deposition assays

*Neisseria gonorrhoeae* were incubated with CCP1-2 pseudo-IgG (10 µg/ml) or CCP1-2-IgM (10 µg/ml) for 30 min. at 37°C. After washing, bacteria were incubated with 10% NHS for 15 min. at 37°C. After washing, bacteria were incubated with a mouse anti-C5b9 mAb (Hycult Bioteh, clone aE11), and a goat anti-mouse IgG AF-conjugated. Bacteria were fixed and then analyzed by FACS. The assay was made in triplicate. This assay clearly indicates that CCP1-2 pseudo-IgG elicit MAC depositions on *Neisseria gonorrhoeae* **(****figure 23C****).** Controls using NHS alone in the absence of molecule, or ΔNHS in the presence of molecule, show no MAC formation. CCP1-2 pseudo-IgG-directed MAC formation on *Neisseria gonorrhoeae* is statistically stronger when compared to CCP1-2-IgM construct.

### 6.7. Neisseria gonorrhoeae survival assay

*Neisseria gonorrhoeae* were incubated with CCP1-2 pseudo-IgG (2 or 10 µg/ml) for 30 min. at 37°C. After washing, bacteria were incubated with 10% NHS or ΔNHS for either 30 min. or 90 min. Bacteria were plated to LB agar dishes overnight, and counted the next day. The number of bacteria was represented as Log(CFU/ml). The assay was made in triplicate.

This assay confirms that CCP1-2 pseudo-IgG-induced MAC formation previously observed leads to subsequent bacteria killing **(****figure 23D****)**. At 10 µg/ml, bacteria growth was fully abrogated after 30 min. incubation with NHS. When NHS was decomplemented, bacteria growth was fully recovered in the presence of 10 µg/ml CCP1-2 pseudo-IgG. At 2 µg/ml, bacteria growth was reduced of about 27% after 30 min. incubation with NHS, and was fully abrogated after 90 min. incubation with NHS. CCP1-2 pseudo-IgG displays a high bactericidal effect against *Neisseria gonorrhoeae* that is complement dependent.

### 6.8. Conclusive remarks

This example confirms that the pseudo-IgG scaffold has a superior ability to direct complement-mediated MAC formation and subsequent killing towards pathogenic bacteria, compared to the IgM scaffold, which was unexpected since IgM is the antibody isotype with highest complement activation efficacy. This example furthermore indicates that, beyond antibody fragments such as scFv or nanobody, another ligands can be used as targeting moiety. At last, due to the fact that CCP1-2 N-terminal domain of C4bp alpha-chain is recognized by multiple pathogens (bacteria and fungi), CCP1-2 pseudo-IgG is not an antibody-like biologics recognizing a single target, but it is a multi-target pseudo-IgG high broad-spectrum therapeutic use.

### 7. Example 7. Homodimeric protein complexes targeting Sphingomyelinase D (SmaseD)

### 7.1. Introduction

*Sphingomyelinase D* (*SmaseD,* 32 kDa) is an enzyme is found in the *Loxosceles* spider venom, the main cytotoxic component that is responsible for the dermo-necrotic and hemolytic activities observed in envenomed patients with *Loxosceles* venom and subsequently subject to so-called *loxoscelism.* The development of neutralizing therapeutic antibodies directed against the *SmaseD* provides an efficient alternative to conventional serum therapy as currently implemented in Brazil. The group of Dr. Larissa M Alvarenga, the laboratory of biochemistry, department of parasitology of the University of Parana in Curitiba, Brazil, has characterized the LimAb7 murine antibody that is to date the only monoclonal antibody able to neutralize the *SmaseD* and subsequent dermo-necrotic activity of the *L. intermedia* venom. The group of Dr. Larissa M Alvarenga has produced a scFv from the LimAb7 murine antibody, referred to herein as scFv-*Li*7 (such as described in Karim-Silva et al., Generation of recombinant antibody fragments with toxin-neutralizing potential in loxoscelism, Immunology Letters, 2016, Volume 176, p. 90-96). To improve the neutralizing activity of the scFv-*Li*7, present inventors have generated (i) a HomoDiBolo (homoB2C) to show that a scFv C-terminal of the C4bpβ dimerization scaffold (non-natural "upside down" position) keeps its biological features, and (ii) a HomoTetraBolo (homoB4).

### 7. 2. Expression vector (pEF-IRESpac) and cassette design

The pEF-IRESpac bicistronic expression vector is a vector suitable for the stable expression of recombinant therapeutic glycoproteins in mammalian cells. The pEF-IRESpac contains an "Internal Ribosome Entry Site" (IRES) downstream of the multiple cloning site (MCS), followed by the Puromycin resistance gene (*pac*)*.* The protein expression is driven by the strong human polypeptide chain elongation factor 1α promoter for high protein expression. The signal peptide, from the tumor necrosis factor receptor superfamily member 16 (TNFR16, UniProt no. P08138.1) was cloned between the restriction sites ECoRI and Bgl2. The cDNA encoding the different cassettes (HomoB2C, HomoB4, HomoB4 bis, HomoB4 ter) and spanned between the Bgl2 and NotI restriction sites were codon-optimized for human cells, synthesized and cloned into pEF-IRESp by Proteogenix (Illkirsch, Strasbourg). The C4bp C-terminal β-chain (UniProt no. P20851.1, aa: 137-252) comprises (3x SGGGGS (SEQ ID NO: 3)) linkers on both ends - beginning with the BspE1 restriction site - and is followed by the sequence encoding the scFv*Li*7 and a 8x His-tag, and ends with a stop codon and the NotI restriction site.

The cassettes for the homodimers (also referred to herein as HomoDiBolos (HomoB2C)) & homotetramers (also referred to herein as HomoTetraBolos (HomoB4)):
- HomoB2C [Signal peptide-AA-[3x(SGGGGS)]-C4bp(beta)-[3x(SGGGGS)]-scFv*Li*7(V_{H}-V_{L})-His8x], wherein AA are two alanine residues,
- HomoB4 [Signal peptide-scFv*Li*7(V_{H}-V_{L})-[3x(SGGGGS)]-C4bp(beta)-[3x(SGGGGS)]-scFv*Li*7(V_{H}-V_{L})-His8x]
- HomoB4 **bis** contains the first 8 amino acids (QIVLSQSP (SEQ ID NO: 5)) of rituximab (RTX) - replacing the first 8 amino acids of the original scFv*Li*7 - which is located downstream (i.e. 3') of the signal peptide, as shown in figure 24 A:
   [Signal peptide-QIVLSQSP-scFv*Li*7(**V**_{L}-**V**_{H})-[3x(SGGGGS)]-C4bp(beta)-[3x(SGGGGS)]-scFv*Li*7(V_{H}-V_{L})-His8x]
- HomoB4 **ter** contains the 3x (SGGGGS (SEQ ID NO: 3)) linker after the signal peptide as shown in figure 24 A: [Signal peptide-**(3xSGGGGS)**-scFv*Li*7(V_{H}-V_{L})-[3x(SGGGGS)]-C4bp(beta)-[3x(SGGGGS)]-scFv*Li*7(V_{H}-V_{L})-His8x]

### 7. 3. Transient transfections, micro-purifications using Sepharose Ni-beads

HEK293T cells were used to express the different molecules. HEK293T cells were plated at densities 1.3×10⁶ cells/well into a 6-well cell culture plate and complete DMEM medium the day prior to transfection. The next day, the complete DMEM medium was replaced by optiMEM medium. Cells were transfected with the expression vectors using the lipofectamine 3000 kit according to the instruction datasheet (Thermo Fisher, catalog number L3000001).

Forty-eight hours after transfection, optiMEM supernatants from transfected cells were collected in 15-ml Falcon tubes. Supernatants were incubated overnight with agarose Ni-beads to capture the produced His-tagged molecules (HomoB2C, homoB4, homoB4 bis, homoB4 ter). The next day, the tubes were centrifuged (4000 rpm, 4°C, 4 min.). The supernatants were discarded and the beads were washed twice with 12 ml PBS. Ni-beads were incubated with 40 µl elution buffer (20 mM phosphate buffer, pH7.2, 500 mM NaCl, 1 M Imidazole). After vortexing, the tubes were centrifuged and the eluate was collected for further analysis.

### 7. 4. Stable transfection, cell culture, subcloning

Forty-eight to seventy two hours after transfection, cells were transferred into a 10-cm cellule culture dish and a DMEM medium supplemented with Pen/Strep antibiotics (P/S), Glutamine (Gln), 10% (v/v) fetal bovine serum (FBS) and 20 µg/ml puromycin (PURO20) selection antibiotic. After 2 weeks, growing clones that are resistant to puromycin were individually manually picked up and transferred into 96-well plates. About a week later, supernatants from single isolated clones were screened for tetrabolo expression levels using a symmetrical HIS ELISA.

### 7. 5. Quantification using symmetrical HIS ELISA

A 6-His Tag rabbit anti-his polyclonal antibody (Bethyl, A190-114-P) was coated onto a NUNC MaxiSorp^{™} 96-well flat-bottom polystyrene 96-well *ELISA* plate for 48h (100 ng/well/100µl PBS). The plate was washed (5x) with 150 µl PBS-1% (w/v) BSA and blocked for 1h with 100 µl PBS-5% (w/v) BSA at 4°C. Crude tetrabolo-expressing cell culture supernatants (up to 200 µl) were incubated for 1h at 4°C on the ELISA plate, and after washing for 5 times, the ELISA plate was incubated with a mouse anti-HIS HRP-conjugated revelation monoclonal antibody (SIGMA) in PBS-1% (w/v) BSA. After 5x washings, the ELISA plate was stained with 100 µl OPD (O-phenylenediamine dihydrochloride)/H₂O₂ chromogenic substrate, and the staining reaction was stopped with 0.5N H₂SO₄ and was read using a spectrophotometer/absorbance at 492 nm. A molecule of known concentration was used as gold standard (the 2D3 V_{H}H anti-HER2.C4bpα.His8x multimers).

Quantitative analysis of the homoMultiBolo, HomoB2C (homoDiBolo), and the three HomoB4 (homoTetraBolos) molecules expressed in the cell culture supernatants from transfected HEK293 cells was evaluated using symmetrical His ELISA and demonstrated that all molecules are successfully expressed and exported by the cells (data not shown).

Subsequently, the production of the molecules was up-scaled and purified using fast protein liquid chromatography (FPLC), immobilized metal affinity chromatography (IMAC) technology using 1 or 5 ml Ni-sepharose Excel columns. The elution solution was concentrated and dialyzed. The presence of glycerol allows preventing any unwanted precipitation during the concentration with Amicon.

### 7. 6. Analysis of the specific binding of HomoB2C and HomoB4 to the SmaseD from Loxosceles intermedia venom using ELISA (figure 24 B and C)

The ELISA procedure was performed as described above (section 7.4), with the following modifications: an ELISA plate was coated with 100 µl PBS/well containing 220 ng venom from *Loxosceles intermedia* (2.2 µl/mL) (kind gift from Pr. Philippe Billiald, Faculté de Pharmacie, Laboratoire de Biochimie, Université Paris Saclav). After blocking (PBS/ 5% (w/v) BSA), the ELISA plate was incubated with 2-fold serial dilutions of crude DMEM cell culture supernatants containing HomoB2C, homoB4, homoB4 bis and homoB4 ter. The concentrations of the diverse molecules was established using a HIS-HIS ELISA with a standard curve. As positive and negative controls, a horse polyclonal serum *anti-Loxosceles intermedia* venom and a horse non-immune serum were used as positive & negative controls (positive and negative SALOX), respectively, with a 2-fold serial dilution (starting dilution 1: 1000). An anti-HIS mouse HRP-conjugated secondary monoclonal antibody was used to characterize the molecules and a goat anti-horse IgG HRP-conjugated was used to reveal the horse *anti-Loxosceles intermedia* antibodies.

It was demonstrated that specific binding of HomoB2C, HomoB4, HomoB4bis and HomoB4ter were able to specifically bind to the *SmaseD* from *Loxosceles intermedia* venom, in a concentration dependent manner. Furthermore, it was observed that a higher valence of scFvLi7 results in a higher binding affinity (data not shown).

### 7. 7. SDS-PAGE & Western blotting

### 7. 7. 1. Characterization of the HomoDiBolos (HomoB2C) and HomoTetraBolos (HomoB4) using SDS-PAGE, Western blotting and revelation with an anti-His antibody

Fifteen µl of micro-purified tetrabolos from transient transfections or 1 µg purified molecules from stable transfections were mixed to 4X Laemmli sample buffer without or with 10% (v/v) β2-mercaptoethanol (reducing conditions) and were electrophoresed using 4-15% Mini-Protean^{®} Tris-Glycin eXtended (TGX^{™}) precast protein gel (Bio-Rad) and SDS-polyacrylamide gel electrophoresis (SDS-PAGE) using XT MES running buffer (Bio-Rad). After electrophoresis, gel was electroblotted onto a low-fluorescence background PVDF membrane (activated in methanol prior to use) using TTB (25 mM Tris, 192 mM glycine, 0.01% (v/v) SDS, 20% (v/v) methanol, pH 8.8). Protein transfer was carried out using a Bio-Rad Mini *Trans*-Blot^{®} electrophoretic transfer cell. After blocking with PBS-5% (w/v) low-fat milk, the membrane was incubated for 1h in the same buffer with 1µg rabbit anti-His 6x-tag pAb. After three washings with TBS-Tween buffer, pH 7.2, the membrane was incubated for 1h with a goat anti-rabbit IgG AF488-conjugated antibody in PBS-1% (w/v) low-fat milk. After three washings, the membrane was dried-off and analyzed using an Amersham Typhoon scanner using the adapted filter for AF488 (FITC) fluorochrome (Cy2).

SDS-PAGE and WB demonstrated that under non-reducing conditions the molecules are present as dimers (with HomoB4 having a MW of about 240 kDa and homoB2C having a MW of about 70 kDa), while under reducing conditions the molecules are expressed as a single molecular specifies (with the single molecular species of HomoB4 having a MW of about 120 kDa and the single molecular species of homoB2C having a MW of about 70 kDa).

### 7. 7. 2. Binding of the HomoDiBolos (HomoB2C) to SmaseD present in Loxosceles intermedia (Li) venom analyzed using SDS-PAGE & Western blotting (figure 24 D)

Five µg/lane of venom from *Li* were electrophoresed (under non-reducing conditions) using 15% Mini-Protean^{®} TGX^{™} precast protein gel on a Mini-PROTEAN^{®} Tetra cell (Bio-Rad) using SDS-PAGE. After electrophoresis, gel was electroblotted onto a nitrocellulose membrane. The membrane was then blocked for 1h using PBS-5% (w/v) low-fat milk. The membrane was then sliced into pieces to probe simultaneously for multiple conditions of incubation and revelations.
- The first slices were incubated with (i) a horse immune anti-*SmaseD Li* serum (1:20000), (ii) the second slices with the purified HomoB2C-scFv*Li*7 (10 µg/ml) or (iii) the third slices with an irrelevant monoclonal antibody.
- After three washings (using Tris-buffered Saline + 0.05% (v/v) Tween20), (i) the first slices were incubated with a horse polyclonal sera *anti-Loxoceles intermedia* venom (SALOX) diluted 1: 10,000, (ii) the second slices with a rabbit anti-His 6x-tag pAb and the third slices with a secondary antibody HRP-conjugated.
- After three washings (using Tris-buffered Saline + 0.05% Tween20), (i) the first slices were then incubated with a goat anti-horse IgG HRP-conjugated secondary antibody, and the (ii) second slices were incubated with a goat anti-rabbit IgG HRP-conjugated secondary antibody.

After three washings, the slices were revealed with ECL Western blotting HRP-substrate, DAB (3,3'-Diaminobenzidine)/chloronaphtol and with Rouge Ponceau.

It was demonstrated that like the horse anti-*SmaseD Li* serum (positive control), purified HomoB2C-scFv*Li*7 (10 µg/ml) was able to bind to specifically bind *L. intermedia* venom, while no binding was observed with the irrelevant mAb (negative control).

### 7. 8. Conclusion

The scFv*Li*7 anti-*SmaseD* was cloned C-terminally (and optionally also N-terminally) of the C4bpβ dimerization scaffold and expressed as a dimeric molecule with 2 (HomoDiBolos) or 4 (HomoTetraBolos) valences in order to enhance its ability to neutralize the *SmaseD* from the *Loxosceles intermedia* brown spider venom. Present inventors have shown that the HomoDiBolos (HomoB2C) & HomoTetraBolos (HomoB4) bind to *SmaseD,* and a slightly better binding was observed for HomoTetraBolos when compared to 2-valence counterpart as observed using ELISA. The HomoDiBolo was shown to be useful for demonstrating that, when a scFv is cloned C-terminally (downstream) of the C-terminal C4bp beta-chain dimerization scaffold in a non-natural "upside down" location: (i) the dimerization process still takes place and (ii) the scFv*Li*7 keep unexpectedly its binding capacity to its *SmaseD* target. The Homo- or Hetero-DiBolos can be used as therapeutics for applications such as ligand-receptor interactions as targeting moieties, but also as effector moieties to immune interventions through immune system modulation.

Using a scFv with neutralizing properties for *SmaseD,* present inventors showed that the scFv anti-*SmaseD,* when cloned downstream of the C4bpβ dimerization scaffold, keeps its binding capacity to the *SmaseD* (HomoB2C or HomoDiBolos), and the HomoTetraBolos (HomoB4) displayed superior binding capacity to *SmaseD* when compared to homoB2C. The DiBolos can be homo- (e.g. single transfection) or heterodimeric (e.g. co-transfection). This example shows that scFv and V_{H}H can be indifferently introduced upstream or downstream of the C4bpβ without altering their binding capacities.

## Claims

1. A dimeric protein complex comprising
a first polypeptide comprising a first functional component and the C-terminal fragment of the C4b binding protein (C4bp) beta-chain, wherein said first functional component is coupled to the C-terminus of the C-terminal fragment of the C4bp beta-chain; and
a second polypeptide comprising a second functional component and the C-terminal fragment of the C4bp beta-chain, wherein said second functional component is coupled to the C-terminus of the C-terminal fragment of the C4bp beta-chain;
wherein said first polypeptide further comprises a third functional component that is coupled to the N-terminus of said C-terminal fragment of the C4bp beta-chain;
wherein said second polypeptide further comprises a fourth functional component that is coupled to the N-terminus of said C-terminal fragment of the C4bp beta-chain;
wherein said first, second, third and fourth functional components are proteins or polypeptides;
wherein two of said first, second, third and/or fourth functional components comprise a binding domain;
wherein said first and second polypeptides are identical or different; and
wherein the C-terminal fragment of the C4bp beta-chain is the 194-252 fragment of the C4bp beta-chain.

2. The dimeric protein complex according to claim 1, wherein said binding domain is a single chain variable fragment of an antibody (scFv) or a single domain variable fragment of a heavy chain antibody (V_{H}H) specific for the antigen, an antibody-like scaffold, an extracellular domain of a viral envelope protein, a therapeutic agent, a cognate extracellular domain of a receptor or ligand for the antigen or an antigen-binding portion of said receptor or ligand, a soluble receptor, or a synthetic receptor.

3. The dimeric protein complex according to claim 1 or 2, wherein said binding domain specifically binds to a tumor specific antigen (TSA), a tumor associated antigen (TAA), a bacterial antigen, a viral antigen or a virus-associated antigen, a fungal antigen, an NK cell receptor, a cytokine, a toxin or a contaminant.

4. The dimeric protein complex according to any one of claims 1 to 3, wherein two of said first, said second, said third or said forth functional components comprise a binding domain specifically binding to an NK cell receptor; and wherein two of said first, said second, said third or said forth functional components comprise a binding domain specifically binding to a TSA, a TAA, a bacterial antigen, a viral antigen, a viral-associated antigen or a fungal antigen.

5. The dimeric protein complex according to claim 4,
wherein the third and fourth functional components comprise a scFv specifically binding NKG2A or a scFv specifically binding KIR; optionally wherein the first and second functional components comprise a soluble IL-15 receptor alpha chain (sIL15Ralpha) or a complement receptor type 1.

6. The dimeric protein complex according to claim 4, wherein the third and fourth functional components comprise a scFv specifically binding PsI or PcrV of *P. Aeruginosa*; optionally wherein the first and/or second functional components comprise a scFv specifically binding NKG2D and/or SLAMF7.

7. The dimeric protein complex according to any one of claims 1 to 3, wherein two of said first, said second, said third or said forth functional components comprise SCR3, 4 and 5 from factor H-related protein 1 (FHR1); and wherein two of said first, said second, said third or said forth functional components comprise a binding domain specifically binding to a TSA, a TAA, a bacterial antigen, a viral antigen, a virus-associated antigen, or a fungal antigen.

8. The dimeric protein complex according to claim 7, wherein the third and fourth functional components comprise SCR 3, 4 and 5 from FHR1; and optionally wherein the first and/or second functional components comprise a scFv specifically binding PsI.

9. The dimeric protein complex according to any one of claims 1 to 3,
wherein the first, second, third and/or fourth functional components comprise a scFv specifically binding sphingomyelinase D from *Loxosceles.*

10. A nucleic acid encoding the first polypeptide of the dimeric protein complex as defined in any one of claims 1 to 9; and/or encoding the second polypeptide of the dimeric protein complex as defined in any one of claims 1 to 9.

11. An expression cassette comprising the nucleic acid according to claim 10.

12. An expression vector comprising the nucleic acid according to claim 10 or the expression cassette according to claim 11.

13. A pharmaceutical composition comprising the dimeric protein complex according to any one of claims 1 to 9, the nucleic acid according to claim 10, the expression cassette according to claim 11, or the expression vector according to claim 12, and a pharmaceutically acceptable carrier.

14. The dimeric protein complex according to any one of claims 1 to 9, the nucleic acid according to claim 10, the expression cassette according to claim 11, the expression vector according to claim 12, or the pharmaceutical composition according to claim 13, for use as a medicament.

15. The dimeric protein complex according to any one of claims 1 to 9, the nucleic acid according to claim 10, the expression cassette according to claim 11, the expression vector according to claim 12, or the pharmaceutical composition according to claim 13, for use in the treatment of a neoplastic disease or an infectious disease.

16. The dimeric protein complex according to any one of claims 1 to 9, the nucleic acid according to claim 10, the expression cassette according to claim 11, the expression vector according to claim 12, for use in a method of molecular imaging of a living body.
